# EUROPÄISCHE PATENTSCHRIFT

(11) **EP 2 881 063 B1**
(45) Veröffentlichungstag und Bekanntmachung des Hinweises auf die Patenterteilung: **26.06.2019**
(21) Anmeldenummer: 14004402.5
(22) Anmeldetag: 25.04.2013
(51) Int. Cl.: A61B 34/30, B25J 19/00, A61B 90/00, A61B 50/30, A61B 90/90

(54) **Chirurgierobotersystem**
Surgical robot system
Système de robot chirurgical

(30) Priorität: 27.04.2012 DE 102012008535; 06.08.2012 DE 102012015541; 18.09.2012 DE 102012018432
(43) Veröffentlichungstag der Anmeldung: 10.06.2015
(62) Teilanmeldung aus: 13719024.5
(73) Patentinhaber: KUKA Deutschland GmbH, 86165 Augsburg (DE)
(72) Erfinder: Lohmeier, Sebastian, 80639 München (DE); Nguyen-Xuan, Cuong, 79183 Waldkirch (DE); Neff, Thomas, 86163 Augsburg (DE); Schober, Wolfgang, 86554 Pöttmes (DE)
(74) Vertreter: Schlotter, Alexander Carolus Paul

(56) Entgegenhaltungen:
- EP-A1- 2 428 337
- WO-A2-2011/149187
- US-A1- 2009 248 039
- US-A1- 2010 268 249
- US-A1- 2011 245 833
- US-A1- 2011 277 775

## Beschreibung

Ein Aspekt der vorliegenden Erfindung betrifft ein Chirurgierobotersystem mit einer Roboteranordnung und einer Instrumentenanordnung mit wenigstens einem durch die Roboteranordnung geführten Instrument, eine solche Instrumentenanordnung sowie ein Verfahren zur Montage eines solchen Chirurgierobotersystems bzw. einer solchen Instrumentenanordnung.

Chirurgische Instrumente sollen möglichst steril sein. Auf der anderen Seite sind Roboter und Antriebe, bedingt beispielsweise durch Schmiermittel, Abtrieb und dergleichen, nur schwer sterilisierbar.

Ein Ansatz besteht darin, das Instrument selber antriebslos auszubilden und es durch den mit ihm verbundenen Roboter zu aktuieren, beispielsweise telemanipulatorisch einen Endeffektor wie etwa eine Zange, Schere oder dergleichen zu bewegen. Das antriebslose Instrument selber ist leicht sterilisierbar. Der nicht sterile Roboter mit dem Antrieb des Instruments wird durch eine statische sterile Barriere umhüllt. Die EP 1 015 068 A1 schlägt einen an einer sterilen Hülle des Roboters befestigten Adapter vor, durch den der Instrumentenantrieb mechanisch hindurchgeführt wird.

Wird der Antrieb in das Instrument integriert, wie dies auch bei der vorliegenden Erfindung der Fall ist, können vorteilhafterweise kompaktere Roboter verwendet werden, da die Hindurchführung des Instrumentenantriebs entfallen kann.

Dabei ergeben sich jedoch mehrere Probleme: zum Einen bauen Instrumente mit integriertem Antrieb größer als antriebslose Instrumente. Dies kann die Handhabung, insbesondere bei mehreren kooperierenden Robotern, erschweren. Zum Anderen wird der in der Regel nicht oder nur schwer sterilisierbare Antrieb nicht mehr durch die sterile Hülle des Roboters abgedeckt.

Die US 2011/0277775 A1 betrifft einen sterilen Adapter, einen Vorhang mit dem Adapter und ein Verfahren zum Einkleiden eines Manipulatorarms. In einer Ausführung weist der sterile Adapter ein Gehäuse, welches dafür eingerichtet ist, ein entferntes Ende eines Instrumentenmanipulators, welcher eine Mehrzahl von Manipulatoraktuatorausgängen aufweist, in sich aufzunehmen, und eine Membranschnittstelle auf, welche eine Mehrzahl von Aktuatorschnittstellen aufweist.

Die US2011/0245833 A1 betrifft in ihrer Tiefe Steuer- und messbare Antriebseinrichtungen. Es werden Vorrichtungen und Verfahren zur Erzeugung eines Bohrlochs in einem Knochen offenbart. Die Vorrichtungen und Verfahren betreffen das steuern des Antriebsund das Messen der Arbeit eines Arbeitswerkzeugs in einer Weise, dass ein Bediener Verletzungen an umgebenden Strukturen vermeiden kann. Eine Aufgabe eines Aspekts der vorliegenden Erfindung ist es, wenigstens eines der vorgenannten Probleme zu lösen bzw. ein verbessertes Chirurgierobotersystem zur Verfügung zu stellen.

Diese Aufgabe wird durch ein Chirurgierobotersystem mit den Merkmalen des Anspruchs 10 gelöst. Anspruch 1 stellt eine Instrumentenanordnung für ein solches Chirurgierobotersystem unter Schutz, Anspruch 11 bzw. 13 ein Verfahren zur Montage eines solchen Chirurgierobotersystems bzw. einer solchen Instrumentenanordnung. Die Unteransprüche betreffen vorteilhafte Weiterbildungen.

Ein weiterer Aspekt der vorliegenden Offenbarung betrifft ein Chirurgierobotersystem mit einer Roboteranordnung und einer damit gekoppelten Instrumentenanordnung, ein chirurgisches Instrument, eine sterilisierbare Antriebseinheit für ein chirurgisches Instrument und ein Verfahren zum Sterilisieren einer solchen Antriebseinheit.

Um Sterilitätsanforderungen zu erfüllen, werden Operationssaalgegenstände üblicherweise vorab sterilisiert, meist, indem sie mit Heißdampf und/oder-luft beaufschlagt werden.

Nach betriebsinternem Stand der Technik sind bereits Chirurgierobotersysteme mit einem oder mehreren Robotern und durch diese geführten chirurgischen Instrumenten bekannt, die einen Instrumentenschaft und eine damit koppelbare Antriebseinheit zur Aktuierung von Freiheitsgraden eines Endeffektors aufweisen.

Bestimmte Komponenten solcher Chirurgierobotersysteme sind teilweise nicht für thermische Belastungen vorgesehen, wie sie bei einer Sterilisierung auftreten. Dies gilt insbesondere für bestimmte elektronische Bauteile von Antriebseinheiten von robotergeführten chirurgischen Instrumenten, insbesondere für Positionssensoren, die insbesondere zur teleoperativen Aktuierung eines Endeffektors bei minimalinvasiver Roboterchirurgie vorteilhaft sind.

Daher wird bisher das komplette Chirurgierobotersystem mit einer sterilen Einmalhülle abgedeckt, was kostspielig und abfallintensiv ist und die Handhabung erschwert.

Eine Aufgabe eines Aspekts der vorliegenden Offenbarung ist es, die Sterilisierung von Antriebseinheiten chirurgischer Instrumente von Chirurgierobotersystemen zu verbessern.

Ein weiterer Aspekt der vorliegenden Offenbarung betrifft ein Chirurgierobotersystem mit einer Roboter- und einer Instrumentenanordnung, eine Instrumentenanordnung für ein solches Chirurgierobotersystem, eine manuelle Betätigungseinheit für eine solche Instrumentenanordnung sowie Verfahren zum Bestücken einer solchen Roboteranordnung mit einem Instrument und eines Instruments mit einer Antriebseinheit.

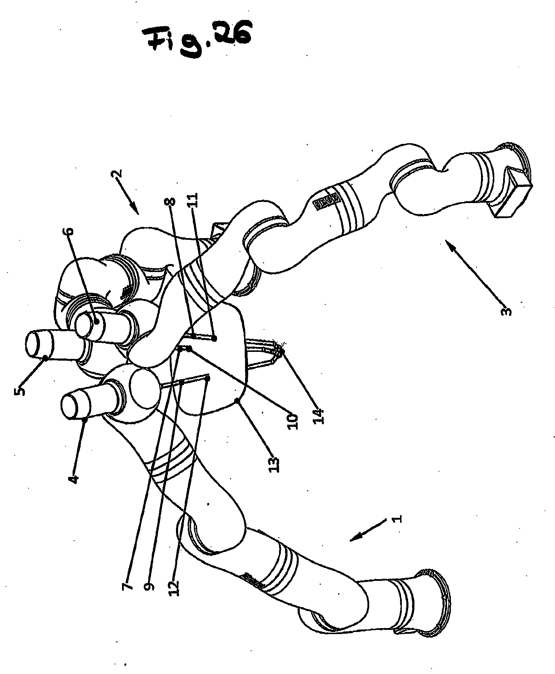

Aus der deutschen Patentanmeldung 10 2012 008 535.4 der Anmelderin, betreffend ein Chirurgierobotersystem, deren Offenbarungsgehalt vollständig in die Offenbarung der vorliegenden Erfindung einbezogen wird, ist ein gattungsgemäßes Chirurgierobotersystem bekannt. Fig. 26 zeigt zur Erläuterung exemplarisch ein erfindungsgemäßes Chirurgierobotersystem mit drei Robotern 1, 2, 3, die jeweils ein Instrument 4, 5 bzw. 6 führen, das am proximalen, roboternahen Ende jeweils eine Antriebseinheit und am distalen, roboterfernen Ende einen Endeffektor mit einem oder mehreren Freiheitsgraden zur Positionierung in einem Operationsgebiet 14 aufweisen. Zwischen dem proximalen und distalen Ende erstreckt sich ein Instrumentenschaft 7, 8 bzw. 9, der das Operationsgebiet 14 im Inneren eines Patienten durch eine kleine Öffnung 10,11 bzw. 12 beispielsweise in einer Bauchdecke 13 erreichen. Nicht dargestellt ist eine haptische Eingabestation, von der aus das Chirurgieroboter-System teleoperiert wird.

Eine Aufgabe eines Aspekts der vorliegenden Offenbarung ist es, ein gattungsgemäßes Chirurgierobotersystem zu verbessern.

### Chirurgierobotersystem

Ein Chirurgierobotersystem nach einem Aspekt der vorliegenden Offenbarung umfasst eine Roboteranordnung mit einem oder mehreren, insbesondere zwei, drei oder vier Robotern. Ein oder mehrere Roboter der Roboteranordnung können in einer Ausführung sechs oder mehr Gelenke, insbesondere Drehgelenke, aufweisen, wobei mehr als sechs Gelenke eine vorteilhafte Positionierung des redundanten Roboters ermöglichen können. Der bzw. die Roboter weisen in einer Ausführung eine Steuerung auf. Dabei können mehrere Roboter eine gemeinsame Zentralsteuerung und/oder Einzelsteuerungen aufweisen. Die Roboteranordnung, insbesondere ein oder mehrere Roboter, können in einer Ausführung an einem Operationstisch angeordnet, insbesondere lösbar befestigt, sein.

### Instrumentenanordnung

Eine Instrumentenanordnung nach einem Aspekt der vorliegenden Offenbarung umfasst ein oder mehrere durch die Roboteranordnung geführte Instrumente, sie ist entsprechend zur Befestigung an der Roboteranordnung eingerichtet bzw. als robotergeführte Instrumentenanordnung ausgebildet. In einer Ausführung ist an einem oder mehreren Robotern der Roboteranordnung je ein Instrument, vorzugsweise lösbar, befestigt bzw. befestigbar, insbesondere formschlüssig, reibschlüssig und/oder magnetisch, insbesondere elektromagnetisch. In einer Weiterbildung kann die Instrumentenanordnung mehrere, insbesondere unterschiedliche, Instrumente aufweisen, die, insbesondere wahlweise, an demselben oder verschiedenen Robotern der Roboteranordnung befestigbar sind.

Ein oder mehrere Instrumente der Instrumentenanordnung weisen jeweils einen ein- oder mehrteiligen, insbesondere rohrförmigen und/oder flexiblen oder vollständig oder teil- bzw. segmentweise starren, Instrumentenschaft auf, der zur teilweisen Einführung in einen Patienten vorgesehen ist. An seinem distalen Ende kann ein ein- oder mehrteiliger Endeffektor, insbesondere lösbar, angeordnet sein, insbesondere ein Skalpell, Zangen- bzw. Scherenschenkel oder dergleichen. Zusätzlich oder alternativ kann am distalen Ende beispielsweise ein Lichtquelle, ein optisches Aufnahmemittel, insbesondere ein Kamerachip, und/oder ein Lichtleiterende angeordnet sein, so dass das Instrument als Endoskop ausgebildet sein kann.

Ein Instrument ist in einer Ausführung ein endo- bzw. minimal-invasives chirurgisches Instrument ("MIC"), insbesondere ein endoskopisches, beispielsweise laparoskopisches oder thorakoskopisches. Insbesondere kann der Instrumentenschaft dazu vorgesehen bzw. eingerichtet sein, durch einen Eintritt, der vorzugsweise im Wesentlichen dem Außendurchmesser des Instrumentenschaftes entspricht, insbesondere durch einen Trokar, in den Patienten eingeführt und dort aktuiert zu werden.

Der Instrumentenschaft, insbesondere ein distales Teil und/oder ein Endeffektor des Instrumentenschaftes, kann ein oder mehrere Freiheitsgrade aufweisen. Insbesondere können ein oder mehrere Teile des Endeffektors je ein oder zwei Drehfreiheitsgrade um Drehachsen aufweisen, die vorzugsweise senkrecht zu einer Längsachse des Instrumentenschaftes stehen. Beispielsweise kann ein zweiteiliger Endeffektor eine Zange bzw. Schere darstellen, deren Schenkel gegensinnig um dieselbe Drehachse verschwenken.

Zur Aktuierung eines oder mehrerer Freiheitsgrade des Instrumentenschaftes weist ein Instrument in einer Ausführung eine Antriebsstranganordnung mit einem oder mehreren Antriebssträngen auf. Unter einem Antriebsstrang wird vorliegend insbesondere eine Anordnung von einem oder mehreren Übertragungsmitteln zur mechanischen, hydraulischen und/oder pneumatischen Übertragung von Bewegungen und/oder Kräften verstanden, wobei vorliegend zur kompakteren Darstellung auch antiparallele Kräftepaare, d.h. Drehmomente, verallgemeinernd als Kräfte bezeichnet werden. Solche Übertragungsmitteln können insbesondere Seilzüge, Schubstangen, Gelenke, Getriebe, insbesondere Kulissengetriebe zur Umsetzung einer Dreh- und einer translatorischen Bewegung ineinander, Umlenkrollen, Kupplungselemente und dergleichen sein bzw. umfassen. Somit wird vorliegend unter einem Antriebsstrang insbesondere eine Kette von miteinander mechanisch gekoppelten Übertragungsmitteln verstanden, die eine eingangsseitige Aktuierung durch eine Antriebseinheit ausgangsseitig auf den Instrumentenschaft, insbesondere einen Endeffektor des Instrumentenschaftes, übertragen und so einen Freiheitsgrad des Instrumentenschaftes aktuieren. Zwei oder mehr Antriebsstränge der Antriebsstranganordnung können insbesondere, wenigstens teil- bzw. abschnittsweise, parallel angeordnet sein und/oder sich kreuzen.

Nach einer Ausführung weist ein Instrument weiter eine modulare Antriebseinheit zur Aktuierung der Antriebsstranganordnung auf. Unter einer modularen Antriebseinheit wird insbesondere eine Antriebseinheit verstanden, die als bauliche Einheit ausgebildet und als Ganzes handhabbar, insbesondere lösbar mit dem Instrument, insbesondere einem Instrumentengehäuse verbindbar, ist.

Die Antriebseinheit ist zur Aktuierung einer oder mehrerer Freiheitsgrade des Instrumentenschaftes über die Antriebsstranganordnung ausgebildet und kann hierzu einen oder mehrere translatorische und/oder Drehantriebe aufweisen, die insbesondere einen oder mehrere Hydraulik-, Pneumatik- und/oder Elektromotoren aufweisen können. Die Antriebsstranganordnung kann in einer Ausführung translatorische und/oder Drehfreiheitsgrade des Instrumentenschaftes aktuieren und hierzu translatorische und/oder rotatorische Aktuierungen der Antriebseinheit translatorisch und/oder rotatorisch übertragen und gegebenenfalls ineinander umsetzen. In einer Ausführung kann die Antriebseinheit eine oder mehrere angetriebene Wellen aufweisen, deren Drehbewegung die Antriebsstranganordnung aktuiert. Zusätzlich oder alternativ kann die Antriebseinheit eine oder mehrere Kolben(stangen) aufweisen, deren translatorische bzw. Linearbewegung die Antriebsstranganordnung aktuiert. Die Antriebsstranganordnung kann solche Dreh- bzw. Linearbewegungen, beispielsweise über Seilzug- oder Schubstangengetriebe, an die Freiheitsgrade des Instrumentenschaftes vermitteln bzw. übertragen. In einer Ausführung weist die Antriebseinheit elektrische Kontakte zur Energieversorgung und/oder zur Signalübertragung auf, die insbesondere zur Koppelung mit der nachfolgend erläuterten elektro-mechanischen Schnittstelle ausgebildet sein können.

### Instrumentengehäuse

Nach einem Aspekt der vorliegenden Offenbarung weisen ein oder mehrere Instrumente der Instrumentenanordnung jeweils ein Instrumentengehäuse mit einem Antriebsstrang-Gehäuseteil auf, an dem wenigstens ein Teil der Antriebsstranganordnung angeordnet ist und das in einer Ausführung lösbar oder fest bzw. dauerhaft mit dem Instrumentenschaft verbunden, insbesondere integral ausgebildet sein kann. Bei dauerhaft mit dem Instrumentenschaft verbundenem Antriebsstrang-Gehäuseteil kann die gesamte Antriebsstranganordnung, ausgehend von einer Eingangsschnittstelle zur Antriebseinheit, an, insbesondere in dem Antriebsstrang-Gehäuseteil angeordnet sein. Bei lösbar verbundenem Instrumentenschaft und Antriebsstrang-Gehäuseteil kann ein Teil der Antriebsstranganordnung an, insbesondere in dem Antriebsstrang-Gehäuseteil und ein hiermit koppelbarer weiterer Teil an, insbesondere in dem Instrumentenschaft angeordnet sein. In einer Ausführung kann zwischen dem Instrumentengehäuse und dem Instrumentenschaft ein, insbesondere aktuierter und/oder rotatorischer, Freiheitsgrad vorgesehen bzw. ausgebildet sein, um den Instrumentenschaft, insbesondere sein distales Ende mit einem Endeffektor oder dergleichen, um die Längsachse zu drehen. Auch dies wird als "verbunden" bezeichnet.

Das Instrumentengehäuse weist zudem ein Antriebseinheit-Gehäuseteil mit einem Hohlraum auf, der zur Aufnahme einer Antriebseinheit eingerichtet bzw. in dem eine Antriebseinheit, insbesondere lösbar, aufgenommen ist, wobei das Antriebseinheit-Gehäuseteil einen Verschluss zur sterilen Abdichtung einer Einführöffnung des Hohlraums sowie eine dynamische Sterilbarriere aufweist, die den Hohlraum steril abgrenzt und über die hinweg bzw. hindurch die Antriebsstranganordnung aktuierbar ist.

Wie nachstehend weiter erläutert, kann die, insbesondere nicht sterile, Antriebseinheit durch Aufnahme in einem Hohlraum des Antriebseinheit-Gehäuseteils, der durch den Verschluss und die dynamische Sterilbarriere steril abgedichtet ist, vorteilhaft in ein robotergeführtes chirurgisches Instrument integriert werden, das entsprechenden Sterilisationsanforderungen im OP-Bereich genügen muss, ohne dass eine umständliche und beschädigungsträchtige Umhüllung der Antriebseinheit durch eine Folie, einen Schlauch oder dergleichen erforderlich ist. Unter steril wird entsprechend vorliegend insbesondere steril im medizinischen, insbesondere (mikro)chirurgischen Sinne verstanden.

Unter einer dynamischen Sterilbarriere wird vorliegend insbesondere eine Sterilbarriere verstanden, die eine Bewegung der Antriebseinheit und/oder der Antriebsstranganordnung ermöglicht und dabei zwei Seiten bzw. Räume steril gegeneinander abdichtet, insbesondere eine Sterilbarriere, durch die Kräfte und/oder Bewegungen der Antriebseinheit übertragen bzw. geführt werden können.

In einer Ausführung kann eine dynamische Sterilbarriere beweglich ausgebildet sein und eine Bewegung der Antriebseinheit und/oder der Antriebsstranganordnung mit ausführen. So kann beispielsweise eine elastisch deformierbare Membran eine translatorische Bewegung eines Kolbens der Antriebseinheit mit ausführen und mechanisch an die Antriebsstranganordnung übertragen. Gleichermaßen kann ein steril abgedichtetes, drehbares Koppelelement eine Drehbewegung einer Welle der Antriebseinheit mit ausführen und mechanisch an die Antriebsstranganordnung übertragen. In einer Ausführung grenzt die dynamische Sterilbarriere Antriebseinheit und Antriebsstranganordnung steril gegeneinander ab.

In einer anderen Ausführung kann eine dynamische Sterilbarriere eine sterile Bewegungsdichtung aufweisen, durch die ein Übertragungsmittel eines oder mehrerer Antriebsstränge der Antriebsstranganordnung hindurchgeführt ist, insbesondere eine berührungslose Dichtung wie eine Spalt- oder Labyrinthdichtung, oder eine berührende Dichtung, insbesondere eine elastische, vorgespannte Lippe oder dergleichen. Beispielsweise kann ein Zugseil oder eine Schubstange der Antriebsstranganordnung durch eine Spalt-, Labyrinth- oder streifende Lippendichtung hindurchgeführt sein, die so eine Seite der Antriebsstranganordnung steril gegen die gegenüberliegende Seite abgrenzt. In einer Ausführung grenzt die dynamische Sterilbarriere zwei Abschnitte der Antriebsstranganordnung steril gegeneinander ab.

Das Instrumentengehäuse, insbesondere das Antriebseinheit- und/oder das Antriebsstrang-Gehäuseteil, ist in einer bevorzugten Ausführung formstabil, insbesondere starr, ausgebildet. Es kann insbesondere Kunststoff und/oder Metall aufweisen, insbesondere hieraus hergestellt sein. Durch diese formstabile Ausbildung kann, wie vorstehend erläutert, die Handhabung und Integration einer nicht sterilen Antriebseinheit in ein Instrument eines Roboterchirurgiesystems erheblich verbessert, insbesondere vereinfacht werden. In einer Ausführung kann jedoch auch das Antriebseinheit-Gehäuseteil wenigstens teilflexibel ausgebildet sein. Es kann insbesondere einen formstabilen, starren Teil aufweisen, der zur Verbindung mit dem Antriebsstrang-Gehäuseteil und/oder zur Handhabung vorgesehen bzw. eingerichtet sein kann, an dem insbesondere ein oder mehrere Handgriffe, Griffmulden oder dergleichen ausgebildet sein können. Verbunden mit diesem formstabilen, starren Teil kann ein flexibler Teil des Antriebseinheit-Gehäuseteils, insbesondere ein Folienschlauch, sein, der besonders kostengünstig herstellbar ist und die Lagerung vereinfacht.

In einer Ausführung sind Antriebsstrang-Gehäuseteil und Antriebseinheit-Gehäuseteil lösbar, insbesondere formschlüssig, reibschlüssig und/oder magnetisch, insbesondere elektromagnetisch, miteinander verbunden, beispielsweise miteinander verschraubt, verrastet oder dergleichen. Dies ermöglicht eine noch einfachere sterile Handhabung der nicht sterilen Antriebseinheit, wenn diese in dem eigenständigen Antriebseinheit-Gehäuseteil aufgenommen ist und mit diesem insbesondere während einer Operation gehandhabt werden kann.

Insbesondere ermöglicht es, die lösbare Verbindung, zwei oder mehr Antriebsstrang-Gehäuseteile und/oder zwei oder mehr Antriebseinheit-Gehäuseteile vorzuhalten und wahlweise miteinander zu verbinden. So können beispielsweise zwei oder mehr gleiche oder unterschiedliche Antriebseinheit-Gehäuseteile mit gleichen oder unterschiedlichen Antriebseinheiten vorgehalten werden, um bei Bedarf ausgewechselt bzw. mit demselben Antriebsstrang-Gehäuseteil verbunden zu werden. Gleichermaßen können zwei oder mehr gleiche oder unterschiedliche Antriebsstrang-Gehäuseteile vorgehalten werden, um bei Bedarf ausgewechselt bzw. mit demselben Antriebseinheit-Gehäuseteil verbunden zu werden.

Zusätzlich oder alternativ zu einem Auswechseln des Antriebseinheit-Gehäuseteiles, insbesondere auch bei einem dauerhaft mit einem Antriebsstrang-Gehäuseteil verbundenen Antriebseinheit-Gehäuseteil, kann in einer Ausführung die lösbar in dem Hohlraum aufgenommene modulare Antriebseinheit wahlweise ausgewechselt werden. Um dabei Verwechslungen auszuschließen, können zwei oder mehr Antriebseinheiten und/oder zwei oder mehr Antriebseinheit-Gehäuseteile unterschiedliche, insbesondere mechanische, Kodierungen aufweisen. Eine mechanische Kodierung kann insbesondere durch eine komplementäre Kontur einer Antriebseinheit und einem Antriebseinheit-Gehäuseteil dargestellt werden, beispielsweise durch ineinander eingreifende Vorsprünge und Aussparungen, die unterschiedliche Formen, Größen und/oder Anordnungen aufweisen. Zusätzlich oder alternativ zu einer mechanischen Kodierung können Antriebseinheit und/oder Antriebseinheit-Gehäuseteil optische und/oder elektrische Kodierungen aufweisen, beispielsweise Schaltkreise, die nur durch das passende Gegenstück geschlossen werden oder dergleichen. Zusätzlich oder alternativ zu einer, insbesondere mechanischen, Kodierung von Paaren von Antriebseinheit und Antriebseinheit-Gehäuseteil können auch lösbar miteinander verbindbare Antriebseinheit-Gehäuseteile und Antriebsstrang-Gehäuseteile paarweise, insbesondere mechanisch, kodiert sein.

In einer Ausführung sind Antriebsstrang-Gehäuseteil und Antriebseinheit-Gehäuseteil dauerhaft miteinander verbunden, insbesondere integral miteinander ausgebildet. Dies kann vorteilhaft insbesondere ein kompakteres und/oder robusteres Instrumentengehäuse zur Verfügung stellen.

Die dynamische Sterilbarriere kann lösbar mit dem Antriebsstrang- und/oder Antriebseinheit-Gehäuseteil verbunden sein. Insbesondere kann sie in den Hohlraum im Antriebseinheit-Gehäuseteil eingeführt und dort, insbesondere form- oder reibschlüssig, fixiert sein. Dies ermöglicht eine kostengünstige Herstellung der dynamischen Sterilbarriere als Einmal-Artikel. Gleichermaßen kann die dynamische Sterilbarriere dauerhaft mit dem Antriebsstrang- und/oder Antriebseinheit-Gehäuseteil verbunden, insbesondere integriert sein, was insbesondere verhindert, dass die dynamische Sterilbarriere vergessen wird.

### Elektro-mechanische Schnittstelle

Nach einer Ausführung der vorliegenden Offenbarung weist die Instrumentenanordnung eine elektro-mechanische Schnittstelle zur lösbaren Anbindung des Instrumentengehäuses, insbesondere des Antriebsstrang-Gehäuseteils, an die Roboteranordnung auf. Unter einer elektro-mechanischen Schnittstelle wird vorliegend insbesondere ein Element verstanden, das zur mechanischen Befestigung eines Instruments an einem Roboter und zur Übertragung von elektrischer Leistung und/oder elektrischen Signalen eingerichtet ist. Ein solches Element kann lösbar, insbesondere formschlüssig oder reibschlüssig, mit dem Instrument und/oder dem Roboter verbunden, beispielsweise verschraubt oder verrastet sein.

In einer Weiterbildung ist die elektro-mechanische Schnittstelle mittels einer mechanischen Steckverbindung mit dem Instrumentengehäuse und/oder der Roboteranordnung verbunden. Hierzu kann die elektro-mechanische Schnittstelle auf der dem Instrumentengehäuse und/oder dem Roboter zugewandten Seite als Steckverbinder ausgebildet sein, der zur Steckverbindung mit einem entsprechenden Steckverbinder des Instrumentengehäuses bzw. Roboters ausgebildet ist, beispielsweise als radialer Absatz, der formschlüssig in eine Aussparung eingreift oder dergleichen.

In einer Weiterbildung kann die Schnittstelle, insbesondere formschlüssig geführt durch die Steckverbindung, eine statische Sterilbarriere des Roboters, insbesondere eine folienartige Hülle, und/oder des Instruments, insbesondere eine statische sterile Dichtung, insbesondere eine berührungslose Dichtung wie eine Spalt- oder Labyrinthdichtung, oder eine berührende Dichtung wie eine streifende Lippendichtung, öffnend durchdringen.

Ein oder mehrere elektrische Kontakte der elektro-mechanischen Schnittstelle können zugleich die mechanischen Steckverbinder bilden oder in diese integriert sein. Gleichermaßen können ein oder mehrere elektrische Kontakte der elektro-mechanischen Schnittstelle auch als berührende Kontakte, die nicht gesteckt werden, insbesondere federnde Kontaktstifte oder Blattfeder-Kontakte, die insbesondere auf eine starre Gegenfläche kontaktiert werden, ausgebildet sein.

Anstelle einer elektro-mechanischen Schnittstelle kann auch eine rein mechanische Schnittstelle vorgesehen sein, insbesondere, wenn in einer Weiterbildung eine drahtlose Energie- und/oder Signalübertragung an die Antriebseinheit vorgesehen ist.

### Verschluss

Der Verschluss zum sterilen Abdichten der Einführöffnung kann in einer Ausführung deckelartig ausgebildet sein. Er kann lösbar, insbesondere form- oder reibschlüssig oder magnetisch, insbesondere elektromagnetisch, mit dem Antriebseinheit-Gehäuseteil verbindbar sein, beispielsweise steckbar, verrastbar, verschraubbar oder dergleichen. In einer Weiterbildung erstreckt er sich über den Rand der Einführöffnung hinaus, so dass ein Bereich der Einführöffnung, an dem die nicht sterile Antriebseinheit beim Einführen in das bis dahin sterile Antriebseinheit-Gehäuseteil anstreift und diesen so kontaminiert, durch den Verschluss mit abgedichtet wird.

Der Verschluss bzw. die Einführöffnung kann auf einer dem Instrumentenschaft abgewandten Stirnseite des Instrumentengehäuses angeordnet sein, so dass die Antriebseinheit auf der dem Instrumentenschaft gegenüberliegenden Seite entnommen bzw. eingeführt werden kann. Zusätzlich oder alternativ kann, beispielsweise zur kompakteren Raumausnutzung oder besseren Handhabbarkeit, der Verschluss bzw. die Einführöffnung auf einer dem Instrumentenschaft zugewandten Stirnseite des Instrumentengehäuses neben dem Instrumentenschaft angeordnet sein. Insbesondere zur besseren Handhabbarkeit kann der Verschluss bzw. die Einführöffnung auch auf einer Mantelfläche des Instrumentengehäuses angeordnet sein, die sich vorzugsweise - wenigstens im Wesentlichen - seitlich zu einer Längsachse des Instrumentenschaftes erstrecken kann. Mit anderen Worten kann die Antriebseinheit auch seitlich - bezogen auf die Längsachse des Instrumentenschaftes - in das Antriebseinheit-Gehäuseteil eingeführt werden. Insbesondere an einem deckelartigen Verschluss kann in einer Ausführung ein Fixiermittel zur Fixierung der Antriebseinheit in dem Hohlraum angeordnet sein und diese so beim Schließen des Deckels fixieren. Zusätzlich oder alternativ können Fixiermittel an anderer Stelle des Hohlraums angeordnet sein. In einer Ausführung sind ein oder mehrere Fixiermittel spannend ausgebildet. Hierunter wird vorliegend insbesondere verstanden, dass sie die Antriebseinheit zur Fixierung, vorzugsweise elastisch, verspannen. Hierzu kann ein Fixiermittel ein elastisches Element, beispielsweise eine Anordnung von einer oder mehreren Federn, aufweisen. Zusätzlich oder alternativ können ein oder mehrere Fixiermittel verrastend ausgebildet sein und die Antriebseinheit form- und/oder reibschlüssig in dem Hohlraum fixieren.

### Antriebseinheit

Nach einem Aspekt der vorliegenden Offenbarung ist die Antriebseinheit lateral zu einer Längsachse des Instrumentenschaftes hin zu einer Anbindung des Instrumentengehäuses an die Roboteranordnung versetzt. Hierunter wird vorliegend insbesondere verstanden, dass die Antriebseinheit in einer Richtung senkrecht zur Längsachse des Instrumentenschaftes gesehen nicht mit der Längsachse fluchtet, sondern gegenüber dieser zu einer Kontaktfläche des Instruments hin versetzt ist, die zur Befestigung des Instruments an einem Roboter der Roboteranordnung vorgesehen bzw. eingerichtet ist. Die Antriebseinheit kann insbesondere in lateraler Richtung zwischen der Längsachse des Instrumentenschaftes und der Anbindung an die Roboteranordnung angeordnet sein. Gleichermaßen kann sie sich auch lateral über die Längsachse des Instrumentenschaftes hinaus erstrecken, wobei jedoch der Volumen- und/oder Massenmittel und/oder eine Symmetrieachse der Antriebseinheit vorzugsweise in lateraler Richtung zwischen der Längsachse des Instrumentenschaftes und der Anbindung an die Roboteranordnung angeordnet ist. Durch den lateralen Versatz vom Instrumentenschaft weg baut das Instrument im Bereich bzw. in Richtung seiner Längsachse vorteilhaft kleiner. Auf diese Weise können insbesondere die Längsachsen mehrerer Instrumente bzw. Instrumentenschäfte kooperierender Roboter der Roboteranordnung näher zueinander angeordnet und so auf kleinerem Raum operiert werden.

Insbesondere, um die Beweglichkeit solcher eng benachbart operierender Instrumente zu erhöhen, ist in einer Weiterbildung vorgesehen, dass wenigstens ein Instrumentengehäuse sich in lateraler Richtung zur Längsachse des Instrumentenschaftes hin verjüngt, insbesondere einen keilförmigen Querschnitt aufweist. Hierdurch kann vorteilhaft ein Schwenkbereich des Instruments um die Längsachse des Instrumentenschaftes vergrößert werden, bevor das sich verjüngende Instrumentengehäuse, bei dem es sich insbesondere um einen Antriebsstrang-Gehäuseteil handeln kann, mit einem anderen Instrument kollidiert.

### Montageverfahren

Um eine Instrumentenanordnung zu montieren, sieht die Offenbarung nach einem Aspekt vor, zunächst ein steriles Antriebseinheit-Gehäuseteil zur Verfügung zu stellen und eine Umgebung der Einführöffnung steril abzudecken, vorzugsweise mittels eines abnehmbaren Sterilschutzes. Anschließend wird die nicht sterile Antriebseinheit in den Hohlraum des Antriebseinheit-Gehäuseteils eingeführt und kontaminiert dabei diesen Hohlraum. Nun wird, gegebenenfalls nach Entfernen der sterilen Abdeckung, der Verschluss steril abgedichtet, beispielsweise ein steriler Deckel steril dichtend ein- bzw. aufgesetzt.

Auf diese Weise kann die nicht sterile Antriebseinheit in dem sterilen Antriebseinheit-Gehäuseteil aufgenommen und anschließend mit diesem gemeinsam steril gehandhabt werden.

Um ein Roboterchirurgiesystem zu montieren, sieht die Offenbarung nach einem Aspekt vor, die Roboteranordnung, insbesondere durch ein Umhüllen eines oder mehrerer Roboter mit einer folienartigen statischen Sterilbarriere, steril zu verpacken. Zusätzlich oder alternativ können ein oder mehrere Antriebseinheiten der

Instrumentenanordnung, insbesondere wie vorstehend beschrieben, steril in einem Antriebseinheit-Gehäuseteil verpackt werden, indem sie in dieses eingeführt und anschließend der Verschluss steril abgedichtet wird.

Dann wird eine mechanische Steckverbindung einer elektro-mechanischen Schnittstelle zwischen der Roboteranordnung und der Instrumentenanordnung hergestellt. Hierzu kann die elektro-mechanische Schnittstelle mittels einer mechanischen Steckverbindung mit der Roboteranordnung und/oder der Instrumentenanordnung verbunden werden, beispielsweise ein, insbesondere radialer, Absatz bzw. eine Aussparung der Schnittstelle formschlüssig in eine entsprechende Aussparung bzw. auf einen entsprechenden, insbesondere radialen, Absatz der steril verpackten Roboter- bzw. Instrumentenanordnung eingeführt bzw. aufgesetzt werden. Vorzugsweise durch diese mechanische Steckverbindung geführt, wird diese statische Sterilbarriere von der elektro-mechanischen Schnittstelle, die hierzu insbesondere einen oder mehrere elektrisch leitende Vorsprünge aufweisen kann, durchstoßen. Durch das Durchstoßen dichtet die statische Sterilbarriere weiterhin steril ab. Da dabei nur vom Sterilen ins Nicht-Sterile eingedrungen wird, wird das Sterile auch nicht kontaminiert. Allgemein ist in einer Ausführung eine mechanische Steckverbindung der elektro-mechanischen Schnittstelle einer elektrischen Steckverbindung der elektro-mechanischen Schnittstelle vorauseilend ausgebildet. Anschließend wird die elektro-mechanische Schnittstelle fixiert. Dies kann form- oder reibschlüssig insbesondere über die mechanische Steckverbindung selber erfolgen. Zusätzlich oder alternativ kann ein Verschrauben der elektro-mechanischen Schnittstelle an dem Roboter und/oder dem Instrument, insbesondere unter Durchstoßen der statischen Sterilbarriere, vorgesehen sein.

Wie vorstehend ausgeführt, kann durch die vorliegende Offenbarung ein robotergeführtes chirurgisches Instrument mit integrierter Antriebseinheit auf vorteilhafte Weise steril gehandhabt werden. In einer Ausführung sind entsprechend der Instrumentenschaft und/oder ein Instrumentengehäuse, insbesondere ein Antriebseinheit-Gehäuseteil und/oder ein Antriebsstrang-Gehäuseteil, insbesondere seine Antriebsstranganordnung, steril bzw. sterilisiert. Durch das Einführen der nicht sterilen Antriebseinheit wird lediglich der Hohlraum kontaminiert, der jedoch durch den steril abgedichteten Verschluss und die dynamische Sterilbarriere gegen die Außenumgebung steril abgegrenzt wird. Durch die elektro-mechanische Schnittstelle kann unter Wahrung der Sterilität eine elektrische Verbindung mit dem Roboter und/oder der Antriebseinheit hergestellt werden, über die elektrische Leistung und/oder Steuersignale zwischen Roboter und Antriebseinheit übertragen werden können. Gleichermaßen kann die Antriebseinheit beispielsweise drahtlos, insbesondere über wechselnde elektromagnetische Felder, etwa induktiv und/oder per Funk, mit Energie versorgt und/oder angesteuert werden. Gleichermaßen kann die Antriebseinheit einen Energiespeicher, beispielsweise eine Batterie oder einen wiederaufladbaren Akkumulator, aufweisen und/oder eine autonome Steuerung aufweisen.

Eine sterilisierbare Antriebseinheit nach einem Aspekt der vorliegenden Offenbarung weist eine Aktuatoranordnung mit einem oder mehreren Aktuatoren zur Aktuierung von einem oder mehreren Freiheitsgraden eines Endeffektors eines chirurgischen Instruments auf, in einer Ausführung eines Endoskops mit distaler Kinematik. Ein Aktuator kann in einer Ausführung zumindest einen, vorzugsweise kraft- und/oder positionsgeregelten, Elektromotor aufweisen, insbesondere zumindest ein, vorzugsweise kraft- und/oder positionsgeregelter, Elektromotor sein. Ein positionsgeregelter Elektromotor kann vorteilhaft eine teleoperative Aktuierung eines Endeffektors bei minimalinvasiver Roboterchirurgie verbessern.

Die Antriebseinheit weist weiter eine Bauteilanordnung mit einem oder mehreren elektronischen Bauteilen auf. Ein elektronisches Bauteil kann in einer Ausführung ein Positionserfassungsmittel, insbesondere ein Positionssensor, zur Ermittlung einer Position eines Aktuators der Aktuatoranordnung aufweisen, insbesondere sein, etwa ein Resolver, Inkrement- oder Absolutwertgeber oder Winkelencoder. Zusätzlich oder alternativ kann ein elektronisches Bauteil in einer Ausführung der vorliegenden Erfindung zum Verarbeiten und/oder Speichern von Daten eingerichtet sein, beispielsweise zum Filtern von Signalen oder dergleichen, es kann insbesondere ein Mikorchip oder -controller aufweisen, insbesondere sein. In einer Ausführung der vorliegenden Erfindung weist das elektronische Bauteil eine Temperaturobergrenze von höchstens 100°C, insbesondere höchstens 90°C auf.

Die Antriebseinheit weist ein sterilisierbares Gehäuse auf. Ein sterilisierbares Gehäuse kann insbesondere zur Beaufschlagung mit Heißdampf und/oder -luft mit einer Temperatur von wenigstens 100°C, insbesondere wenigstens 120°C, vorzugsweise wenigstens 130°C, insbesondere für wenigstens 5 Minuten, vorzugsweise wenigstens 20 Minuten und/oder bei einem Druck von wenigstens 2 bar, insbesondere wenigstens 3 bar vorgesehen bzw. ausgebildet sein. Das Gehäuse kann in einer Ausführung - wenigstens im Wesentlichen - zylinder- oder kastenförmig ausgebildet sein.

Das Gehäuse ist in einer Ausführung fluiddicht, insbesondere gegenüber dem vorgenannten Heißdampf und/oder luftdicht. Es kann in einer Ausführung zwei- oder mehrteilig sein, wobei in einer Weiterbildung wenigstens zwei Gehäuseteile lösbar miteinander verbindbar bzw. verbunden sein können, um Zugang zu einem Inneren des Gehäuses zu schaffen. Zwei miteinander verbundene Gehäuseteile weisen in einer Ausführung ein, insbesondere elastisches, Dichtmittel, insbesondere eine O-Ring-Dichtung auf. Sie können in einer Ausführung miteinander verschraubt, verrastet, verclipst oder dergleichen sein.

Die Aktuator- und die Bauteilanordnung sind im Inneren des Gehäuses angeordnet. In einer Ausführung ist die Aktuatoranordnung mittels eines Trägers an dem Gehäuse lösbar oder dauerhaft befestigt. Die Bauteilanordnung kann insbesondere an der Aktuatoranordnung und/oder dem Gehäuse lösbar oder dauerhaft befestigt sein.

Das Gehäuse weist eine Gehäusewand auf. Eine Gehäusewand im Sinne der vorliegenden Erfindung kann eine Außen- und/oder Innenwand des Gehäuses sein. Eine Gehäusewand im Sinne der vorliegenden Offenbarung kann das Innere des Gehäuses vollständig umschließen und hierzu mehrere, insbesondere gegeneinander abgewinkelte Wandteile aufweisen, beispielsweise die Seitenwände eines kastenförmigen Gehäuses oder die Mantelfläche eines zylinderförmigen Gehäuses sowie jeweils deren stirnseitige Deckel, von denen vorzugsweise wenigstens einer lösbar befestigt ist. Die Gehäusewand kann in einer Ausführung formsteif sein. Sie kann Metall und/oder Kunststoff aufweisen, insbesondere hieraus bestehen.

Nach einem Aspekt der vorliegenden Offenbarung ist an der Gehäusewand eine thermische Isolierschicht angeordnet. Diese kann auf einer der Bauteilanordnung zugewandten Seite der Gehäusewand angeordnet sein. Zusätzlich oder alternativ kann eine thermische Isolierschicht auf einer der Bauteilanordnung abgewandten Seite der Gehäusewand angeordnet sein. Nach einer Ausführung kann also insbesondere die Gehäusewand eine Außenwand des Gehäuses sein, auf deren dem Gehäuseinneren bzw. der Bauteilanordnung zugewandten Innenseite eine thermische Isolierschicht angeordnet ist. Gleichermaßen kann die Gehäusewand eine Innenwand des Gehäuses sein, auf deren dem Gehäuseinneren bzw. der Bauteilanordnung abgewandten Außenseite eine thermische Isolierschicht angeordnet ist. In einer Weiterbildung kann auch auf der dem Gehäuseinneren bzw. der Bauteilanordnung zugewandten Innenseite einer solchen Innenwand eine weitere thermische Isolierschicht angeordnet bzw. die Gehäuseinnenwand sandwichartig zwischen zwei thermischen Isolierschichten angeordnet sein.

Eine thermische Isolierschicht kann in einer Ausführung die innere und/oder äußere Oberfläche der Gehäusewand, wenigstens im Wesentlichen, vollständig abdecken bzw. ein Gehäuseinneres, wenigstens im Wesentlichen, vollständig umschließen. Gleichermaßen kann eine thermische Isolierschicht in einer Ausführung nur auf einem oder mehreren Teilen bzw. Abschnitten der Gehäusewand angeordnet sein, vorzugsweise wenigstens auf Höhe der Bauteilanordnung bzw. der Bauteilanordnung gegenüberliegend.

Durch eine thermische Isolierschicht auf einem oder mehreren Abschnitten der Gehäusewand kann die Wärmeleitung durch die gesamte Gehäusewand und damit eine Temperaturbeaufschlagung der Bauteilanordnung vorteilhaft reduziert werden. Deckt eine thermische Isolierschicht die Gehäusewand, wenigstens im Wesentlichen, vollständig ab, so kann der Wärmeeintrag ins Gehäuseinnere und damit auf die Bauteilanordnung insbesondere minimiert werden.

Zusätzlich oder alternativ zu einer thermischen Isolierschicht an der Gehäusewand kann nach einem Aspekt der vorliegenden Offenbarung zwischen der Bauteilanordnung und der Aktuatoranordnung eine thermische Isolierschicht angeordnet sein. Hierdurch kann vorteilhaft eine Wärmeleitung von der Aktuatoranordnung zu der Bauteilanordnung und damit eine Temperaturbeaufschlagung der Bauteilanordnung reduziert werden.

Eine thermische Isolierschicht an der Gehäusewand und/oder zwischen Bauteil- und Aktuatoranordnung kann jeweils ein- oder mehrlagig sein. Eine oder mehrere Lagen einer thermischen Isolierschicht können in einer Ausführung einen oder mehrere Thermodämmstoffe, insbesondere Mineralwolle, PUR-Hartschäume oder dergleichen, aufweisen, insbesondere hieraus bestehen. Zusätzlich oder alternativ können eine oder mehrere Lagen einer thermischen Isolierschicht eine Vakuumisolation aufweisen. Hierzu kann die jeweilige Lage zwei voneinander beabstandete Flächen aufweisen, welche zwischen sich einen fluid-, insbesondere luftdichten Raum begrenzen, der vorzugsweise mit Luft oder Gas unter Unterdruck gefüllt ist. In einer Weiterbildung kann in dem Raum ein poröser Stützkern angeordnet sein. In einer Ausführung bildet die Gehäusewand eine Fläche einer Vakuumisolationslage. In einer Weiterbildung ist die Gehäusewand, wenigstens abschnittsweise, doppelwandig ausgebildet und bildet die zwei voneinander beabstandeten Flächen einer Vakuumisolationslage.

Eine thermische Isolierschicht im Sinne der vorliegenden Offenbarung kann insbesondere bei 20°C eine Wärmeleitfähigkeit λ aufweisen, die höchstens 1 W/(K•m), insbesondere höchstens 0,5 W/(K•m), vorzugsweise höchstens 0,05 W/(K•m) beträgt. Durch eine thermische Isolierschicht an der Gehäusewand kann vorteilhaft ein Wärmeeintrag ins Gehäuseinnere während eines Sterilisierens, insbesondere mit Heißdampf oder -luft, reduziert werden. Im Betrieb kann jedoch insbesondere die Aktuatoranordnung Abwärme entwickeln, deren Abfuhr durch eine thermische Isolierschicht an der Gehäusewand nachteilig reduziert sein kann.

Insbesondere daher kann in einer Ausführung vorgesehen sein, dass - insbesondere auf Höhe einer Befestigung der Aktuatoranordnung an der Gehäusewand bzw. der Aktuatoranordnung gegenüberliegend an der Gehäusewand nur eine thermische Isolierschicht mit größerer Wärmeleitfähigkeit, insbesondere dünner und/oder mit weniger Lagen, angeordnet ist, oder die Gehäusewand in diesem Bereich ganz oder teilweise isolierschichtfrei ausgebildet ist. Auf diese Weise kann im Betrieb Abwärme der Aktuatoranordnung über deren Anbindung bzw. Befestigung an die Gehäusewand geleitet und von dieser an die Umgebung abgegeben werden.

In einer Ausführung weist, insbesondere hierzu, eine Antriebseinheit eine Wärmeleitmittelanordnung mit einem oder mehreren Wärmeleitmitteln mit einer Wärmeabgabefläche auf, welche auf einer der Aktuatoranordnung gegenüberliegenden Außenseite der Gehäusewand angeordnet ist. Die Wärmeabgabefläche kann insbesondere an einer äußeren Oberfläche des Gehäuses angeordnet sein oder von dieser abstehen. Ein Wärmeleitmittel im Sinne der vorliegenden Erfindung kann insbesondere bei 20°C eine Wärmeleitfähigkeit λ aufweisen, die wenigstens 10 W/(K•m), insbesondere wenigstens 100 W/(K•m), vorzugsweise wenigstens 200 W/(K•m) beträgt. Ein Wärmeleitmittel im Sinne der vorliegenden Erfindung kann insbesondere eine thermische Isolierschicht durchgreifen bzw. eine Wärmeabgabefläche auf der Außenseite der Gehäusewand und eine damit materialverbundene Wärmeaufnahmefläche im Inneren des Gehäuses aufweisen, um kanalisiert Wärme von der Wärmeaufnahme- zu der Wärmeabgabefläche zu übertragen.

In einer Ausführung kontaktieren ein oder mehrere Wärmeleitmittel bzw. deren Wärmeaufnahmeflächen einen Träger bzw. eine Befestigung der Aktuatoranordnung an der Gehäusewand, vorzugsweise sind sie mit einer solchen Anbindung der Aktuatoranordnung, insbesondere lösbar oder integral, verbunden. Auf diese Weise kann Abwärme der Aktuatoranordnung mittels Wärmeleitung über die Wärmeaufnahmefläche(n), von dieser mittels Wärmeleitung und/oder Konvektion an die Wärmeabgabefläche(n) geleitet und von dieser bzw. diesen an die Umgebung abgegeben werden.

Die Wärmeabgabefläche einer oder mehrerer Wärmeleitmittel kann eine gegenüber einer Grundfläche der Wärmeabgabefläche vergrößerte Oberfläche aufweisen, um die Wärmeabgabe zu vergrößern. Insbesondere kann eine Wärmeabgabefläche eine oder mehrere Kühlrippen, -lamellen und/oder -stifte aufweisen.

Die Wärmeabgabefläche einer oder mehrerer Wärmeleitmittel kann lösbar mit dem jeweiligen Wärmeleitmittel verbunden sein. Insbesondere kann eine Steck- und/oder Clipsverbindung zwischen Wärmeabgabefläche und Wärmeleitmittel ausgebildet bzw. vorgesehen sein. Dies kann es ermöglichen, eine Verbindungsfläche des Wärmeleitmittels zu der von diesem gelösten Wärmeabgabefläche, die eine kleinere Oberfläche aufweist als die Wärmeabgabefläche, und die Wärmeabgabefläche jeweils für sich zu sterilisieren, wobei aufgrund der kleineren Verbindungsfläche weniger Wärme in das Gehäuseinnere eingetragen wird. Bei befestigter Wärmeabgabefläche kann über diese hingegen Abwärme der Aktuatoranordnung stärker abgeführt werden. Gleichermaßen kann die Wärmeabgabefläche auch dauerhaft mit dem Wärmeleitmittel verbunden, insbesondere integral mit diesem ausgebildet, insbesondere urgeformt, sein.

Ein oder mehrere Wärmeleitmittel der Wärmeleitmittelanordnung, die auch als stationäre Wärmeleitmittel bezeichnet werden, können fest bzw. dauerhaft mit dem Gehäuse, insbesondere der Aktuatoranordnung, verbunden, insbesondere integral mit einem Träger der Aktuatoranordnung ausgebildet sein. Insbesondere durch eine abnehmbare Wärmeabgabefläche und/oder eine lokale thermische Kopplung mit der Aktuatoranordnung kann deren Abwärme im Betrieb abgeführt und gleichwohl der Wärmeeintrag in die Bauteilanordnung während der Sterilisierung reduziert werden.

In einer Ausführung weist die Wärmeleitmittelanordnung ein oder mehrere schaltbare Wärmeleitmittel auf, die zwischen einem ersten, wärmeleitenderen und einem zweiten, weniger wärmeleitenderen Zustand umschaltbar sind. Unter einem wärmeleitenderen Zustand im Sinne der vorliegenden Offenbarung wird insbesondere ein Zustand verstanden, in dem ein Wärmeleitmittel bei ansonsten gleichen Randbedingungen, insbesondere bei gleicher Temperaturdifferenz zwischen Gehäuseinnerem und - äußerem, von einem Wärmestrom Φ durchflossen wird, der wenigstens das 10fache, insbesondere wenigstens das 100fache des Wärmestroms in dem weniger wärmeleitenderen Zustand beträgt. Ein zweiter, weniger wärmeleitenderer Zustand im Sinne der vorliegenden Erfindung kann insbesondere ein thermisch isolierender Zustand sein, in dem das Wärmeleitmittel eine Wärmeleitfähigkeit aufweist, die höchstens 0,05 W/(K•m) beträgt.

Auf diese Weise können ein oder mehrere schaltbare Wärmeleitmittel der Wärmeleitmittelanordnung vorteilhafterweise während der Beaufschlagung mit erwärmtem Fluid in den zweiten, weniger wärmeleitenderen Zustand umgeschaltet werden, um den Wärmeeintrag während der Sterilisierung zu reduzieren, und in den ersten, wärmeleitenderen Zustand umgeschaltet werden, um Abwärme der Aktuatoranordnung während des Betriebs besser abzuführen.

In einer Ausführung können ein oder mehrere schaltbare Wärmeleitmittel einen Spalt und ein bewegliches Element zum wahlweisen wärmeleitenden Überbrücken dieses Spaltes aufweisen. Der Spalt kann insbesondere fluiddicht ausgebildet sein und in einer Weiterbildung einen Unterdruck bzw. ein Vakuum aufweisen, um seine Wärmeleitfähigkeit zu reduzieren. In einer Ausführung kann der Spalt durch eine elastische Hülle begrenzt sein, die in einer Weiterbildung eine Faltung aufweisen bzw. balgartig ausgebildet sein kann. Im ersten, wärmeleitenderen Zustand überbrückt das bewegliche Element den Spalt und erhöht so die Wärmeleitfähigkeit des Wärmeleitmittels, im zweiten, weniger wärmeleitenderen Zustand wird der Spalt nicht überbrückt und isoliert auf diese Art thermisch, so dass das Wärmeleitmittel durch Bewegen des beweglichen Elements umschaltbar ist. der Spalt kann in einer Ausführung in der thermischen Isolierschicht angeordnet bzw. ausgebildet sein.

In einer Ausführung können ein oder mehrere schaltbare Wärmeleitmittel jeweils ein oder mehrere Peltierelemente aufweisen. Unter einem Peltierelemente wird im Sinne der vorliegenden Erfindung insbesondere ein elektrothermischer Wandler verstanden, der basierend auf dem Peltier-Effekt bei Stromdurchfluss eine Temperaturdifferenz erzeugt, insbesondere ein sogenannter TEC ("thermoelectric cooler").

In einer Ausführung können ein oder mehrere Wärmeleitmittel eine Fluidpassage mit einem Arbeitsfluid aufweisen, welches Wärme mit einer Wärmeabgabefläche und einer Wärmeaufnahmefläche des Wärmeleitmittels austauschen kann. Das Arbeitsfluid kann im Betrieb insbesondere gasförmig und/oder flüssig vorliegen. Insbesondere kann das Wärmeleitmittel ein sogenanntes Wärmerohr ("heat pipe") aufweisen, insbesondere sein.

In einer Weiterbildung kann ein Wärmeleitmittel mit einer Fluidpassage mit einem Arbeitsfluid als schaltbares Wärmeleitmittel ausgebildet sein. Hierzu kann es ein Strömungsmittel zum wahlweisen aktiven Strömen und/oder Sperren des Arbeitsfluids aufweisen. Ein Strömungsmittel zum wahlweisen aktiven Strömen kann insbesondere eine steuerbare, insbesondere wahlweise aktivierbare, Umwälzpumpe zum Umwälzen des Arbeitsfluids zwischen Wärmeaufnahme- und -abgabefläche aufweisen, insbesondere sein. Durch Aktivieren oder stärkeres Umwälzen kann das Wärmeleitmittel in den ersten, wärmeleitenderen Zustand, durch Deaktivieren oder schwächeres Umwälzen in den zweiten, weniger wärmeleitenderen Zustand umgeschaltet werden. Zusätzlich oder alternativ, insbesondere bei einem umwälzpumpenlosen Wärmerohr, kann ein Strömungsmittel zum wahlweisen Sperren des Arbeitsfluids ein steuerbares, insbesondere öffnen- und schließbares, Ventil aufweisen. Zusätzlich oder alternativ kann ein schaltbares Wärmeleitmittel mit einem Wärmerohr zwei Wärmerohrabschnitte mit durch einen Spalt beabstandeten thermischen Kontaktflächen und einem beweglichen Element zum wahlweisen wärmeleitenden Überbrücken dieses Spaltes aufweisen.

Ein Chirurgierobotersystem nach einem Aspekt der vorliegenden Offenbarung weist eine Roboteranordnung mit einem oder mehreren Robotern auf, die jeweils ein chirurgisches Instrument führen, welches, insbesondere mittels eines Roboterflansches bzw. einer hierzu eingerichteten Roboterschnittstelle, lösbar mit dem jeweiligen Roboter gekoppelt ist, insbesondere mechanisch, in einer Weiterbildung auch elektrisch und/oder thermisch. Ein oder mehrere der Roboter können in einer Ausführung jeweils sechs oder mehr Freiheitsgrade, insbesondere Drehfreiheitsgrade, aufweisen. Sie können stationär oder mobil sein. Insbesondere können ein oder mehrere der Roboter, insbesondere lösbar, an einem Operationstisch befestigt sein. Zusätzlich oder alternativ können ein oder mehrere der Roboter auf einer fahrbaren Plattform befestigt sein. Das bzw. die robotergeführten Instrumente werden in einer Ausführung durch die Roboteranordnung positioniert.

Ein chirurgisches Instrument nach einem Aspekt der vorliegenden Offenbarung ist in einer Ausführung robotergeführt, indem es wie vorstehend lösbar mit einem Roboter gekoppelt bzw. hierzu eingerichtet ist, insbesondere eine hierzu eingerichtete Roboterschnittstelle aufweist. Es weist einen Instrumentenschaft, der in einer Ausführung zur teilweisen Einführung in einen Patienten, insbesondere durch einen Trokar, vorgesehen bzw. eingerichtet ist, und einen Endeffektor auf, der in einer Ausführung dazu vorgesehen ist, intrakorporal zu agieren bzw. durch eine oder mehrere chirurgische oder natürliche Öffnungen in einen Patienten eingeführt zu werden. Der Endeffektor weist in einer Weiterbildung einen, zwei, drei oder mehr Bewegungsfreiheitsgrade auf, wobei in einer Weiterbildung ein oder mehrere der Freiheitsgrade einen, insbesondere durch Anschläge, begrenzten Arbeitsbereich aufweisen können. Der Endeffektor kann beispielsweise ein Skalpell, eine Zange, Klemme, Schere oder dergleichen aufweisen, insbesondere sein. Gleichermaßen kann der Endeffektor ein optische Schnittstelle zum Aussenden und/oder Empfangen von Licht, insbesondere Laserlicht oder einem Kamerabild, und/oder eine Fluidöffnung zum Einführen und/oder Absaugen von Fluiden, insbesondere Flüssigkeiten und/oder Gasen, aufweisen, insbesondere sein.

In einer Ausführung kann der Instrumentenschaft mit der Roboteranordnung gekoppelt sein bzw. eine hierzu eingerichtete Roboterschnittstelle aufweisen. Zusätzlich oder alternativ kann die Antriebseinheit mechanisch und/oder elektrisch mit der Roboteranordnung gekoppelt sein bzw. eine hierzu eingerichtete Roboterschnittstelle aufweisen.

Die sterilisierbare Antriebseinheit, insbesondere deren Aktuatoranordnung, ist in einer Ausführung mittels einer Schnittstelle lösbar mit dem Instrumentenschaft gekoppelt. Auf diese Weise kann vorteilhaft derselbe Instrumentenschaft wahlweise mit verschiedenen Antriebseinheiten gekoppelt werden, beispielsweise, um unterschiedliche Freiheitsgrade zu aktuieren, eine Aktuierungsleistung und/odergenauigkeit zu variieren und/oder einen Energiespeicher, insbesondere einen Akkumulator, der Antriebseinheit wieder aufzuladen.

Entsprechend weist in einer Ausführung der vorliegenden Offenbarung eine sterilisierbare Antriebseinheit eine Schnittstelle zur lösbaren Kopplung der Aktuatoranordnung mit einem Instrumentenschaft eines chirurgischen Instruments auf. Antriebseinheit und Instrumentenschaft können in einer Ausführung lösbar miteinander verbunden sein, insbesondere miteinander verschraubt, geklemmt, verrastet, verclipst oder dergleichen.

Die Schnittstelle kann eine oder mehrere translatorisch und/oder rotatorisch bewegbare Abtriebsachsen der Aktuatoranordnung aufweisen. Eine translatorisch bewegbare Abtriebsachse kann insbesondere eine sterilisierbare Axialdichtung aufweisen, vorzugsweise eine schleifende Dichtung, insbesondere eine sogenannte Kolbenstangendichtung mit einem oder mehreren elastisch deformierten Elementen, die zwischen der Abtriebsachse und einer Führung elastisch komprimiert bzw. verspannt sind, wobei bei einer translatorischen Bewegung der Abtriebsachse relativ zu der Führung eine translatorische Relativbewegung zwischen dem bzw. den elastisch deformierten Elementen und der Abtriebsachse und/oder der Führung unter Schleifkontakt auftritt.

Eine rotatorisch bewegbare Abtriebsachse kann insbesondere eine sterilisierbare Radialdichtung aufweisen, vorzugsweise eine schleifende Dichtung, insbesondere eine sogenannte Radialwellendichtung mit einem oder mehreren elastisch deformierten Elementen, die zwischen der Abtriebsachse und einer Führung elastisch komprimiert bzw. verspannt sind, wobei bei einer rotatorischen Bewegung der Abtriebsachse relativ zu der Führung eine rotatorische Relativbewegung zwischen dem bzw. den elastisch deformierten Elementen und der Abtriebsachse und/oder der Führung unter Schleifkontakt auftritt.

In einer Ausführung weist die Schnittstelle eine Hülle auf, welche eine oder mehrere Durchtrittsöffnungen des Gehäuses fluiddicht abdeckt und jeweils einen diese Durchtrittsöffnung durchgreifenden Teil einer Abtriebsachse der Aktuatoranordnung umhüllt, wobei vorzugsweise die Hülle durch eine Bewegung dieses Teils der Abtriebsachse(n) elastisch deformiert wird. Die Hülle kann hierzu insbesondere eine Faltung aufweisen bzw. balgartig ausgebildet sein.

Zum Sterilisieren einer Antriebseinheit wird nach einem Aspekt der vorliegenden Offenbarung eine äußere Oberfläche der Antriebseinheit, insbesondere für eine vorgegebene Zeitdauer, insbesondere für wenigstens 5 Minuten, vorzugsweise wenigstens 20 Minuten, und/oder bei einem Druck von wenigstens 2 bar, insbesondere wenigstens 3 bar mit erwärmtem Fluid, insbesondere Dampf oder Luft, vorzugsweise mit wenigstens 100°C, insbesondere wenigstens 120°C, vorzugsweise wenigstens 130°C, beaufschlagt.

Ein oder mehrere schaltbare Wärmeleitmittel sind dabei vorzugsweise in den zweiten, weniger wärmeleitenderen Zustand geschaltet. Lösbare Wärmeabgabeflächen sind vorzugsweise von dem jeweiligen Wärmeleitmittel getrennt und können zusammen mit dem Gehäuse beaufschlagt werden.

Im Betrieb kann Abwärme der Aktuatoranordnung vorteilhaft abgeführt werden, wenn schaltbare Wärmeleitmittel in den ersten, wärmeleitenderen Zustand geschaltet sind. Hierzu weist die Antriebseinheit in einer Ausführung ein Umschaltmittel zum wahlweisen Umschalten wenigstens eines schaltbaren Wärmeleitmittels der Wärmeleitmittelanordnung in den ersten, wärmeleitenderen Zustand auf.

Das wahlweise Umschalten kann manuell erfolgen. In einer Ausführung ist das Umschaltmittel zum automatischen Umschalten, insbesondere in Abhängigkeit von einer Temperatur in einem Inneren des Gehäuses und/oder einer Arbeitsgröße der Aktuatoranordnung, ausgebildet bzw. eingerichtet. Es kann beispielsweise eine Temperatur in einem Inneren des Gehäuses erfassen und bei Überschreiten eines vorgegebenen Grenzwertes ein oder mehrere schaltbare Wärmeleitmittel in den ersten, wärmeleitenderen Zustand schalten. Gleichermaßen kann es eine Arbeitsgröße der Aktuatoranordnung, beispielsweise eine Betriebsdauer und/oder verrichtete mechanische oder elektrische Arbeit, etwa ein Integral einer von der Aktuatoranordnung aufgenommenen elektrischen Leistung, erfassen und bei Überschreiten eines vorgegebenen Grenzwertes ein oder mehrere schaltbare Wärmeleitmittel in den ersten, wärmeleitenderen Zustand schalten, da damit eine entsprechende Abwärme verbunden ist. Gleichermaßen kann die Wärmeleitmittelanordnung auch nach der Sterilisierung in den ersten Zustand geschaltet werden. Das Umschaltmittel kann insbesondere eine Bewegung eines beweglichen Elements, eine Strombeaufschlagung eines Peltierelements, eine Umwälzpumpe, ein Ventil eines schaltbaren Wärmeleitmittels steuern. In einer Ausführung weist das Umschaltmittel einen Mechanismus auf, der durch Temperierung eines Bimetalls, einer Formgedächtnislegierung oder dergleichen selbsttätig betätigt wird.

In einer Ausführung kann durch die Kopplung der Antriebseinheit mit dem Instrumentenschaft und/oder mit der Roboteranordnung ein schaltbares Wärmeleitmittel, insbesondere mechanisch, in den ersten Zustand geschaltet werden. Beispielsweise kann durch die Kopplung ein Element bewegt werden, um einen Spalt zu überbrücken, oder ein Ventil geöffnet werden, um ein Strömen von Arbeitsfluid in einem Wärmeleitmittel freizugeben.

Ein Chirurgierobotersystem nach einem Aspekt der vorliegenden Offenbarung umfasst eine Roboteranordnung mit einem oder mehreren, insbesondere zwei oder mehr gleich- und/oder zwei oder mehr verschiedenartigen Robotern. In einer Weiterbildung weisen ein oder mehrere Roboter der Roboteranordnung je wenigstens 6, insbesondere wenigstens 7 Freiheitsgrade auf, um ein robotergeführtes Instrument, insbesondere telemanipuliert, zu positionieren.

Das Chirurgierobotersystem umfasst weiter eine Instrumentenanordnung bzw. ein Instrumentensystem bzw. -satz nach einem Aspekt der vorliegenden Offenbarung, die bzw. das bzw. der ein oder mehrere, insbesondere zwei oder mehr gleich- und/oder zwei oder mehr verschiedenartige, Instrumente umfasst, die zur Befestigung an einem Roboter der Roboteranordnung eingerichtet sind, insbesondere eine Instrumentenschnittstelle zur Befestigung an einem Roboter der Roboteranordnung aufweisen. Eine Instrumentenschnittstelle ist in einer Weiterbildung zur lösbaren, insbesondere form- und/oder kraft-, insbesondere reibschlüssigen, Befestigung an einer entsprechenden, insbesondere komplementären Roboterschnittstelle der Roboteranordnung eingerichtet.

Ein oder mehrere Instrumente der Instrumentenanordnung weisen jeweils einen Instrumentenschaft auf, der zur teilweisen Einführung in einen Patienten vorgesehen ist. Hierzu kann der Schaft abschnittsweise oder über seine gesamte Länge starr oder beweglich, insbesondere gelenkig oder flexibel, ausgebildet sein und/oder eine Länge aufweisen, die wenigstens das 15fache, vorzugsweise wenigstens das 20fache seines maximalen Durchmessers beträgt. Zur kompakteren Darstellung wird auch ein proximaler Flansch des Instrumentenschaftes, der insbesondere zur Befestigung des Instruments am Roboter eine Instrumentenschnittstelle und/oder zur Befestigung einer Antriebseinheit eine entsprechende Antriebsschnittstelle aufweisen kann, als Teil des Instrumentenschaftes bezeichnet.

In einer Weiterbildung weist der Instrumentenschaft an seinem distalen Ende einen Endeffektor mit einem oder mehreren Freiheitsgraden, insbesondere ein Skalpell, eine Klemme, Zange oder Schere, einen Sender und/oder Empfänger, insbesondere eine Lichtquelle, ein Lichtleiterende und/oder eine Kamera auf. Zur Aktuierung eines oder mehrerer Freiheitsgrade des Instrumentenschafts, insbesondere von Instrumentenschaftteilen relativ zueinander und/oder von einem Endeffektor, kann in einer Weiterbildung ein Antriebsstrang in dem Instrumentenschaft angeordnet sein. Unter einem Antriebsstrang wird vorliegend insbesondere verallgemeinernd eine Anordnung zur mechanischen, pneumatischen, hydraulischen und/oder elektrischen Übertragung von Kräften und/oder Bewegungen verstanden, die in einer Weiterbildung insbesondere ein oder mehrere Zug- und/oder Druckstäbe, Seile, Bänder, Rollen, Getriebe, Hydraulikleitungen und dergleichen aufweisen kann. Diesbezüglich wird auch auf die deutschen Patentanmeldung 10 2012 008 537.0 und die internationalen Patentanmeldungen PCT/EP2012/000358 und PCT/EP2012/000719 der Anmelderin verwiesen.

Zur Aktuierung von einem oder mehreren Freiheitsgraden des Instrumentenschaftes, insbesondere eines Endeffektors, ist nach einem Aspekt der vorliegenden Offenbarung eine Antriebseinheit vorgesehen, die in einer Ausführung modular ausgebildet sein, insbesondere eine mechanische Antriebsschnittstelle zur lösbaren Verbindung mit der Antriebsstranganordnung aufweisen kann. Unter einer modularen Ausbildung wird vorliegend insbesondere eine Ausbildung derart verstanden, dass die modular ausgebildete Einheit als Ganzes bzw. als eine Baueinheit handhabbar, insbesondere mehrfach mit anderen Teilen verbind- bzw. von diesen lösbar ist, und vorzugsweise ein eigenes Gehäuse aufweist.

In einer Weiterbildung umfasst eine Instrumentenanordnung zwei oder mehr modulare Antriebseinheiten, die wahlweise mit demselben Instrumentenschaft verbindbar sind, und/oder zwei oder mehr Instrumentenschäfte, die wahlweise mit derselben modularen Antriebseinheit verbindbar sind. Insbesondere können die mechanischen Antriebsschnittstellen von einer oder mehreren modularen Antriebseinheiten und die Antriebsstranganordnungen von einem oder mehreren Instrumentenschäften aufeinander abgestimmt und verbindbar, insbesondere komplementär bzw. kongruent ausgebildet sein, beispielsweise miteinander zusammenwirkende Kupplungsmittel aufweisen.

Eine Antriebseinheit weist in einer Weiterbildung ein Antriebsteil mit einem oder mehreren Antrieben, die insbesondere je wenigstens einen Motor, insbesondere Elektromotor, ein Getriebe, Stromsensor, Referenz- und Endschalter und/oder einen Positions- und/oder Kraftsensor zur Erfassung einer Position einer Abtriebswelle bzw. einer auf eine Abtriebswelle wirkende Kraft aufweisen können, sowie ein Elektronikteil mit einem oder mehreren Steuer- und/oder Kommunikationsmitteln auf. Ein Steuermittel kann insbesondere zur Steuerung des Antriebsteils, insbesondere von dessen Antrieb(en), eingerichtet sein, ein Kommunikationsmittel zur Kommunikation mit dem Antriebsteil, insbesondere dessen Antrieb(en) und/oder Sensor(en), und/oder zur Kommunikation mit einem Roboter der Roboteranordnung, insbesondere einer (Instrumenten)Steuerung. Entsprechend kann ein Elektronikteil insbesondere die gesamte Antriebs-, insbesondere Leistungselektronik eines oder mehrerer Antriebe des Antriebsteils oder einen Teil davon aufweisen bzw. bilden. Zusätzlich oder alternativ kann ein Elektronikteil ein oder mehrere Mittel zur Signalverarbeitung, insbesondere von Sensorsignalen des Antriebsteils, aufweisen bzw. bilden.

Nach einem Aspekt der vorliegenden Offenbarung ist der Elektronikteil einer oder mehrerer Antriebseinheiten der Instrumentenanordnung modular ausgebildet und weist eine Schnittstelle zur, insbesondere elektrischen und/oder mechanischen, lösbaren Verbindung mit einem Antriebsteil der jeweiligen Antriebseinheit, eine Schnittstelle zur, insbesondere elektrischen und/oder mechanischen, vorzugsweise lösbaren, Verbindung mit dem Instrumentenschaft, und/oder eine Schnittstelle zur, insbesondere elektrischen und/oder mechanischen, vorzugsweise lösbaren, Verbindung mit der Roboteranordnung auf.

Zusätzlich oder alternativ kann auch der Antriebsteil einer oder mehrerer Antriebseinheiten der Instrumentenanordnung modular ausgebildet sein und eine Schnittstelle zur, insbesondere elektrischen und/oder mechanischen, lösbaren Verbindung mit einem Elektronikteil der jeweiligen Antriebseinheit, eine Schnittstelle zur, insbesondere elektrischen und/oder mechanischen, vorzugsweise lösbaren, Verbindung mit dem Instrumentenschaft, insbesondere dessen Antriebsstrang, und/oder eine Schnittstelle zur, insbesondere elektrischen und/oder mechanischen, vorzugsweise lösbaren, Verbindung mit der Roboteranordnung aufweisen.

Durch diese Aufteilung der mechatronischen Antriebseinheit in einen Elektronikteil und einen Antriebsteil, von denen wenigstens einer modular ausgebildet ist, entsprechend nachfolgend auch als Elektronikmodul bzw. Antriebsmodul bezeichnet wird, und eine Schnittstelle zur lösbaren Verbindung mit dem anderen von dem Elektronik- und dem Antriebsteil aufweist, kann vorteilhaft Gewicht und Volumen der vom OP-Personal handzuhabenden Bauteile reduziert und so die Bedienerfreundlichkeit des Robotersystems verbessert werden. Beispielsweise kann ein Elektronikmodul unabhängig vom Instrument mit einem an dem Instrument fest oder lösbar befestigten Antriebsteil gehandhabt werden und beispielsweise vorab am Roboter befestigt werden oder bei Entfernung des Instruments (zunächst) am Roboter verbleiben.

Aus einer Trennung sämtlicher Elektronikbaugruppen von den Motoren und Sensoren des Antriebsmoduls kann eine hohe Anzahl elektrischer Kontakte zwischen beiden Modulen resultieren. Daher können in einer Ausführung auch ein oder mehrere Steuer- und/oder Kommunikationsmittel, insbesondere Elektronik-Baugruppen, im Antriebsmodul angeordnet sein. Insbesondere kann die Anzahl der Leitungen durch eine Integration der Leistungselektronik und/oder der Stromregelung in das Antriebsmodul gemäß einer Weiterbildung der vorliegenden Offenbarung reduziert werden.

Eine Schnittstelle des Elektronikmoduls kann in einer Ausführung insbesondere die mechanische und/oder elektrische Instrumentenschnittstelle zur Befestigung des Instruments durch das Elektronikmodul an einem Roboter der Roboteranordnung bilden. Insbesondere kann das Elektronikmodul fest oder lösbar mit dem Roboter verbunden sein bzw. werden, so dass seine Schnittstelle zur Verbindung mit dem Antriebsteil und/oder mit dem Instrumentenschaft eine Instrumentenschnittstelle bildet. Ist das Elektronikmodul lösbar über eine Schnittstelle mit dem Roboter verbunden, bildet diese eine (weitere) Instrumentenschnittstelle zur Befestigung des Instruments durch das Elektronikmodul an dem Roboter.

In einer Ausführung ist das Elektronikmodul sterilisierbar ausgebildet, beispielsweise, indem seine Steuer- und/oder Kommunikationsmittel bis auf die Schnittstelle(n) in einem Gehäuse hermetisch aufgenommen, insbesondere eingeformt sind. Zusätzlich oder alternativ kann es ganz oder teilweise von einer sterilen Hülle umgeben sein, die in einer Weiterbildung auch den Roboter, mit dem das Elektronikmodul verbunden ist, ganz oder teilweise umgeben kann.

Nach einem Aspekt der vorliegenden Erfindung sind eine oder mehrere modulare manuelle Betätigungseinheiten zum wahlweisen Ersetzen einer modularen Antriebseinheit eines Instruments der Instrumentenanordnung des Chirurgierobotersystems vorgesehen. Unter einem wahlweisen Ersetzen wird vorliegend insbesondere verstanden, dass wahlweise eine manuelle Betätigungseinheit anstelle einer motorischen Antriebseinheit an dem Instrument, insbesondere seinem Instrumentenschaft, befestigt wird bzw. wenigstens eine Antriebs- und wenigstens eine Betätigungseinheit so aufeinander abgestimmt sind, dass sie gegeneinander austauschbar sind.

Insbesondere kann eine manuelle Betätigungseinheit eine mechanische Antriebsschnittstelle zur Verbindung mit einer Antriebsstranganordnung des Instruments aufweisen, die einer mechanischen Antriebsschnittstelle der wahlweise zu ersetzenden Antriebseinheit zur Verbindung mit dieser Antriebsstranganordnung entspricht. Mit anderen Worten kann die Instrumentenanordnung nach diesem Aspekt wenigstens eine manuelle Betätigungseinheit und wenigstens eine modulare Antriebseinheit aufweisen, deren mechanische Antriebsschnittstellen einander entsprechen. Die mechanischen Antriebsschnittstellen der wahlweise an dem Instrument lösbar zu befestigenden Betätigungseinheit und Antriebseinheit können insbesondere jeweils Kupplungsmittel zur Verbindung mit der Antriebsstranganordnung aufweisen, die einander in ihrer Funktion, geometrischen Konfiguration und/oder Anordnung relativ zueinander bzw. zu einem Befestigungsmittel zur lösbaren Befestigung der Betätigungs- bzw. Antriebseinheit an dem Instrument entsprechen.

Die mechanischen Antriebsschnittstellen der Betätigungs- bzw. Antriebseinheit können einander auch in der Anzahl der durch sie aktuierbaren Freiheitsgrade, beispielsweise in der Anzahl der Kupplungsmittel, insbesondere Achsen, entsprechen. Gleichermaßen ist es möglich, dass durch die Betätigungseinheit und die Antriebseinheit unterschiedliche Freiheitsgrade des Instruments, insbesondere eine unterschiedliche Anzahl Freiheitsgrade, aktuierbar ist, beispielsweise, indem an der Stelle einer oder mehrerer Achsen der Antriebsstranganordnung bei der mechanischen Antriebsschnittstelle der Betätigungs- oder der Antriebseinheit kein Kupplungsmittel vorgesehen ist, diese Achse(n) der Antriebsstranganordnung bei angeschlossener Betätigungs- bzw. Antriebseinheit sozusagen blind geschaltet, in einer Ausführung in einer, insbesondere vorgegebenen, Stellung gesperrt, sind. In einer Weiterbildung kann die Aktuierbarkeit einer oder mehrerer Freiheitsgrade der Antriebsstranganordnung durch die manuelle Betätigungseinheit wahlweise gesperrt werden, vorzugsweise, indem ein entsprechender Betätigungsfreiheitsgrad der Betätigungseinheit oder eine entsprechende Achse ihrer mechanischen Antriebsschnittstelle, insbesondere mechanisch, hydraulisch oder elektromagnetisch, wahlweise blockiert wird. In einer Ausführung weist die Betätigungseinheit hierzu eine Blockiervorrichtung mit mechanischen Elementen auf, mit denen einzelne Teile der mechanischen Antriebsschnittstelle, insbesondere Kupplungsmittel, in einer vorgegebenen Position fixierbar sind.

Ein Vorteil robotergeführter Instrumente ist die Möglichkeit, Freiheitsgrade in das distale Ende zu integrieren, um eine gegenüber manuellen laparoskopischen Instrumenten gesteigerte Beweglichkeit im Eingriffsgebiet zu erreichen. Zugleich wird dem Operateur durch die robotische Führung und Aktuierung des Instruments eine einfache Bedienung der Instrumente ermöglicht. Wenn ein Instrument, das ursprünglich zur manuellen Bedienung ausgebildet ist, an einen Roboter angebunden und mit Hilfe von dessen Freiheitsgrade und/oder einer instrumenteneigenen Antriebseinheit aktuiert wird, kann bei einer Funktionsstörung auf manuelle Operationstechnik konvertiert und mit den gleichen Instrumenten weiteroperiert werden.

Gemäß dem vorstehend genannten Aspekt der vorliegenden Offenbarung kann vorteilhaft ein Instrument mit einer robotisch optimierten Antriebsschnittstelle verwendet werden, indem die mechanische Antriebsschnittstelle der manuellen Betätigungseinheit zur Verbindung mit einer Antriebsstranganordnung des Instruments der mechanischen Antriebsschnittstelle der motorischen Antriebseinheit strukturell nachgebildet wird bzw. entspricht. Auf diese Weise wird eine menschliche Bedienerschnittstelle für ein robotergeführtes Instrument mit distaler Kinematik bereitstellt. Dies kann insbesondere den Vorteil eines einfacheren Aufbaus der Antriebseinheit, einer besseren Skalierbarkeit hinsichtlich der distalen Kinematik sowie eines einheitlichen Steuerkonzepts ergeben.

In einer Weiterbildung kann die mechanische Antriebsschnittstelle der manuellen Betätigungseinheit auch elektrische Kontakte aufweisen, über die insbesondere Informationen zwischen Betätigungseinheit und Instrument ausgetauscht werden können und/oder Energie zwischen Betätigungseinheit und Instrument übertragen werden kann, beispielweise von bzw. zu einem Sensor an einem Endeffektor des Instrumentenschaftes.

Gemäß einer Ausführung weist eine manuelle Betätigungseinheit eine Basis auf, die ein Befestigungsmittel zur lösbaren Befestigung an einem oder verschiedenen Instrumenten der Instrumentenanordnung aufweist, und an der ein Handhebel mit einem oder mehreren Freiheitsgraden gelagert ist, dessen Aktuierung, insbesondere skaliert, auf die mechanische Antriebsschnittstelle der Betätigungseinheit übertragen wird, um so den Antriebsstrang bzw. die Freiheitsgrade des mit der Betätigungseinheit lösbar verbundenen Instruments bzw. Instrumentenschaftes zu aktuieren. Unter einer skalierten Übertragung wird insbesondere eine Übertragung verstanden, bei der ein linearer oder rotatorischer Betätigungsweg über- oder untersetzt und/oder kinematisch transformiert wird, insbesondere auf eine andere Achse und/oder von einer translatorischen in eine rotatorische oder von einer rotatorischen in eine translatorische Bewegung. Das Befestigungsmittel der Basis kann dazu eingerichtet sein, mit einem, insbesondere komplementären, Befestigungsmittel des Instrumentenschaftes zusammenzuwirken, beispielsweise in Form einer Steck-, Rast- und/oder Schraubverbindung.

Nach einem Aspekt der vorliegenden Offenbarung weist die Instrumentenschnittstelle zur Befestigung eines oder mehrerer Instrumente der Instrumentenanordnung an der Roboteranordnung eine Befestigungssperre auf, die durch eine Antriebseinheit dieses Instruments lösbare ist, insbesondere durch eine an dem Instrumentenschaft befestigte Antriebseinheit, vorzugsweise nur durch eine korrekt an dem Instrumentenschaft befestigte und/oder funktionstüchtige Antriebseinheit.

Auf diese Weise kann verhindert werden, dass der Roboter ein nicht funktionstüchtiges Instrument führt.

Die Befestigungssperre kann in einer Ausführung mechanisch und/oder elektromagnetisch wirken, beispielsweise einen verstellbaren Vorsprung oder Riegel aufweisen, der im ausgefahrenen Zustand eine Befestigung an der Roboteranordnung formschlüssig verhindert. Die Befestigungssperre kann in einer Ausführung durch die Antriebseinheit mechanisch und/oder sensorisch aktuiert werden, beispielsweise, indem ein Vorsprung der Antriebseinheit einen Hebel der Befestigungssperre betätigt oder die Antriebseinheit von der Befestigungssperre sensorisch erkannt wird, insbesondere über einen mechanischen Schalter, induktiv, kapazitiv, optisch und/oder mittels RFID. Hierzu kann wenigstens eine Antriebseinheit einen RFID-Transponder aufweisen, die Befestigungssperre einen RFID-Reader.

Zusätzlich oder alternativ zu einer Befestigungssperre kann die Roboteranordnung ein Anwesenheitserkennungsmittel zum Erkennen einer Anwesenheit einer Antriebseinheit an einem robotergeführten Instrument aufweisen. Hierzu kann insbesondere, vorzugsweise an einem Roboter, insbesondere an einer Schnittstelle des Roboters zur Anbindung der Instrumentenschnittstelle des Instruments, ein, insbesondere mechanischer, induktiver, kapazitiver, optischer und/oder RFID-Sensor bzw. Reader vorgesehen sein.

Nach einem Aspekt der vorliegenden Offenbarung ist ein Instrumentenmagazin zum Speichern von einem oder mehreren Instrumenten der Instrumentenanordnung vorgesehen, so dass, insbesondere während eines OP-Betriebs, wahlweise nicht benötigte bzw. nicht robotergeführte Instrumenten der Instrumentenanordnung gespeichert bzw. gelagert werden können, insbesondere mit einer modularen Antriebseinheit versehene Instrumente. Zusätzlich oder alternativ kann das Instrumentenmagazin dazu eingerichtet sein, ein oder mehrere Instrumentenschäfte und/oder modulare Antriebseinheiten der Instrumentenanordnung separat bzw. getrennt voneinander zu speichern. In einer Ausführung kann das Instrumentenmagazin als translatorisches und/oder rotatorisches Wechselmagazin ausgebildet sein, das wahlweise durch translatorische und/oder rotatorische Bewegung verschiedene Instrumente an demselben Ort zur Aufnahme durch einen Roboter der Roboteranordnung positionieren kann.

Durch ein Instrumentenmagazin kann die Roboteranordnung, insbesondere während einer OP, einfach mit unterschiedlichen Instrumenten bestückt werden. Vorzugsweise ist hierzu eine Kontaktoberfläche des Instrumentenmagazins zur Lagerung der Instrumentenanordnung steril bzw. sterilisierbar ausgebildet oder mit einer sterilen Hülle abgedeckt. Hierbei führt der Roboter der Roboteranordnung das Ablegen bzw. das Entnehmen eines Instrumentes in das bzw. aus dem Instrumentenmagazin vorzugsweise selbsttätig durch, so dass vorteilhafterweise keine zusätzliche Hilfseinrichtung bzw. keinen zusätzlichen Roboter zum Wechseln des Instrumentes notwendig ist.

In einer Weiterbildung weist das Instrumentenmagazin ein Energiezufuhrmittel zur kontaktierenden oder kontaktfreien Energieversorgung von einem oder mehreren in dem Magazin gespeicherten Instrumenten bzw. Antriebseinheiten auf. Eine kontaktfreie Energieversorgung kann beispielsweise mittels einer transformatorischen Magnetanordnung ohne geschlossenen Eisenkern erfolgen, bei der Energiezufuhrmittel und Antriebseinheit(en) Primär- und Sekundärspule(n) aufweisen. Die Energieübertragung kann vorzugsweise im Mittelfrequenzbereich erfolgen. Durch eine kontaktierende oder kontaktfreie Energieversorgung von im Instrumentenmagazin gespeicherten Antriebseinheiten können in einer Ausführung wiederaufladbare Energiespeicher der Antriebseinheiten aufgeladen werden. Entsprechend weisen in einer Ausführung der vorliegenden Offenbarung ein oder mehrere Antriebseinheiten der Instrumentenanordnung wiederaufladbare Energiespeicher auf.

Zusätzlich oder alternativ zu der Energieversorgung können wenigstens zwei von dem Instrumentenmagazin, der Instrumentenanordnung und der Roboteranordnung, insbesondere Instrumentenmagazin und Instrumentenanordnung, ein Kommunikationsmittel zur uni- oder bidirektionalen drahtgebundenen oder drahtlosen, insbesondere induktiven oder funkbasierten, Kommunikation zwischen den wenigstens zwei von dem Instrumentenmagazin, der Instrumentenanordnung und der Roboteranordnung, insbesondere zwischen Instrumentenmagazin und Instrumentenanordnung, aufweisen. Insbesondere kann wenigstens eines von dem Instrumentenmagazin, der Instrumentenanordnung und der Roboteranordnung einen Sender und wenigstens ein anderes von dem Instrumentenmagazin, der Instrumentenanordnung und der Roboteranordnung einen Empfänger aufweisen. Hierdurch kann beispielsweise ein Status von im Instrumentenmagazin gespeicherten Instrumenten abgefragt und/oder solche Instrumente initialisiert, (re)kalibriert und/oder zurückgesetzt werden. Insbesondere können also Instrumentenmagazin und Instrumentenanordnung, Instrumentenmagazin und Roboteranordnung, insbesondere einer Steuerung der Roboteranordnung, und/oder Instrumentenmagazin und Roboteranordnung, insbesondere einer Steuerung der Roboteranordnung, dasselbe oder unterschiedliche Kommunikationsmittel aufweisen, insbesondere ein oder mehrere vorstehend beschriebene Kommunikationsmittel.

Zur Energieversorgung von an einem robotergeführten Instrument angeordneten Antriebseinheiten sind diese in einer Ausführung über eine sterile Kabelverbindung mit einer stationären Energiequelle verbunden. Zusätzlich oder alternativ kann eine kontaktfreie Energieversorgung vorgesehen sein, wie sie vorstehend bereits mit Bezug auf die Energieversorgung von in einem Instrumentenmagazin gespeicherten Antriebseinheiten und exemplarisch auch in EP 2 396 796 A1 und EP 2 340 611 A1 beschrieben sind, deren Offenbarungsgehalt vollständig in die Offenbarung der vorliegenden Erfindung einbezogen wird. Wie vorstehend beschrieben, kann zusätzlich oder alternativ zu einer Energieversorgung auch eine uni- oder bidirektionale Signalübertragung zwischen einer Antriebseinheit eines robotergeführten Instruments und einer Instrumentensteuerung vorgesehen sein, die verallgemeinernd als Teil der Roboteranordnung verstanden wird. Zusätzlich oder alternativ kann, wie vorstehend beschrieben, eine Enegr- und/oder Signalübermittlung auch über eine Schnittstelle der Roboteranordnung und eine damit verbundene Schnittstelle des Elektronik- und/oder des Antriebsteils vorgesehen sein. Daher kann nach einem Aspekt der vorliegenden Offenbarung allgemein das Chirurgierobotersystem eine sterile Kabelverbindung, eine Schnittstellenverbindung oder ein kabelloses Energie- und/oder Signalübertragungsmittel zur Energie- und/oder Signalübertragung zwischen der Instrumentenanordnung und der Roboteranordnung oder einem Instrumentenmagazin aufweisen.

Gleichermaßen ist es möglich, eine Antriebseinheit eines robotergeführten Instruments über dessen Instrumentenschnittstelle durch den Roboter mit Energie zu versorgen.

Insbesondere in letzteren Fall kann bei einem Wechsel eines Instruments bzw. einer Antriebseinheit von einem Instrumentenmagazin auf bzw. an einen Roboter und umgekehrt, d.h. bei Anbindung bzw. Ablage des Instruments bzw. der Antriebseinheit deren Energieversorgung unterbrochen werden. Insbesondere für diesen Fall weisen nach einem Aspekt der vorliegenden Offenbarung eine oder mehrere Antriebseinheiten jeweils einen elektrischen Energiespeicher zur, wenigstens temporären, autarken Energieversorgung der Antriebseinheit auf. Der Energiespeicher ist vorzugsweise so ausgebildet, dass er wenigstens während einer Wechselzeit wenigstens ein Steuer- und/oder Kommunikationsmittel eines Elektronikteils einer Antriebseinheit mit Energie versorgen kann bzw. dass er für wenigstens 10 Sekunden und/oder für höchstens 5 Minuten ein Steuer- und/oder Kommunikationsmittel mit Energie versorgen kann.

In einer Weiterbildung ist es auch möglich, den Energiespeicher so zu dimensionieren bzw. auszubilden, dass er die Antriebseinheit, insbesondere während einer OP bzw. für wenigstens 30 Minuten und/oder für höchstens 5 Stunden autark mit Energie versorgen kann. Auf diese Weise kann vorteilhaft eine kabelgebundene Energieversorgung der Instrumente mit entsprechenden räumlichen Beschränkungen und/oder Interferenzen entfallen.

Nach einem Aspekt der vorliegenden Offenbarung weist das Chirurgierobotersystem einen ersten Kommunikationskanal und einen oder mehrere weitere Kommunikationskanäle zwischen der Roboteranordnung, insbesondere einer Instrumentensteuerung, und einem oder mehreren Instrumenten der Instrumentenanordnung auf. Hierdurch kann insbesondere die Betriebssicherheit erhöht werden.

In einer Weiterbildung arbeiten ein oder mehrere dieser weiteren Kommunikationskanäle auf einem anderen physikalischen Träger bzw. Vermittler als der erste Kommunikationskanal, um nicht nur redundant, sondern diversitär zu diesem zu sein. Beispielsweise kann einer von dem ersten und einem weiteren Kommunikationskanal strom- oder spannungsbasiert ausgebildet sein, der andere von dem ersten und dem weiteren Kommunikationskanal elektromagnetisch, optisch, induktiv oder kapazitiv. Vorzugsweise ist wenigstens ein weiterer Kommunikationskanal nach dem Ruhestromprinzip bzw. derart ausgebildet, dass ein Wegfall eines Signals auf diesem Kommunikationskanal als Fehler identifiziert wird. Wenigstens ein weiterer Kommunikationskanal kann insbesondere nur zur Übertragung von Statusinformationen eingerichtet sein. Vorzugsweise wird die Roboteranordnung und/oder die Instrumentenanordnung, insbesondere die robotergeführte Instrumentenanordnung, sicher stillgesetzt, falls auf wenigstens einem weiteren Kommunikationskanal ein Fehlersignal übertragen wird, worunter verallgemeinernd, wie vorstehend beschrieben, auch der komplementäre Wegfall eines Freigabesignals verstanden wird. In einer Ausführung kann mittels des ersten und/oder eines weiteren Kommunikationskanals die Anwesenheit eines mit der Antriebseinheit bestückten Instruments übertragen bzw. erkannt werden.

Nach einem Aspekt der vorliegenden Offenbarung weist ein Chirurgierobotersystem ein ein- oder mehrteiliges, insbesondere optisches und/oder akustisches, Anzeigemittel auf zum Anzeigen eines Status eines Instruments der Instrumentenanordnung, insbesondere eines Wechselstatus und/oder Betriebsstatus. Unter einem Wechselstatus wird vorliegend insbesondere ein Status eines Instruments verstanden, das gewechselt, d.h. an einem Roboter der Roboteranordnung befestigt oder von diesem getrennt werden soll. Unter einem Betriebsstatus wird vorliegend insbesondere ein Status eines Instruments verstanden, der dessen Betriebsbereitschaft, insbesondere deren (Rest)Dauer, und/oder dessen bisherigen Betrieb, insbesondere dessen bisherige Betriebszeit oder dessen bisherige Einsatzanzahl, beschreibt.

Auf diese Weise kann dem OP-Personal ein eindeutiger und rascher Überblick zur Verfügung gestellt werden, welche Instrumente demnächst gewechselt werden bzw. zur Verfügung stehen.

Ein Aspekt der vorliegenden Erfindung betrifft ein Verfahren zum, insbesondere wahlweisen, Bestücken einer Roboteranordnung eines erfindungsgemäßen Chirurgierobotersystems mit einem oder mehreren Instrumenten der Instrumentenanordnung und/oder zum, insbesondere wahlweisen, Bestücken eines oder mehrerer solcher Instrumente mit einer Antriebseinheit.

Nach einer Ausführung umfasst das Verfahren eine Registrierung eines Instruments durch eine modulare Antriebseinheit bei Kopplung bzw. Verbindung dieser Antriebseinheit mit dem Instrument, insbesondere Instrumentenschaft. Die Registrierung kann insbesondere automatisch erfolgen. In einer Weiterbildung wird dabei, insbesondere nach dem Herstellen einer mechanischen Verbindung von Instrument und Antriebseinheit, eine Kopplung mit einem Instrument von der Antriebseinheit erkannt und ein Registrierungsvorgang zur konkreten Bestimmung des angeschlossenen Instruments aktiviert. Das Instrument kann sich selbst aktiv identifizieren bzw. aktiv Signale bzw. Daten an die Antriebseinheit übermitteln, oder passiv identifiziert werden.

Die in der Antriebseinheit registrierten Instrumentendaten, beispielsweise eine Identifikationsnummer und/oder Spezifikation des Instrumentenschaftes, etwa seine Freiheitsgrade und/oder kinematischen Parameter, insbesondere individuelle Kalibrierdaten, werden in einer Ausführung an die Roboteranordnung bzw. ihrer Instrumentensteuerung übermittelt, vorzugsweise bei Registrierung oder nach Anbindung an einen Roboter.

In einer Ausführung werden Daten registrierter Instrumente in der Roboteranordnung, insbesondere ihrer Instrumentensteuerung, insbesondere in einer Datenbank, abgespeichert und vorzugsweise periodisch oder ereignisgesteuert aktualisiert.

In einer Ausführung sind ein oder mehrere Instrumente, insbesondere Instrumentenschäfte, der Instrumentenanordnung in einem Instrumentenmagazin ohne Antriebseinheit gespeichert, die Applikation der Antriebseinheit an ein Instrument erfolgt während des Wechselvorgangs. Dafür verfügt das Instrumentenmagazin in einer Weiterbildung über einen Antriebseinheits-Manipulator zur Handhabung der Antriebseinheit während des Instrumentenwechsels. Der Antriebseinheits-Manipulator verfügt vorzugsweise über keine eigenen Bewegungsfreiheitsgrade - mit Ausnahme eines Spannmechanismus für die Antriebseinheit, sämtliche Positionierbewegungen werden von dem bzw. den instrumentenführenden Robotern der Roboteranordnung ausgeführt. Dadurch kann die Anzahl der Antriebseinheiten und/oder die Gesamtzahl der im Robotersystem enthaltenen aktuierten Freiheitsgrade reduziert werden. Zusätzlich kann dieses Konzept den Aufwand zur Energieversorgung der Antriebseinheiten reduzieren, da keine Energieversorgung der im Magazin abgelegten antriebseinheitslosen Instrumente erforderlich ist, es muss in einer Ausführung lediglich die Energieversorgung der Antriebseinheit für den Zeitraum gewährleistet sein, in dem sie nicht am Manipulatorarm adaptiert ist und von dort mit Energie versorgt wird. Die Energieversorgung der Antriebseinheit während dieses Zeitraums kann insbesondere über den Antriebseinheits-Manipulator erfolgen.

Um unterschiedliche Antriebseinheiten handhaben zu können, kann der Antriebseinheits-Manipulator optional mit mehreren Greifern, die auch unterschiedlich ausgeprägt sein können, ausgestattet sein. In einer Weiterbildung weist der Antriebseinheits-Manipulator eine oder mehrere translatorische und/oder rotatorische Bewegungsmöglichkeiten auf, um jeweils die benötigte Antriebseinheit bereitzustellen.

Zwei oder mehr der vorstehend erläuterten Aspekte und deren Ausführungen und Weiterbildungen können vorteilhafterweise miteinander kombiniert sein.

Die vorliegende Offenbarung betrifft insbesondere folgende Ausgestaltungen: Die erste Ausgestaltung betrifft ein Chirurgierobotersystem mit einer Roboteranordnung mit wenigstens einem Roboter, und einer Instrumentenanordnung mit wenigstens einem durch die Roboteranordnung geführten Instrument nach einer der Ausgestaltungen zwei bis zwölf.

Eine zweite Ausgestaltung betrifft eine Instrumentenanordnung für ein Chirurgierobotersystem nach Ausgestaltung eins, mit wenigstens einem Instrument mit einem Instrumentenschaft, der zur teilweisen Einführung in einen Patienten vorgesehen ist, einer Antriebsstranganordnung mit wenigstens einem Antriebsstrang zur Aktuierung eines Freiheitsgrades des Instrumentenschaftes, insbesondere eines Endeffektors, einer modularen Antriebseinheit zur Aktuierung der Antriebsstranganordnung und einem Instrumentengehäuse mit wenigstens einem Antriebsstrang-Gehäuseteil, an dem wenigstens ein Teil der Antriebsstranganordnung angeordnet ist und wenigstens einem Antriebseinheit-Gehäuseteil mit einem Hohlraum, der zur Aufnahme der Antriebseinheit eingerichtet ist, wobei das Antriebseinheit-Gehäuseteil einen Verschluss zur sterilen Abdichtung einer Einführöffnung des Hohlraums sowie eine dynamische Sterilbarriere aufweist, die den Hohlraum steril abgrenzt und über die hinweg die Antriebsstranganordnung aktuierbar ist.

Eine dritte Ausgestaltung betrifft eine Instrumentenanordnung nach Ausgestaltung zwei, wobei das Antriebsstrang-Gehäuseteil und das Antriebseinheit-Gehäuseteil lösbar oder dauerhaft miteinander verbunden, insbesondere integral miteinander ausgebildet sind.

Eine vierte Ausgestaltung betrifft eine Instrumentenanordnung nach Ausgestaltung drei, mit wenigstens zwei von dem Antriebsstrang-Gehäuseteil oder dem Antriebseinheit-Gehäuseteil zur wahlweisen Verbindung mit dem anderen von dem Antriebsstrang-Gehäuseteil und dem Antriebseinheit-Gehäuseteil.

Eine fünfte Ausgestaltung betrifft eine Instrumentenanordnung nach einer der vorhergehenden Ausgestaltungen zwei bis vier, mit wenigstens zwei Antriebseinheiten und/oder Antriebseinheit-Gehäuseteilen mit unterschiedlichen, insbesondere mechanischen, Kodierungen.

Eine sechste Ausgestaltung betrifft eine Instrumentenanordnung nach einer der vorhergehenden Ausgestaltungen zwei bis fünf, wobei die dynamische Sterilbarriere lösbar oder dauerhaft mit dem Antriebsstrang- und/oder Antriebseinheit-Gehäuseteil verbunden ist.

Eine siebte Ausgestaltung betrifft eine Instrumentenanordnung nach einer der vorhergehenden Ausgestaltungen zwei bis sechs, mit einer elektro-mechanischen Schnittstelle zur lösbaren Anbindung des Instrumentengehäuses, insbesondere des Antriebsstrang-Gehäuseteils, an die Roboteranordnung.

Eine achte Ausgestaltung betrifft eine Instrumentenanordnung nach Ausgestaltung sieben, wobei die elektro-mechanische Schnittstelle mittels einer mechanischen Steckverbindung, insbesondere unter Durchdringung einer statischen Sterilbarriere, mit dem Instrumentengehäuse und/oder der Roboteranordnung verbunden ist.

Eine neunte Ausgestaltung betrifft eine Instrumentenanordnung nach einer der vorhergehenden Ausgestaltungen zwei bis acht, mit einem, insbesondere spannenden, verrastenden und/oder verschlussfesten, Fixiermittel zur Fixierung der Antriebseinheit in dem Hohlraum.

Eine zehnte Ausgestaltung betrifft eine Instrumentenanordnung nach einer der vorhergehenden Ausgestaltungen zwei bis neun, wobei die Einführöffnung auf einer dem Instrumentenschaft ab- oder zugewandten Stirnseite oder einer seitlichen Mantelfläche des Instrumentengehäuses angeordnet ist.

Eine elfte Ausgestaltung betrifft eine Instrumentenanordnung, insbesondere nach einer der vorhergehenden Ausgestaltung zwei bis zehn, für ein Chirurgierobotersystem nach Ausgestaltung eins, mit wenigstens einem Instrument mit einem Instrumentenschaft, der zur teilweisen Einführung in einen Patienten vorgesehen ist, einer Antriebsstranganordnung mit wenigstens einem Antriebsstrang zur Aktuierung eines Freiheitsgrades des Instrumentenschaftes, insbesondere eines Endeffektors und einer, insbesondere modularen, Antriebseinheit zur Aktuierung der Antriebsstranganordnung, wobei die Antriebseinheit lateral zu einer Längsachse des Instrumentenschaftes hin zu einer Anbindung des Instrumentengehäuses an die Roboteranordnung versetzt ist.

Eine zwölfte Ausgestaltung betrifft eine Instrumentenanordnung nach Ausgestaltung elf, mit einem Instrumentengehäuse, welches sich in lateraler Richtung zur Längsachse des Instrumentenschaftes hin verjüngt.

Eine dreizehnte Ausgestaltung betrifft ein Verfahren zur Montage einer Instrumentenanordnung nach einer der vorhergehenden Ausgestaltungen zwei bis zwölf, mit den Schritten steriles Abdecken der Umgebung der Einführöffnung, Einführen der Antriebseinheit in den Hohlraum des Antriebseinheit-Gehäuseteils und steriles Abdichten des Verschlusses.

Eine vierzehnte Ausgestaltung betrifft ein Verfahren, insbesondere nach Ausgestaltung dreizehn, zur Montage eines Chirurgierobotersystems nach Ausgestaltung eins, mit den Schritten steriles Verpacken der Roboteranordnung und/oder wenigstens einer Antriebseinheit der Instrumentenanordnung in einem Antriebseinheit-Gehäuseteil, Herstellen einer mechanischen Steckverbindung einer elektro-mechanischen Schnittstelle zwischen der Roboteranordnung und der Instrumentenanordnung, insbesondere durch die mechanische Steckverbindung geführtes, Durchstoßen einer statischen Sterilbarriere der Roboteranordnung und/oder des Instrumentengehäuses und Fixieren der elektro-mechanischen Schnittstelle.

Eine fünfzehnte Ausgestaltung betrifft ein Chirurgierobotersystem mit einer Roboteranordnung mit wenigstens einem Roboter und einer Instrumentenanordnung mit wenigstens einem Instrument nach Ausgestaltung sechzehn, welches, insbesondere lösbar, mit der Roboteranordnung gekoppelt ist.

Eine sechzehnte Ausgestaltung betrifft ein chirurgisches Instrument mit einem Endeffektor, einer sterilisierbaren Antriebseinheit nach Ausgestaltung siebzehn sowie einem Instrumentenschaft zur Aktuierung wenigstens eines Freiheitsgrads des Endeffektors durch die Antriebseinheit.

Eine siebzehnte Ausgestaltung betrifft eine sterilisierbare Antriebseinheit für ein chirurgisches Instrument nach Ausgestaltung sechzehn, mit einer Aktuatoranordnung mit wenigstens einem Aktuator zur Aktuierung eines Freiheitsgrads eines Endeffektors des chirurgischen Instruments, einer Bauteilanordnung mit wenigstens einem elektronischen Bauteil, insbesondere einem Positionserfassungsmittel, und einem sterilisierbaren, insbesondere mehrteiligen, Gehäuse mit einer Gehäusewand, wobei an der Gehäusewand und/oder zwischen der Bauteilanordnung und der Aktuatoranordnung eine, insbesondere mehrlagige, thermische Isolierschicht angeordnet ist.

Eine achtzehnte Ausgestaltung betrifft eine sterilisierbare Antriebseinheit nach der vorhergehenden Ausgestaltung, mit einer Wärmeleitmittelanordnung mit wenigstens einem Wärmeleitmittel mit einer, insbesondere lösbaren, Wärmeabgabefläche, welche auf einer der Aktuatoranordnung gegenüberliegenden Außenseite der Gehäusewand angeordnet ist.

Eine neunzehnte Ausgestaltung betrifft eine sterilisierbare Antriebseinheit nach der vorhergehenden Ausgestaltung, wobei wenigstens ein schaltbares Wärmeleitmittel der Wärmeleitmittelanordnung zwischen einem ersten, wärmeleitenderen und einem zweiten, weniger wärmeleitenderen Zustand umschaltbar ist.

Eine zwanzigste Ausgestaltung betrifft eine sterilisierbare Antriebseinheit nach der vorhergehenden Ausgestaltung, wobei wenigstens ein schaltbares Wärmeleitmittel der Wärmeleitmittelanordnung einen, insbesondere fluiddichten, Spalt, insbesondere in der thermischen Isolierschicht, und ein bewegliches Element zum wahlweisen wärmeleitenden Überbrücken dieses Spaltes aufweist.

Eine einundzwanzigste Ausgestaltung betrifft eine sterilisierbare Antriebseinheit nach Ausgestaltung achtzehn oder einer Weiterbildung dieser Ausgestaltung, wobei wenigstens ein Wärmeleitmittel der Wärmeleitmittelanordnung eine Fluidpassage mit einem Arbeitsfluid, insbesondere ein Wärmerohr, aufweist.

Eine zweiundzwanzigste Ausgestaltung betrifft eine sterilisierbare Antriebseinheit nach der vorhergehenden Ausgestaltung, mit einem Strömungsmittel zum wahlweisen aktiven Strömen und/oder Sperren des Arbeitsfluids.

Eine dreiundzwanzigste Ausgestaltung betrifft eine sterilisierbare Antriebseinheit nach Ausgestaltung neunzehn oder einer Weiterbildung dieser Ausgestaltung, wobei wenigstens ein schaltbares Wärmeleitmittel wenigstens ein Peltierelement aufweist.

Eine vierundzwanzigste Ausgestaltung betrifft eine sterilisierbare Antriebseinheit nach Ausgestaltung achtzehn oder einer Weiterbildung dieser Ausgestaltung, wobei wenigstens ein stationäres Wärmeleitmittel der Wärmeleitmittelanordnung fest mit der Gehäusewand, insbesondere der Aktuatoranordnung, verbunden ist.

Eine fünfundzwanzigste Ausgestaltung betrifft eine sterilisierbare Antriebseinheit nach Ausgestaltung siebzehn oder einer Weiterbildung dieser Ausgestaltung, wobei die thermische Isolierschicht die innere und/oder äußere Oberfläche der Gehäusewand, wenigstens im Wesentlichen, vollständig abdeckt.

Eine sechsundzwanzigste Ausgestaltung betrifft eine sterilisierbare Antriebseinheit nach Ausgestaltung siebzehn einer Weiterbildung dieser Ausgestaltung, wobei die Schnittstelle eine Hülle, welche wenigstens eine Durchtrittsöffnung des Gehäuses fluiddicht abdeckt und einen diese Durchtrittsöffnung durchgreifenden Teil einer Abtriebsachse der Aktuatoranordnung umhüllt, wobei insbesondere die Hülle durch eine Bewegung dieses Teils elastisch deformiert wird, eine sterilisierbare Radialdichtung und/oder eine sterilisierbare Axialdichtung aufweist.

Eine siebenundzwanzigste Ausgestaltung betrifft ein Verfahren zum Sterilisieren einer Antriebseinheit nach Ausgestaltung siebzehn oder einer Weiterbildung dieser Ausgestaltung, insbesondere einer Antriebseinheit eines chirurgischen Instruments nach Ausgestaltung sechzehn, insbesondere eines Chirurgierobotersystems nach Ausgestaltung fünfzehn, wobei eine äußere Oberfläche der Antriebseinheit, insbesondere für eine vorgegebene Zeitdauer, mit erwärmtem Fluid, insbesondere Dampf oder Luft, beaufschlagt wird.

Eine achtundzwanzigste Ausgestaltung betrifft ein Verfahren nach der vorhergehenden Ausgestaltung, wobei wenigstens ein schaltbares Wärmeleitmittel der Wärmeleitmittelanordnung während der Beaufschlagung mit erwärmtem Fluid in den zweiten, weniger wärmeleitenderen Zustand umgeschaltet ist.

Eine neunundzwanzigste Ausgestaltung betrifft ein chirurgisches Instrument nach Ausgestaltung sechzehn mit einer sterilisierbare Antriebseinheit nach Ausgestaltung neunzehn oder einer Weiterbildung dieser Ausgestaltung, mit einem Umschaltmittel zum Umschalten wenigstens eines schaltbaren Wärmeleitmittels der Wärmeleitmittelanordnung in den ersten, wärmeleitenderen Zustand, insbesondere in Abhängigkeit von einer Temperatur in einem Inneren des Gehäuses und/oder einer Arbeitsgröße der Aktuatoranordnung.

Eine dreißigste Ausgestaltung betrifft eine Instrumentenanordnung für ein Chirurgierobotersystem nach einer der Ausgestaltungen neunundreißig bis fünfundvierzig, mit wenigstens einem Instrument mit einem Instrumentenschaft, der zur teilweisen Einführung in einen Patienten vorgesehen ist, einer Instrumentenschnittstelle zur Befestigung des Instruments an der Roboteranordnung, und einer, insbesondere modularen, Antriebseinheit zur Aktuierung von wenigstens einem Freiheitsgrad des Instrumentenschaftes, insbesondere eines Endeffektors, die ein Antriebsteil mit wenigstens einem Antrieb und ein Elektronikteil mit wenigstens einem Steuer- und/oder Kommunikationsmittel aufweist.

Eine einunddreißigste Ausgestaltung betrifft eine Instrumentenanordnung nach der vorhergehenden Ausgestaltung, wobei ein Elektronikteil und/oder ein Antriebsteil wenigstens einer Antriebseinheit modular ausgebildet ist und eine Schnittstelle zur Verbindung miteinander, dem Instrumentenschaft und/oder der Roboteranordnung aufweist.

Eine zweiunddreißigste Ausgestaltung betrifft eine Instrumentenanordnung nach der vorhergehenden Ausgestaltung, wobei das Elektronikteil sterilisierbar ausgebildet oder wenigstens teilweise von einer sterilen Hülle umgeben ist.

Eine dreiunddreißigste Ausgestaltung betrifft eine Instrumentenanordnung nach einer der Ausgestaltungen dreißig bis zweiunddreißig, mit einer modularen manuellen Betätigungseinheit nach einer der Ausgestaltungen sechsunddreißig und achtunddreißig zum wahlweisen Ersetzen einer modularen Antriebseinheit eines Instruments der Instrumentenanordnung des Chirurgierobotersystems.

Eine vierunddreißigste Ausgestaltung betrifft eine Instrumentenanordnung nach einer der Ausgestaltungen dreißig bis dreiunddreißig, wobei die Instrumentenschnittstelle zur Befestigung des Instruments an der Roboteranordnung eine durch eine Antriebseinheit dieses Instruments lösbare Befestigungssperre aufweist.

Eine fünfunddreißigste Ausgestaltung betrifft eine Instrumentenanordnung nach einer der Ausgestaltungen dreißig bis vierunddreißig, wobei eine Antriebseinheit einen elektrischen Energiespeicher zur, wenigstens temporären, autarken Energieversorgung der Antriebseinheit aufweist.

Eine sechsunddreißigste Ausgestaltung betrifft eine manuelle Betätigungseinheit für eine Instrumentenanordnung nach Ausgestaltung dreiunddreißig, mit einer mechanischen Antriebsschnittstelle zur Verbindung mit einer Antriebsstranganordnung des Instruments, die einer mechanischen Antriebsschnittstelle der wahlweise zu ersetzenden Antriebseinheit zur Verbindung mit dieser Antriebsstranganordnung entspricht.

Eine siebenunddreißigste Ausgestaltung betrifft eine manuelle Betätigungseinheit nach der vorhergehenden Ausgestaltung, mit einer Basis, die ein Befestigungsmittel zur lösbaren Befestigung an dem Instrument aufweist, und an der ein Handhebel mit wenigstens einem Freiheitsgrad gelagert ist, dessen Aktuierung, insbesondere skaliert, auf die mechanische Antriebsschnittstelle der Betätigungseinheit übertragen wird.

Eine achtunddreißigste Ausgestaltung betrifft eine manuelle Betätigungseinheit nach einer der Ausgestaltungen sechsunddreißig und siebenunddreißig, wobei durch die Betätigungseinheit und die Antriebseinheit, insbesondere wahlweise, unterschiedliche Freiheitsgrade des Instruments, insbesondere eine unterschiedliche Anzahl Freiheitsgrade, aktuierbar sind.

Eine neununddreißigste Ausgestaltung betrifft ein Chirurgierobotersystem mit einer Roboteranordnung mit wenigstens einem Roboter und einer Instrumentenanordnung nach einer der Ausgestaltungen dreißig bis fünfunddreißig.

Eine vierzigste Ausgestaltung betrifft ein Chirurgierobotersystem nach der vorhergehenden Ausgestaltung, mit einem ersten Kommunikationskanal und wenigstens einem weiteren Kommunikationskanal zwischen der Roboteranordnung und wenigstens einem Instrument der Instrumentenanordnung.

Eine einundvierzigste Ausgestaltung betrifft ein Chirurgierobotersystem nach einer der Ausgestaltungen neununddreißig bis vierzig, mit einer sterilen Kabelverbindung, einer Schnittstellenverbindung oder eines kabellosen Energie- und/oder Signalübertragungsmittel zur Energie- und/oder Signalübertragung zwischen der Roboteranordnung und der Instrumentenanordnung.

Eine zweiundvierzigste Ausgestaltung betrifft ein Chirurgierobotersystem nach einer der Ausgestaltung neununddreißig bis einundvierzig, mit einem Instrumentenmagazin zum wahlweisen Speichern von wenigstens einem von wenigstens zwei Instrumenten der Instrumentenanordnung.

Eine dreiundvierzigste Ausgestaltung betrifft ein Chirurgierobotersystem nach der vorhergehenden Ausgestaltung, mit einem Antriebseinheits-Manipulator zum wahlweisen Bestücken verschiedener Instrumente der Instrumentenanordnung mit einer modularen Antriebseinheit.

Eine vierundvierzigste Ausgestaltung betrifft ein Chirurgierobotersystem nach einer der Ausgestaltungen neununddreißig bis dreiundvierzig, wobei das Instrumentenmagazin, die Instrumentenanordnung und/oder die Roboteranordnung ein Kommunikationsmittel zur Kommunikation zwischen wenigstens zwei von dem Instrumentenmagazin, der Instrumentenanordnung und der Roboteranordnung, insbesondere zwischen Instrumentenmagazin und Instrumentenanordnung, aufweisen.

Eine fünfundvierzigste Ausgestaltung betrifft ein Chirurgierobotersystem nach einer der Ausgestaltungen neununddreißig und vierundvierzig, mit einem Anzeigemittel zum Anzeigen eines Status eines Instruments der Instrumentenanordnung, insbesondere eines Wechselstatus und/oder Betriebsstatus.

Eine sechsundvierzigste Ausgestaltung betrifft ein Verfahren zum, insbesondere wahlweisen, Bestücken einer Roboteranordnung eines Chirurgierobotersystems nach einer der vorhergehenden Ausgestaltungen mit wenigstens einem Instrument der Instrumentenanordnung des Chirurgierobotersystems.

Eine siebenundvierzigste Ausgestaltung betrifft ein Verfahren zum, insbesondere wahlweisen, Bestücken wenigstens eines, insbesondere robotergeführten und/oder in einem Instrumentenmagazin gespeichertes, Instruments eines Chirurgierobotersystems nach einer der vorhergehenden Ausgestaltungen mit einer Antriebseinheit.

Weitere Vorteile und Merkmale ergeben sich aus den Unteransprüchen und den Ausführungsbeispielen. Hierzu zeigt, teilweise schematisiert:
- Fig. 1:: ein Instrument einer Instrumentenanordnung nach einer Ausführung der vorliegenden Erfindung;
- Fig. 2:: ein Instrument einer Instrumentenanordnung nach einer weiteren Ausführung der vorliegenden Erfindung in Fig. 1 entsprechender Darstellung;
- Fig. 3:: ein Instrument einer Instrumentenanordnung nach einer weiteren Ausführung der vorliegenden Erfindung in Fig. 1, 2 entsprechender Darstellung;
- Fig. 4,4A:: ein Roboterchirurgiesystem nach einer Ausführung der vorliegenden Erfindung mit dem Instrument der Fig. 1 in perspektivischer Explosionsdarstellung;
- Fig. 5:: das Roboterchirurgiesystem nach Fig. 4 im montierten Zustand;
- Fig. 6, 7:: Details zur Anbindung des Instruments des Roboterchirurgiesystem nach Fig. 4, 5 in perspektivischer Darstellung (Fig. 6) bzw. in einem Querschnitt (Fig. 7);
- Fig. 8:: ein Verfahren nach einer Ausführung der vorliegenden Erfindung zur Montage einer Instrumentenanordnung nach Fig. 1, 4 und 5.
- Fig. 9:: eine dynamische Sterilbarriere der Instrumentenanordnung nach Fig. 1, 4 und 8;
- Fig. 10:: unterschiedliche mechanische Kodierungen von Antriebseinheiten der Instrumentenanordnung nach einer der Fig. 1 bis 8 und 11(a), 11(b);
- Fig. 11(a), 11(b):: ein Instrument einer Instrumentenanordnung nach einer weiteren Ausführung der vorliegenden Erfindung in Fig. 1 entsprechender Darstellung mit von einem Antriebsstrang-Gehäuseteil getrennten (Fig. 11(a)) bzw. mit diesem verbundenen (Fig. 11 (b)) Antriebseinheit-Gehäuseteil;
- Fig. 12(a):: ein Roboterchirurgiesystem nach einer weiteren Ausführung der vorliegenden Offenbarung in perspektivischer Darstellung;
- Fig. 12(b):: eine Draufsicht auf ein Operationsfeld des Roboterchirurgiesystems nach Fig. 12(a);
- Fig. 13:: ein Verfahren zum Montieren des Roboterchirurgiesystems nach Fig. 4 bis 7;
- Fig. 14:: den Ablauf des Verfahrens nach Fig. 8;
- Fig. 15:: eine sterilisierbare Antriebseinheit eines chirurgischen Instruments eines Chirurgierobotersystems nach einer weiteren Ausführung der vorliegenden Offenbarung;
- Fig. 16 - 24:: jeweils eine sterilisierbare Antriebseinheit eines chirurgischen Instruments eines Chirurgierobotersystems nach weiteren Ausführungen der vorliegenden Offenbarung;

- Fig. 25:: einen Teil der sterilisierbare Antriebseinheit nach einer der Fig. 15 bis 24;
- Fig. 26:: ein Chirurgieroboter-System nach einer weiteren Ausführung der vorliegenden Offenbarung;
- Fig. 27A, 27B:: ein robotergeführtes Instrument einer Instrumentenanordnung nach einer Ausführung der vorliegenden Offenbarung;
- Fig. 28:: ein robotergeführtes Instrument einer Instrumentenanordnung nach einer Ausführung der vorliegenden Offenbarung;
- Fig. 29:: eine manuelle Betätigungseinheit einer Instrumentenanordnung nach einer Ausführung der vorliegenden Offenbarung;
- Fig. 30A, 30B:: das Chirurgieroboter-System der Fig. 27A in anderer Blickrichtung (Fig. 30A) bzw. bei nicht befestigter Antriebseinheit (Fig. 30B);
- Fig. 31:: ein Chirurgierobotersystem nach einer Ausführung der vorliegenden Offenbarung mit einem Instrumentenmagazin; und
- Fig. 32(a) bis 33(d):: ein erfindungsgemäßes Verfahren zum automatischen Bestücken einer Roboteranordnung mit einem Instrument der Instrumentenanordnung und eines Instruments dieser Instrumentenanordnung mit einer Antriebseinheit nach einer Ausführung der vorliegenden Erfindung.

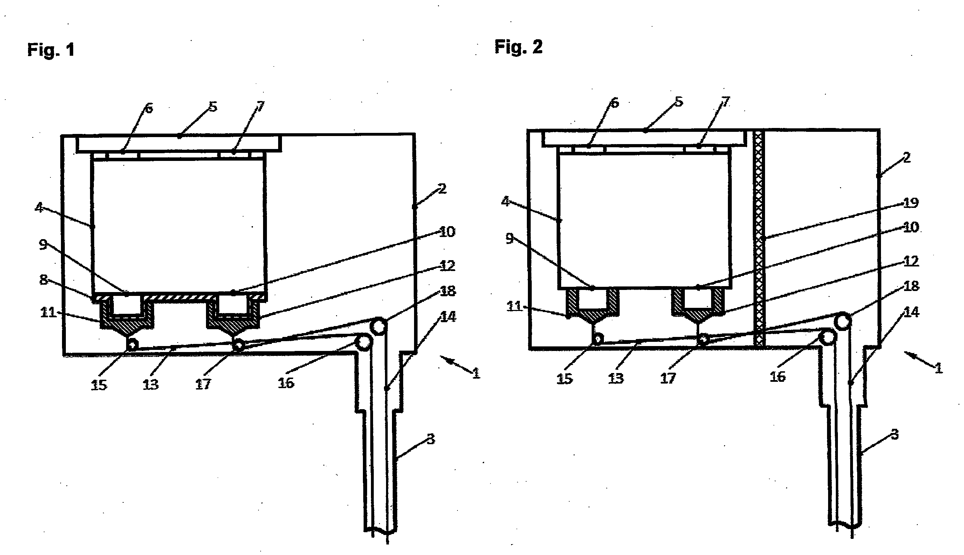

Fig. 1 zeigt ein Instrument 1 einer Instrumentenanordnung nach einer Ausführung der vorliegenden Erfindung in einem Querschnitt mit einem Instrumentengehäuse 2, welches einen Instrumentenschaft 3, einen hiermit integral ausgebildeten Antriebsstrang-Gehäuseteil, in dem eine nachfolgend näher erläuterte Antriebsstranganordnung 11 bis 18 angeordnet ist, und einen hiermit integral ausgebildeten Antriebseinheit-Gehäuseteil mit einem Hohlraum aufweist, in dem eine Antriebseinheit 4 aufgenommen ist. Ein deckelartiger Verschluss 5 dichtet eine Einführöffnung des Hohlraums steril ab. Eine dynamische Sterilbarriere 8, über die hinweg die Antriebsstranganordnung aktuierbar ist, grenzt den Hohlraum steril gegen die Umgebung ab. In einer Abwandlung kann zwischen dem Instrumentengehäuse 2 und dem Instrumentenschaft 3 ein, insbesondere aktuierter und/oder rotatorischer, Freiheitsgrad vorgesehen bzw. ausgebildet sein.

Die Antriebseinheit 4 stellt die mechanische Antriebsleistung für alle aktiven Freiheitsgrade des chirurgischen Instruments 1 zur Verfügung. Sie ist am proximalen Ende des Instruments angeordnet und als eigenständiges Modul konzipiert, deren Verbindung mit dem Instrument gelöst werden kann. Anzahl der aktiven Freiheitsgrade, Stellbereich und Leistungsdaten sind universell ausgelegt, sodass die Antriebseinheit austauschbar ist und sich für den Antrieb unterschiedlicher Instrumente eignet. Gleichermaßen ist es denkbar, verschiedene Antriebseinheiten bereitzustellen, beispielsweise um spezielle Instrumente mit einer größeren Anzahl aktiver Freiheitsgrade oder anderen Besonderheiten mit dem Robotersystem einsetzen zu können.

Die Antriebseinheit 4 wird mit Hilfe einer Antriebsschnittstelle am chirurgischen Instrument in der gewünschten Position positioniert und fixiert. Sie enthält außerdem eine Mehrzahl lösbarer Kupplungen, die den Kraftfluss zwischen den Einzelantrieben der Antriebseinheit und instrumentenseitigen Antriebssträngen der Antriebsstranganordnung herstellen. Die lösbaren Kupplungen in der Antriebsschnittstelle können für Dreh- bzw. rotatorische oder translatorische Stellbewegungen ausgelegt sein, es sind auch beliebige Kombinationen der beiden Prinzipien möglich.

Ein Roboterchirurgiesystem umfasst üblicherweise einige nicht-sterile Komponenten, beispielsweise einen Roboter(arm) und die Antriebseinheit. Diese Komponenten werden mit Sterilbarrieren von den sterilen Komponenten des Roboterchirurgiesystems abgeschirmt, um eine Kontamination des Operationsfeldes zu verhindern. Eine statische Sterilbarriere ist in einer Ausführung als einmalig verwendbarer Folienschlauch ausgeführt.

In der Ausführung der Fig. 1 bilden Instrument 1 und Antriebseinheit 4 während eines operativen Eingriffs eine integrale Einheit. Dabei ist die Antriebseinheit 4 zur Längsachse (vertikal in Fig. 1) des Instrumentenschaftes 3, im Folgenden auch kurz als Schaftachse bezeichnet, lateral versetzt angeordnet und durch eine separate dynamische Sterilbarriere 8 vom sterilen Teil des Instruments 1 abgeschirmt. Somit fungiert die trennbare Instrumenten-Schnittstelle zugleich als Sterilbarriere zwischen der nicht sterilen Antriebseinheit 4 und dem sterilen Instrument 1. Die separate dynamische Sterilbarriere 8 kann nach einem Eingriff aus dem Instrument 1 entfernt und als Einmalartikel oder als aufbereitbares Bauteil ausgebildet sein.

Am proximalen Ende des Instruments 1 befindet sich das Gehäuse 2, in dessen Antriebsschaft-Gehäuseteil die Antriebsstränge der Kinematik und des Endeffektors untergebracht sind, die sich am distalen Ende des Instrumentenschafts 3 befinden. Der hiermit integral ausgebildete Antriebseinheit-Gehäuseteil weist einen Hohlraum zur Aufnahme der nicht-sterilen Antriebseinheit 4 auf. Der Verschlussdeckel 5 schirmt die nicht sterile Antriebseinheit 4 vom sterilen Teil des Instruments 1 ab. Eine mechanische Fixierung der Antriebseinheit 4 im Gehäuse 2 kann beispielsweise über ein oder mehrere am Verschlussdeckel 5 angeordnete Fixiermittel bzw. -elemente 6, 7 erfolgen. Diese Elemente können beispielsweise als lineare oder viskoelastische Federn ausgeführt sein und so eine Vorspannkraft zwischen Deckel und Antriebseinheit erzeugen. Zusätzlich oder alternativ kann die Antriebseinheit 4 mit Spann- oder Rastmechanismen formschlüssig im Gehäuse 2 fixiert werden.

Der Hohlraum im Gehäuse 2 ist vor dem Einsetzen der Antriebseinheit 4 steril, durch das Einsetzen der nicht sterilen Antriebseinheit 4 wird er jedoch kontaminiert. Deshalb ist in dieser Ausführung eine separate Sterilbarriere 8 zwischen der Antriebseinheit 4 und dem Boden des Hohlraums integriert, der den kontaminierten Hohlraum vom sterilen Rest des Instruments 1 abschirmt bzw. -grenzt. Die dynamische Sterilbarriere 8 enthält mehrere Bewegungsübertragungselemente für die Einzelantriebe 9 und 10 der Antriebseinheit, die als Dreh- und/oder Linearantriebe ausgelegt sein können. Beim Einsetzen der Antriebseinheit in das Gehäuse 2 werden die Einzelantriebe 9 bzw. 10 mit instrumentenseitigen Kupplungselementen 11 bzw. 12 der Antriebsstränge und darüber mittels Seilzügen 13,14 mit der distalen Instrumentenkinematik und dem distalen Endeffektor (nicht dargestellt) verbunden. Im Ausführungsbeispiel werden die die Instrumentenkinematik und den Endeffektor aktuierenden Seilzüge 13,14 über Umlenkrollen 15 und 16 bzw. 17 und 18 in den Instrumentenschaft geführt.

In der schematischen Darstellung der Ausführung nach Fig. 1 wird die Antriebseinheit 4 proximal bzw. von der dem Instrumentenschaft 3 abgewandten Seite in den Hohlraum des Gehäuses 2 eingeführt. Alternativ sind auch Instrumente denkbar, in die die Antriebseinheit 4 distal bzw. von der dem Instrumentenschaft 3 zugewandten Seite oder seitlich bzw. durch eine Mantelfläche des Gehäuses 2 eingeführt wird.

Fig. 2 zeigt ein Instrument einer Instrumentenanordnung nach einer weiteren Ausführung der vorliegenden Erfindung in Fig. 1 entsprechender Darstellung. Einander entsprechende Elemente sind durch identische Bezugszeichen bezeichnet, so dass hierzu auf die vorstehende Beschreibung Bezug genommen und nachfolgend nur auf die Unterschiede eingegangen wird.

Bei der Ausführung nach Fig. 2 ist die Sterilbarriere 19 zur Abschirmung der nicht sterilen Antriebseinheit 4 vom sterilen Instrument in das Instrument integriert und wird somit zusammen mit dem Instrument aufbereitet. Der Hohlraum im Gehäuse 2 ist vor dem Einsetzen der Antriebseinheit 4 steril, durch das Einsetzen der nicht sterilen Antriebseinheit 4 wird er jedoch kontaminiert. Deshalb ist in das Gehäuse 2 eine dynamische Sterilbarriere 19 integriert, die den kontaminierten Hohlraum vom sterilen Teil des Instruments 1 abschirmt. Die integrierte dynamische Sterilbarriere 19 kann beispielsweise Spalt- oder Labyrinthdichtungen oder berührende Dichtungen aufweisen, durch die die instrumentenseitigen Antriebsstränge, hier deren Seilzüge 13, 14 hindurchgeführt werden. Wie bei der Ausführung der Fig. 1 mit der separaten, auswechselbaren dynamischen Sterilbarriere 8 ist die Antriebsstranganordnung somit über die dynamische Sterilbarriere hinweg aktuierbar, insbesondere kann sie Kräfte und Bewegungen durch die dynamische Sterilbarriere hindurch übertragen, während diese den durch die nicht sterile Antriebseinheit 4 kontaminierten Hohlraum gegen die sterile Umgebung steril abgrenzt.

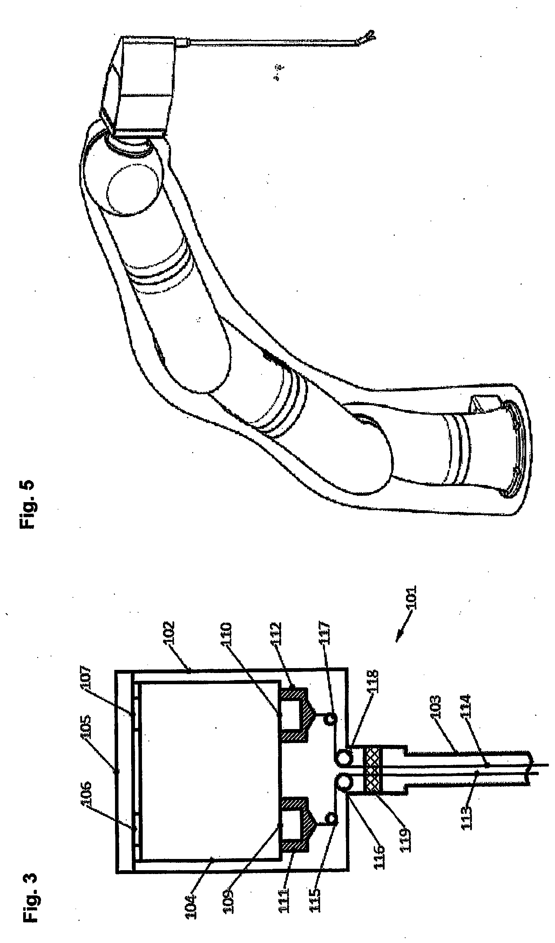

Fig. 3 zeigt ein Instrument einer Instrumentenanordnung nach einer weiteren Ausführung der vorliegenden Erfindung in Fig. 1, 2 entsprechender Darstellung. Einander entsprechende Elemente sind durch identische Bezugszeichen bezeichnet, so dass hierzu auf die vorstehende Beschreibung Bezug genommen und nachfolgend nur auf die Unterschiede eingegangen wird.

Bei der Ausführung nach Fig. 3 befindet sich am proximalen Ende des Instruments 101 ein Gehäuse 102 mit einem Instrumentenschaft 3, einem hiermit integral ausgebildeten Antriebsstrang-Gehäuseteil, in dem die Antriebsstränge der Kinematik und des Endeffektors untergebracht sind, die sich am distalen Ende des Instrumentenschafts 103 befinden, und einem hiermit integral ausgebildeten Antriebseinheit-Gehäuseteil mit einem Hohlraum zur Aufnahme einer nicht sterilen Antriebseinheit 104. Ein Verschlussdeckel 105 schirmt die nicht sterile Antriebseinheit 4 vom sterilen Teil des Instruments 101 bzw. der Umgebung ab bzw. dichtet den Hohlraum steril ab. Eine mechanische Fixierung der Antriebseinheit 104 im Gehäuse 102 kann beispielsweise über ein oder mehrere am Verschlussdeckel 105 angeordnete Fixiermittel bzw. -elemente 106,107 erfolgen. Diese Elemente können beispielsweise als lineare oder viskoelastische Federn ausgeführt sein und so eine Vorspannkraft zwischen Deckel und Antriebseinheit erzeugen. Zusätzlich oder alternativ kann die Antriebseinheit 104 mit Spann- oder Rastmechanismen formschlüssig im Gehäuse 102 fixiert werden.

Die Antriebseinheit weist mehrere Einzelantriebe 109 und 110 auf, die als Dreh- und/oder translatorische bzw. Linearantriebe ausgebildet sein können. Beim Einsetzen der Antriebseinheit in das Gehäuse 102 werden die Einzelantriebe 109 bzw. 110 mit den jeweiligen instrumentenseitigen Kupplungselementen 111 bzw. 112 der Antriebsstränge und über deren Seilzüge 113 bzw. 114 mit der distalen Instrumentenkinematik und dem distalen Endeffektor verbunden (nicht dargestellt).

In der Ausführung der Fig. 3 werden Instrumentenkinematik und Endeffektor mit den Seilzügen 113 und 114 aktuiert, die jeweils über Umlenkrollen 115 und 116 bzw. 117 und 118 in den Instrumentenschaft geführt sind. Der Hohlraum im Gehäuse 102 ist vor dem Einsetzen der Antriebseinheit 104 steril, durch das Einsetzen der nicht-sterilen Antriebseinheit 104 wird er jedoch kontaminiert. Deshalb ist in das Gehäuse 102 eine Sterilbarriere 119 integriert, die den kontaminierten Hohlraum vom sterilen Teil des Instruments 101 bzw. der Umgebung abschirmt. Die integrierte dynamische Sterilbarriere 119 kann, wie vorstehend mit Bezug auf Fig. 2 erläutert, beispielsweise in Form von Spalt- oder Labyrinthdichtungen oder als berührende Dichtungen der instrumentenseitigen Antriebsstränge integriert werden.

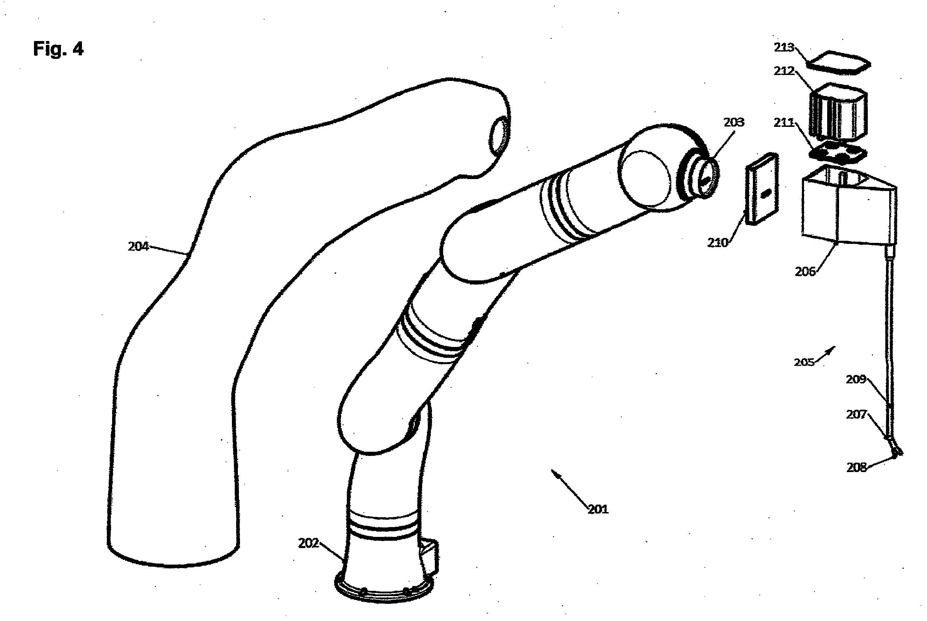

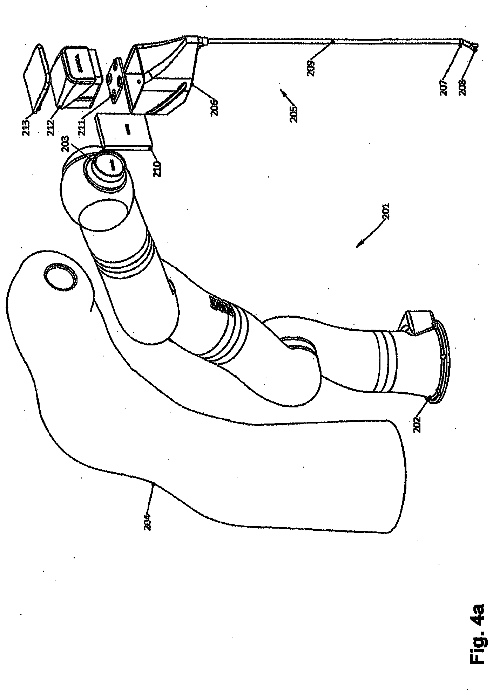

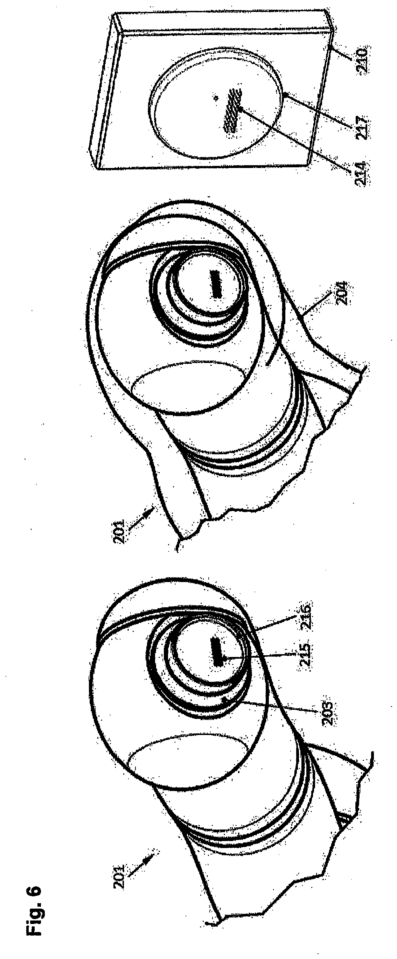

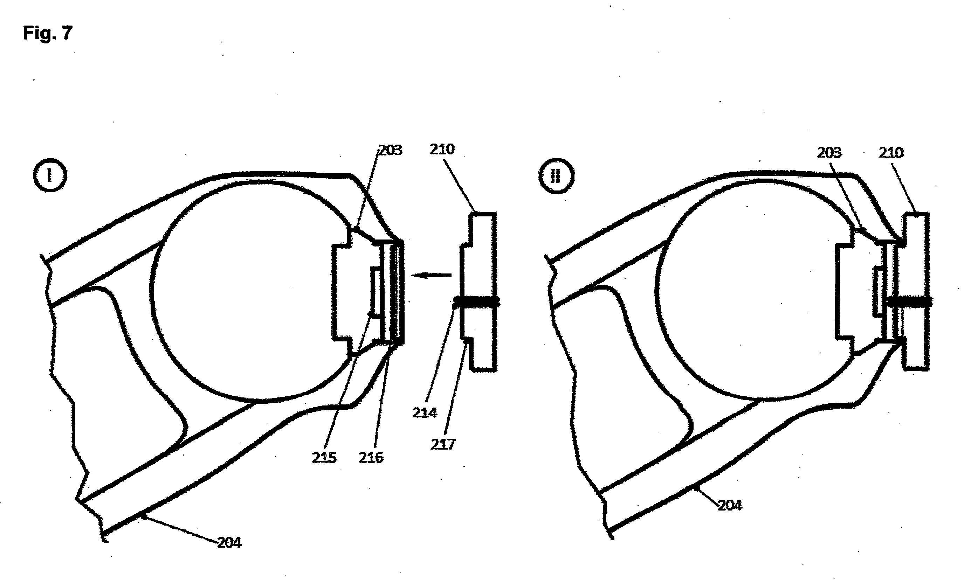

Fig. 4 zeigt ein Roboterchirurgiesystem nach einer Ausführung der vorliegenden Erfindung mit dem Instrument der Fig. 1 in perspektivischer Explosionsdarstellung, Fig. 5 das Roboterchirurgiesystem im montierten Zustand, Fig. 6 und 7 Details zur Anbindung des Instruments in perspektivischer Darstellung (Fig. 6) bzw. in einem Querschnitt (Fig. 7).

Das Roboterchirurgiesystem weist einen Roboter bzw. Manipulatorarm 201 auf, dessen proximale Basis 202 relativ zu einem Patienten (nicht dargestellt) fixiert sein kann. Der Manipulatorarm enthält vorzugsweise 6 oder mehr aktuierte Gelenke, um das distale Ende 203 des Manipulatorarms 201 frei im Raum positionieren zu können. Der Manipulatorarm 201 einschließlich seines distalen Endes 203 ist von einer statischen Sterilbarriere in Form einer sterilen Hülle 204 umgeben, um eine Kontamination des sterilen Operationsfeldes durch nicht sterile Roboterkomponenten zu verhindern. Am solcherart steril umhüllten distalen Ende 203 wird ein chirurgisches Instrument 205 mittels einer sterilen elektro-mechanischen Schnittstelle, nachfolgend auch als Instrumentenadapters 210 bezeichnet, fixiert.

Das chirurgische Instrument 205 entspricht dem vorstehend mit Bezug auf Fig. 1 beschriebenen Instrument 1, so dass ergänzend auf dessen Beschreibung Bezug genommen wird.

Am proximalen Ende des Instruments 205 befindet sich ein Instrumentengehäuse 206, das eine mechanische Schnittstelle zum Instrumentenadapter 210 aufweist. Im gezeigten Ausführungsbeispiel befindet sich distal am Gehäuse 206 ein Instrumentenschaft 209, der eine distale Instrumentenkinematik 207 und einen chirurgischen Endeffektor 208 trägt und integral mit einem Antriebseinheit-Gehäuseteil und einem hiermit integral ausgebildeten Antriebsstrang-Gehäuseteil ausgebildet oder separat an dem Antriebsstrang-Gehäuseteil befestigt ist. Antriebseinheit-Gehäuseteil und Antriebsstrang-Gehäuseteil bilden zusammen das in dieser Ausführung einstückige Instrumentengehäuse 206.

Im Ausführungsbeispiel der Fig. 4 bis 7 wird die Antriebseinheit 212 zusammen mit einer separaten dynamischen Sterilbarriere 211 proximal in das Gehäuse 206 eingeführt. Ein Verschlussdeckel 213 schirmt die nicht sterile Antriebseinheit 212 vom sterilen Operationsfeld ab.

Eine Detailansicht einer möglichen Ausführungsform der Verbindung zwischen dem sterilen Instrumentenadapter 210 und dem distalen Ende 203 des Manipulatorarms 201 ist in Abbildung 6 dargestellt, mit der eine luftdichte Trennung des Manipulatorarms 201 vom sterilen Operationsfeld erreicht wird. Zudem zeichnet sich diese Ausführungsform durch eine besonders einfache Ausführung der sterilen Hülle 204 aus, die aus einem dünnwandigen Folienschlauch, beispielsweise aus Kunststofffolie, gefertigt sein kann. Vorzugsweise ist die Umhüllung des distalen Endes 203 des Manipulatorarms 201 als dünnwandiges Kunststoff-Formteil hergestellt, um die Applikation der sterilen Hülle 204 auf den Manipulatorarm 201 zu vereinfachen. Dieses Formteil kann beispielsweise aus einer Kunststofffolie tiefgezogen oder blasgeformt werden und wird anschließend mit dem dünnwandigen Folienschlauch verschweißt. Durch die einfache Herstellung der sterilen Umhüllung 204 verringern sich für den Anwender die laufenden Kosten für Einmalartikel. Da sich die Antriebseinheit 212 bei den erfindungsgemäßen Ausführungsbeispielen nicht innerhalb der sterilen Hülle 204 angeordnet ist, ist keine Durchführung der Antriebsbewegungen durch die sterile Hülle 204 zum Instrument 205 erforderlich. Müssen für den Betrieb und zur Ansteuerung nötige elektrische Signale und Energie zur Antriebseinheit 212 und damit durch die sterile Hülle 204 geführt werden, sind mehrere Steckbuchsen 215 in eine Aussparung 216 am distalen Ende 203 des Manipulatorarms 201 integriert. Analog sind mehrere Kontaktpins 214 in einen radialen Absatz 217 des sterilen Instrumentenadapters 210 integriert. Aussparung 216 und radialer Absatz 217 zwischen dem distalen Ende 203 des Manipulatorarms und dem sterilen Instrumentenadapter 210 bilden eine mechanische Steckverbindung, die Steckbuchsen 215 und Kontaktpins 214 eine elektrische Verbindung der elektro-mechanischen Schnittstelle 210.

Die mechanische Steckverbindung 216, 217 ist als formschlüssige Verbindung ausgelegt, bei der die Wandstärke der dazwischen liegenden statischen Sterilbarriere in Form der sterilen Hülle 204 berücksichtigt ist, um eine möglichst exakte und kippsteife Verbindung zwischen dem sterilen Instrumentenadapter 210 und dem distalen Ende 203 des Manipulatorarms 201 zu erreichen. Weiterhin ist die Verbindung zwischen den Schnittstelle 216 und 217 voreilend gegenüber der elektrischen Verbindung zwischen den Kontaktpins 214 und den Steckbuchsen 215. Durch die voreilende mechanische Führung der Fügekomponenten 203 und 210 wird die Verbindung der elektrischen Kontakte 214 und 215 stark vereinfacht und somit robuster gegen Fehlbedienung. Um die sterile Hülle 204 möglichst einfach und kostengünstig zu gestalten, sind keine elektrischen Kontakte integriert. Der elektrische Kontakt zwischen den Kontaktpins 214 und den Steckbuchsen 215 wird hergestellt, indem die sterile Hülle 204 an den Kontaktstellen während des Fügens des sterilen Instrumentenadapters 210 an das distale Ende 203 von den Kontaktpins 215 durchstoßen wird. Hierzu sind die Kontaktpins 214 an dem Ende, das in die Steckbuchsen 215 eingeführt wird, angespitzt. Fig. 7 verdeutlicht den Fügevorgang des sterilen Instrumentenadapters 210 an das distale Ende 203 des steril umhüllten Manipulatorarms 201.

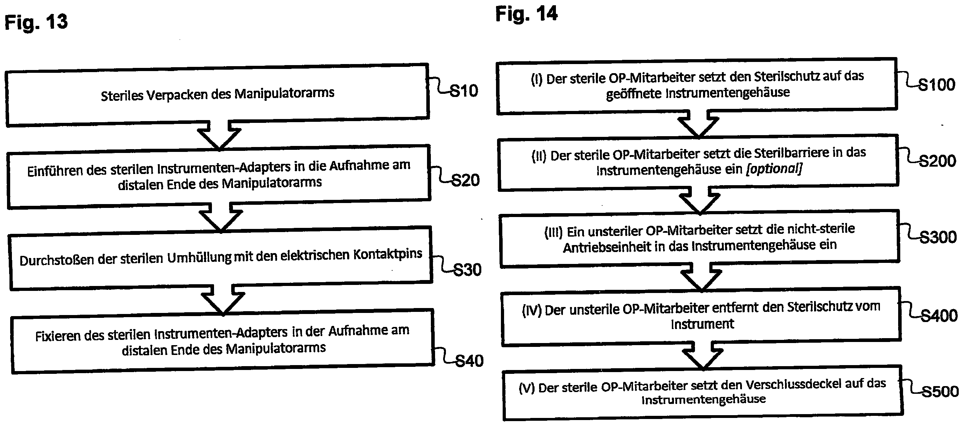

Fig. 13 zeigt ein Verfahren zum Montieren des vorstehend erläuterten Roboterchirurgiesystems:
Zuerst wird der Manipulatorarm 201 mit der sterilen Hülle 204 steril verpackt (Schritt S10). Dann wird der sterile Instrumentenadapter 210 mit seinem radialen Absatz 217 in die Aussparung 216 am distalen Ende 203 des Manipulatorarms 201 eingeführt und so eine mechanische Steckverbindung der elektro-mechanischen Schnittstelle 210 hergestellt (Schritt S20; Fig. 7 links bzw. "I"). Die voreilende mechanische Steckverbindung 216, 217 bietet eine ausreichende Führung des sterilen Instrumentenadapters 210 in der korrekten Orientierung zum distalen Ende 203 des Manipulatorarms 201, so dass die statische Sterilbarriere in Form der sterilen Umhüllung 204 von den Kontaktpins 214 durchstoßen und der elektrische Kontakt zwischen den Kontaktpins 214 und den Steckbuchsen 215 hergestellt wird (Schritt S30). Zuletzt wird der sterile Instrumentenadapter 210 in der Aufnahme 215 am distalen Ende 203 des Manipulatorarms 201 mechanisch fixiert (Schritt S40; Fig. 7 rechts bzw. "II"). Dies kann beispielsweise durch eine Schraubverbindung erfolgen, wobei die sterile Hülle 204 wie bei den Kontaktpins 214 beschrieben von der Schraube durchstoßen wird.

Vorstehend wurde die Montage der elektro-mechanischen Schnittstelle 210 am Roboter erläutert. In gleicher bzw. ähnlicher Weise kann die elektro-mechanische Schnittstelle 210 auch an dem Antriebseinheit-Gehäuseteil angeordnet werden. Beispielsweise kann dieses eine statische Sterilbarriere in Form einer Durchgangsöffnung aufweisen, die mit einer Dichtlippe steril abgedichtet ist, und die durch Kontaktpins der Schnittstelle 210 durchstoßen wird, wenn diese mittels einer Steckverbindung mit dem Antriebseinheit-Gehäuseteil verbunden wird.

Vor einem roboterchirurgischen Eingriff werden zunächst alle benötigten Instrumente vorbereitet und jeweils mit einer eigenen Antriebseinheit bestückt. Hierbei dürfen die Instrumente nicht von den nicht sterilen Antriebseinheiten kontaminiert werden.

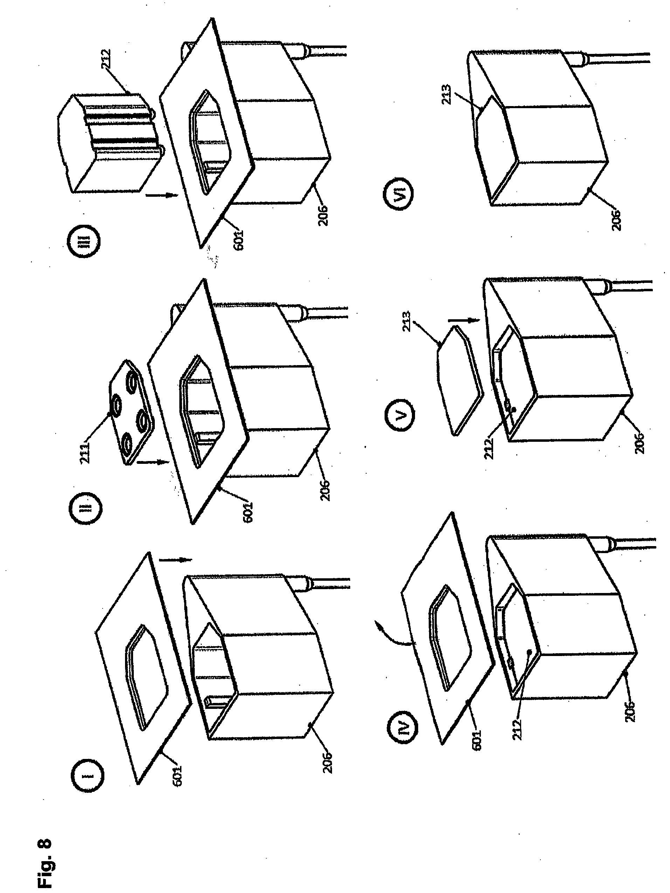

Fig. 8 zeigt in einer Figurenabfolge, Fig. 14 in einem Ablaufdiagramm, ein Verfahren nach einer Ausführung der vorliegenden Erfindung zur Montage der Instrumentenanordnung der Fig. 1, 4 bzw. 5, insbesondere zur Bestückung mit einer Antriebseinheit. Das Instrument der Fig. 8 entspricht dem Instrument 1 der Fig. 1 bzw. 205 der Fig. 4, 5.

Zum Einsetzen der Antriebseinheit sind zwei Personen vorteilhaft:
E in steriler OP-Mitarbeiter zur Handhabung des Instruments und zur Durchführung der "sterilen Handgriffe",
E in unsteriler OP-Mitarbeiter zur Handhabung der Antriebseinheit und zur Durchführung aller "nicht-sterilen Handgriffe".

Vor der Installation der Antriebseinheit werden alle benötigten Komponenten bereitgelegt, wobei die nicht sterile Antriebseinheit 212 getrennt von den sterilen Komponenten (Instrument, separate dynamische Sterilbarriere 211, Verschlussdeckel 213, Sterilschutz 601) bereitgestellt wird, um eine Kontamination zu vermeiden. Sofern der Verschlussdeckel 213 ein integraler Bestandteil des Instruments 201 ist, öffnet der sterile OP-Mitarbeiter den Verschlussdeckel 206 am proximalen Instrumentengehäuse 206, bevor die Antriebseinheit eingesetzt wird. Alternativ kann der Verschlussdeckel 213 auch vom proximalen Instrumentengehäuse 206 abnehmbar sein. In diesem Fall wird er vom sterilen OP-Mitarbeiter beiseitegelegt. Alternativ kann der Verschlussdeckel 206 auch getrennt vom Instrument 201 bereitgestellt werden.

In Schritt S100 (vgl. Fig. 14; Fig. 8: "I") setzt der sterile OP-Mitarbeiter den Sterilschutz 601 auf das geöffnete proximale Instrumentengehäuse 206, um das sterile Instrument 201 beim Einsetzen der Antriebseinheit 212 nicht zu kontaminieren. In Schritt S200 (Fig. 8: "II") setzt der sterile OP-Mitarbeiter die Sterilbarriere 211 in das proximale Instrumentengehäuse 206 ein. Dieser Prozessschritt ist nur bei einer separaten dynamischen Sterilbarriere (vgl. 8 in Fig. 1; 211 in Fig. 8) erforderlich und entfällt bei einer integrierten Sterilbarriere (vgl. 19 in Fig. 2; 119 in Fig. 3). Nach diesen Vorarbeiten kann der unsterile OP-Mitarbeiter in Schritt S300 (Fig. 8: "III") die Antriebseinheit 212 in das proximale Instrumentengehäuse 206 einsetzen und gegebenenfalls fixieren. Danach entfernt der unsterile OP-Mitarbeiter in S400 (Fig. 8: "IV") den Sterilschutz 601 vom proximalen Instrumentengehäuse 206. Zum Schluss setzt der sterile OP-Mitarbeiter den Verschlussdeckel 213 auf das proximale Instrumentengehäuse 206 (Fig. 14: S500; Fig. 8: "VI"). Damit ist die nicht sterile Antriebseinheit 212 im proximalen Instrumentengehäuse 206 eingeschlossen.

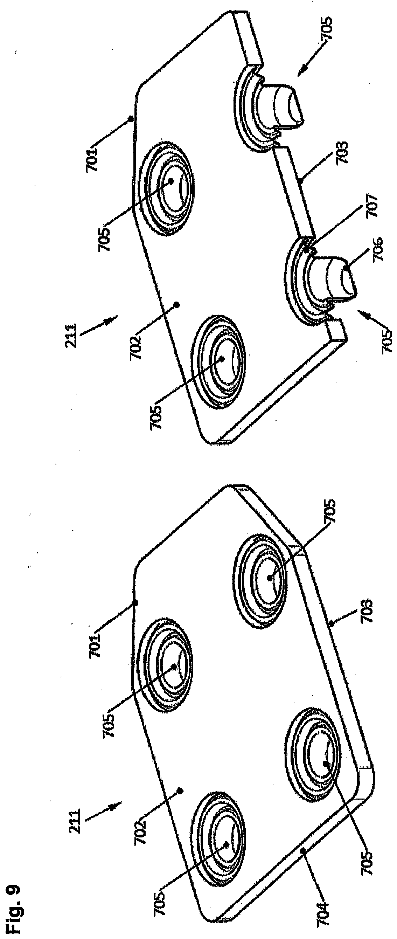

Fig. 9 zeigt in einer perspektivischen Voll- (links in Fig. 9) bzw. Schnittdarstellung (rechts in Fig. 9) eine dynamische Sterilbarriere der Instrumentenanordnung nach Fig. 1, 4 und 8.

Die Sterilbarriere 211 der Fig. 9 entspricht der Sterilbarriere 8 der Fig. 1 bzw. 211 der Fig. 8. Sie besteht aus einer starren Zwischenplatte 701, auf deren Seite 702 die Antriebseinheit aufliegt. Die der Seite 702 gegenüberliegende Seite 703 liegt auf der Bodenfläche des Hohlraums im proximalen Instrumentengehäuse 206 auf. Die äußeren Abmessungen der starren Platte 702 sind gegenüber der Berandung des Hohlraums im proximalen Instrumentengehäuse 206 etwas zurückgesetzt, um die Sterilbarriere von Hand ohne großen Kraftaufwand einsetzen zu können. Um eine bessere Isolation bzw. sterile Abdichtung der Antriebseinheit 212 vom sterilen Teil des Instruments zu erreichen, kann alternativ auf der Seite 703 oder die Randfläche 704 der starren Platte 702 eine umlaufende berührende Dichtung integriert sein (nicht dargestellt). Eine solche Dichtung kann insbesondere eine nahtlos eingeschäumte Dichtung, beispielsweise aus Polyurethan, und/oder eine ringförmige Elastomerdichtung, insbesondere als O-Ring, Dichtlippe oder dergleichen aufweisen.

In der starren Platte 701 sind Durchbrüche entsprechend der Anzahl der aktuierten Freiheitsgrade bzw. Antriebsstränge angeordnet. In jeden dieser Durchbrüche ist jeweils ein Bewegungsübertragungselement 705 integriert, das einen Einzelantrieb der Antriebseinheit 212 unter Wahrung der Sterilität an den zugeordneten instrumentenseitigen Antriebsstrang koppelt. Der rechte Teil von Fig. 9 zeigt einen Schnitt durch die Sterilbarriere 211, um eine mögliche Ausführung eines Bewegungsübertragungselements 705 für lineare Stellbewegungen zu verdeutlichen. In dem gezeigten Beispiel ist das Bewegungsübertragungselement 705 als dünnwandige Membranstruktur ausgebildet. Die Kupplungselemente des Einzelantriebs sind in einer zylinderförmigen Ausstülpung 706 im Zentrum des Bewegungsübertragungselements 705 angeordnet. Das instrumentenseitige Kupplungselement 11 (vgl. Fig. 1) umgibt im angekoppelten Zustand die Ausstülpung 706. Um eine Beweglichkeit der Ausstülpung 706 in einer Richtung normal zur starren Platte 701 zu ermöglichen, ist die Ausstülpung 706 mit einer elastischen Membran 707 in dem Durchbruch der starren Platte befestigt. Somit ist diese separate dynamische Sterilbarriere beweglich ausgebildet.

Ein Vorteil eines austauschbaren Instrumenten-Antriebs ist die Möglichkeit, bei Bedarf auch Instrumente mit abweichenden Anforderungen an die Antriebseinheit (Anzahl der aktuierten Freiheitsgrade, Stellkräfte, etc.) einsetzen zu können. Um dabei Fehlbedienungen und Schäden an den Instrumenten ausschließen zu können, wird eine verwechslungssichere Gestaltung der Antriebseinheiten vorgeschlagen.

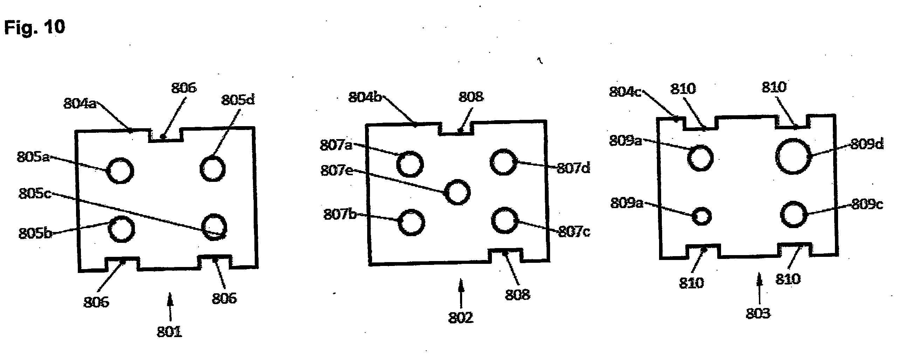

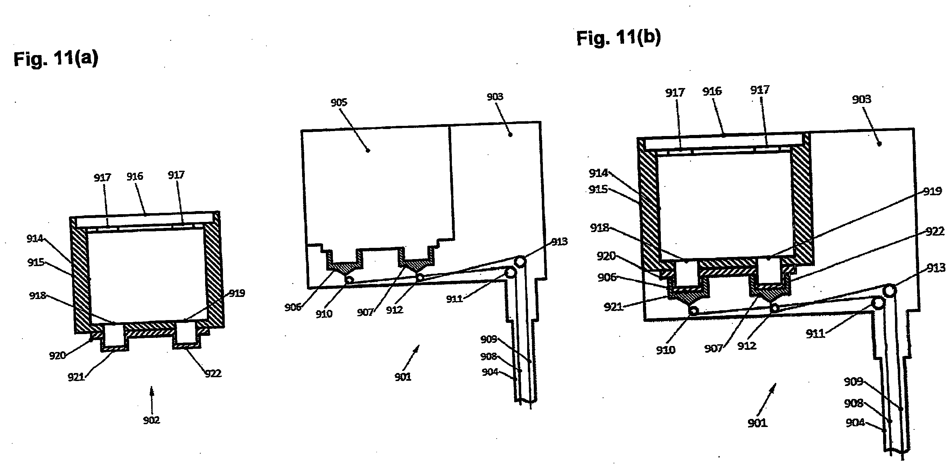

Fig. 10 zeigt unterschiedliche mechanische Kodierungen von Antriebseinheiten der Instrumentenanordnung nach einer der Fig. 1 bis 8 und 11(a), 11(b), um eine Verwechslung einzelner Antriebseinheiten zu verhindern. Die Abbildung zeigt die Ansichten dreier Antriebseinheiten 801, 802, 803 aus Richtung der Kupplungselemente der Einzelantriebe. Die Antriebseinheiten 801, 802, 803 entsprechen beispielsweise der Antriebseinheit 4 der Fig. 1, 2 oder 3, 212 der Fig. 4 und 8 oder 914 der Fig. 11(a), 11(b).

Sie unterscheiden sich in der Konfiguration der Einzelantriebe. Beispielhaft verfügt die Antriebseinheit 801 (links in Fig. 10) über die Einzelantriebe 805a, 805b, 805c, 805d. Antriebseinheit 802 (mittig in Fig. 10) verfügt beispielhaft über die Einzelantriebe 807a, 807b, 807c, 807d, 807e. Antriebseinheit 803 (rechts in Fig. 10) verfügt beispielhaft über die Einzelantriebe 809a, 809b, 809c, 809d.

Um eine Verwechslung der Antriebseinheiten bei der Installation in einem Instrument auszuschließen, verfügen die Gehäuse 804a, 804b bzw. 804c der Antriebseinheiten 801, 802 bzw. 803 über eine unterschiedliche mechanische Kodierung 806, 808 bzw. 810. In dem gezeigten Beispiel sind die mechanischen Kodierungen 806, 808, 810 jeweils als eine Kombination einer oder mehrerer Nuten ausgeführt, die sich in der Einführrichtung der Antriebseinheiten 801, 802, 803 in das proximale Instrumentengehäuse 206 erstrecken. Durch die unterschiedlichen Nutenmuster 806, 808, 810 ist eine Verwechslung der Antriebseinheiten ausgeschlossen.

Fig. 11(a), 11(b) zeigen ein Instrument einer Instrumentenanordnung nach einer weiteren Ausführung der vorliegenden Erfindung in Fig. 1 entsprechender Darstellung mit von einem Antriebsstrang-Gehäuseteil getrennten (Fig. 11 (a)) bzw. mit diesem verbundenen (Fig. 11 (b)) Antriebseinheit-Gehäuseteil. Das Instrument kann bis auf die nachstehend erläuterten Unterschiede den vorstehend beschriebenen Ausführungen entsprechen, so dass diesbezüglich auf deren Beschreibung Bezug genommen und nachfolgend nur auf die Unterschiede eingegangen wird.

Bei dieser Ausführung bildet das Antriebseinheit-Gehäuseteil mit der Antriebseinheit eine eigenständige Funktionseinheit und kann, sofern nötig, intraoperativ vom restlichen Instrument, insbesondere dem Antriebsachsen-Gehäuseteil, getrennt werden. Für die Antriebseinheit ist ein sterilisierbares und mehrfach verwendbares Antriebseinheit-Gehäuseteil vorgesehen, das für das OP-Personal einfacher zu handhaben ist als eine sterile Hülle in Form eines dünnen Folienschlauchs. Alternativ kann das sterile Antriebseinheit-Gehäuseteil auch als Einmalartikel gestaltet sein. Im Unterschied zu den oben beschriebenen Ausführungen ist eine Trennung von Antriebseinheit-Gehäuseteil mit der Antriebseinheit und restlichem Instrument, insbesondere Antriebsachsen-Gehäuseteil, möglich, so dass eine Antriebseinheit während einer Operation für verschiedene Instrumente verwendet werden kann.

Fig. 11(a), 11(b) zeigen die Struktur der lösbar miteinander verbindbaren Funktionseinheiten des Instruments 901, nämlich das separate Antriebseinheit-Gehäuseteil 902 mit der Antriebseinheit 914 (links in Fig. 11(a)) und das Antriebsachsen-Gehäuseteil 903 (rechts in Fig. 11(a)).

Am proximalen Ende des Instrumentenschaftes 904 ist integral das Antriebsachsen-Gehäuseteil 903 ausgebildet oder mit diesem, insbesondere lösbar, verbunden. In dem Antriebsachsen-Gehäuseteil 903 sind die Antriebsstränge der Kinematik und des Endeffektors angeordnet, die sich am distalen Ende des Instrumentenschafts 904 befinden. Das Antriebsachsen-Gehäuseteil 903 verfügt über einen Adapter 905 zur Aufnahme der durch das Antriebseinheit-Gehäuseteil 902 steril verpackten Antriebseinheit 914. Im Antriebsachsen-Gehäuseteil 903 sind mehrere Kupplungselemente 906, 907 sowie Seilzüge 908, 909 instrumentenseitiger Antriebsstränge vorgesehen, die die jeweiligen Einzelantriebe der Antriebseinheit 902 mit der distalen Instrumentenkinematik bzw. dem distalen Endeffektor koppeln. Die Antriebsstränge weisen außer den Kupplungselementen 906, 907 und Seilzügen 908,909 Umlenkrollen 910 bis 913 auf, durch die diese in den Instrumentenschaft 904 geführt werden.

Das Antriebseinheit-Gehäuseteil 902 weist eine nicht sterile Antriebseinheit 914 auf, die in einem sterilen Gehäuse 915 aus einem starren Werkstoff eingehaust ist. Vorteilhafte Werkstoffe für das sterile Gehäuse 915 sind insbesondere korrosionsbeständige Stähle, Titan oder medizintaugliche thermoplastische oder duroplastische Kunststoffe. Ein Verschlussdeckel 916 schirmt die nicht sterile Antriebseinheit 914 vom sterilen Operationsfeld ab. Eine mechanische Fixierung der Antriebseinheit 914 im sterilen Gehäuse 915 kann beispielsweise über ein oder mehrere am Verschlussdeckel 916 angeordnete Fixiermittel bzw. -elemente 917 erfolgen. Diese Elemente können beispielsweise als lineare oder viskoelastische Federn ausgeführt sein und so eine Vorspannkraft zwischen Deckel und Antriebseinheit erzeugen. Zusätzlich oder alternativ kann die Antriebseinheit 914 mit Spann- oder Rastmechanismen formschlüssig im Gehäuse 915 fixiert werden. Um die nicht sterile Antriebseinheit 914 vom sterilen Operationsfeld abzuschirmen, ist eine dynamische Sterilbarriere 920 vorgesehen, die mehrere Bewegungsübertragungselemente 921,922 für die Einzelantriebe 918,919 der Antriebseinheit enthält. Die Einzelantriebe 918,919 können als Dreh- und/oder Linearantriebe ausgebildet sein. Die Sterilbarriere 920 kann sowohl als integraler bzw. nicht zerstörungsfrei abtrennbarer Bestandteil des Gehäuses 915 oder als separates, vom Anwender abnehmbares Bauteil ausgebildet und lösbar mit dem Gehäuse 915 sein. Sie kann als Einmalartikel oder als wieder aufbereitbare Komponente ausgebildet sein. Gehäuse 915, Verschlussdeckel 916 und dynamische Sterilbarriere 920 bilden zusammen das Antriebseinheit-Gehäuseteil 902, welches die nicht sterile Antriebseinheit 914 steril aufnimmt. Diese kann beispielsweise, wie vorstehend mit Bezug auf Fig. 8, 14 beschrieben, in das Antriebseinheit-Gehäuseteil 902 eingebracht werden.

Fig. 11b zeigt das Antriebseinheit-Gehäuseteil 902 mit dem Antriebsachsen-Gehäuseteil 903 zu dem Instrument 901 verbunden. Die Bewegungsübertragung von der Antriebseinheit 902 auf die Antriebsstränge 906 bis 913 erfolgt durch die Verbindung der Einzelantriebe 918,919 mit den jeweiligen instrumentenseitigen Kupplungselementen 906,907 durch bzw. über die Bewegungsübertragungselemente 921,922. Das Ankoppeln der Antriebseinheit 914 an die instrumentenseitigen Antriebsstränge erfolgt bei der Installation des Antriebseinheit-Gehäuseteils 902 am Adapter 905.

In der Ausführung der Fig. 11 befindet sich der Adapter 905 für das Antriebseinheit-Gehäuseteil 902 beispielhaft proximal am Antriebsachsen-Gehäuseteil 903. Alternativ sind auch Instrumente 901 denkbar, bei denen sich der Adapter 905 distal oder seitlich befindet. Zusätzlich oder alternativ kann die mit Bezug auf Fig. 10 erläuterte verwechslungssichere Gestaltung der Antriebseinheiten bei dieser Ausführung vorgesehen sein: Die mechanische Kodierung kann hierbei entweder nur auf dem Gehäuse 915 oder sowohl an der Antriebseinheit 914 als auch dem Gehäuse 915 vorgesehen werden.

Bei dieser Ausführung ist wie bei den Ausführungen der Fig. 1, 2, 4 bis 11 die Antriebseinheit zur Schaftachse lateral versetzt angeordnet. Somit wird wiederum Bezug auf die restliche Beschreibung genommen. Wie mit Bezug auf Fig. 3 gezeigt, ist jedoch grundsätzlich auch eine koaxial zum Instrumentenschaft 904 angeordnete Antriebseinheit möglich.

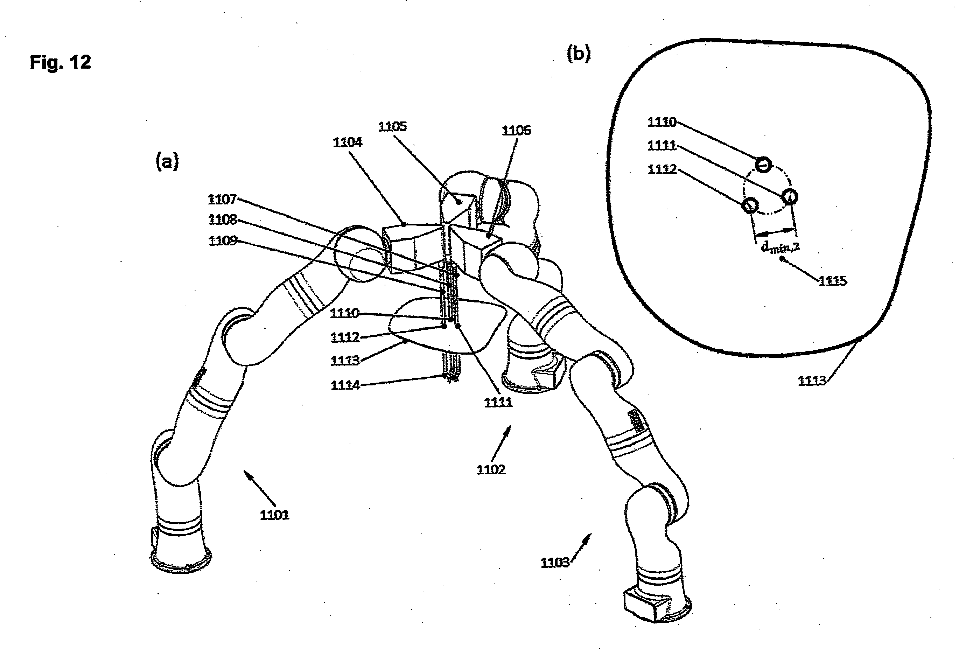

Fig. 12(a) zeigt ein Roboterchirurgiesystem nach einer weiteren Ausführung der vorliegenden Offenbarung in perspektivischer Darstellung, Fig. 12(b) eine Draufsicht auf dessen Operationsfeld.

Bei dieser Ausführung sind die radialen Abmessungen sowohl des Instruments als auch des Manipulatorarms im Bereich des Instrumentenschafts minimiert. Mit dieser Maßnahme kann das Kollisionsrisiko in mehrarmigen Applikationen bzw. bei mehreren kooperierenden Robotern reduziert werden. Für den Anwender bedeutet dies eine gesteigerte Flexibilität bei der Trokarplatzierung und/oder geringere Trokarabstände.

Um den Abstand zu minimieren, ist die Antriebseinheit des Instruments mit einem lateralen Versatz zur Schaftachse angeordnet. Somit können Komponenten des Robotersystems mit größerem Raumbedarf, insbesondere das distale Ende des Manipulatorarms, die Antriebseinheit, mechanische Schnittstellen zum Instrument, außerhalb des engeren Interaktionsradius der Manipulatorarme angeordnet werden. Abbildung 12(a) zeigt beispielhaft eine Applikation mit drei Manipulatorarmen bzw. Robotern. Jeder der Manipulatorarme 1101,1102 bzw. 1103 trägt an seinem distalen Ende ein chirurgisches Instrument 1104,1105 bzw. 1106, das jeweils an seinem proximalen Ende über eine Antriebseinheit verfügt, die lateral versetzt zur Längsachse des Instrumentenschafts 1107,1108 bzw. 1109 angeordnet ist. Aus der Aufstellung der Manipulatorarme 1101,1102,1103 relativ zum Operationsgebiet 1114 und den proximalen Abmessungen der Instrumente 1104,1105,1106 resultieren die Durchtrittspunkte 1110,1111,1112 der Instrumentenschäfte 1107,1108,1109 durch die Bauchdecke 1113. Abbildung 12(b) zeigt den aus dieser Anordnung resultierenden minimalen Abstand 1115 bzw. d_{min, 1} der Durchtrittspunkte 1110,1111,1112.

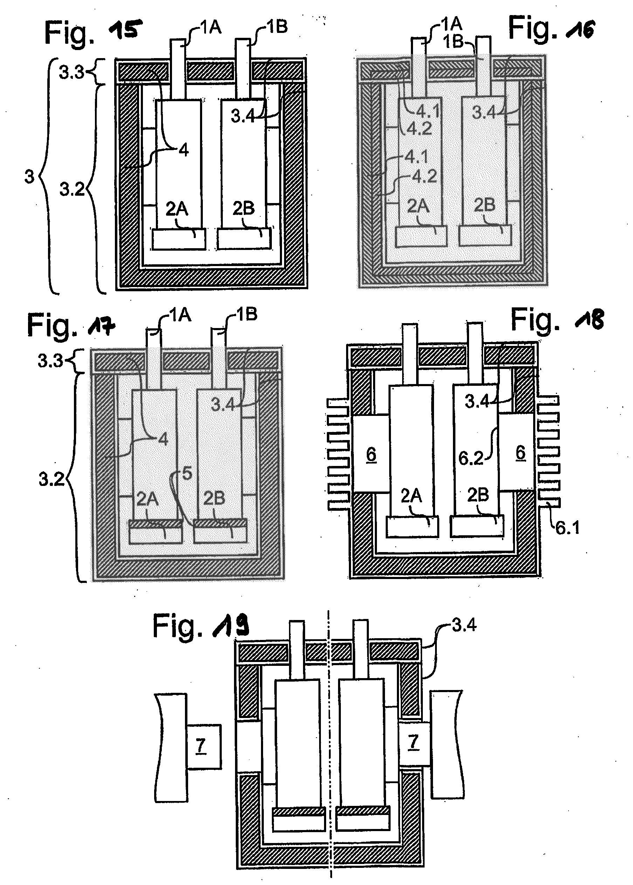

Fig. 15 zeigt eine sterilisierbare Antriebseinheit eines chirurgischen Instruments eines Chirurgierobotersystems nach einer Ausführung der vorliegenden Offenbarung.

Die sterilisierbare Antriebseinheit weist eine Aktuatoranordnung mit einem oder mehreren Aktuatoren in Form von kraft- und/oder positionsgeregelten Elektromotoren auf, von denen in Fig. 15 exemplarisch zwei dargestellt sind, deren Abtriebsachsen mit 1A bzw. 1B bezeichnet sind. Im Ausführungsbeispiel sind diese Abtriebsachsen 1A, 1B translatorisch aktuierbar (vertikal in den Figuren 1 bis 25), beispielsweise, indem die Elektromotoren ein entsprechendes Umsetzungsgetriebe zum Umsetzen einer rotatorischen in einer translatorische Abtriebsbewegung aufweisen oder als Linearelektromotoren ausgebildet sind.

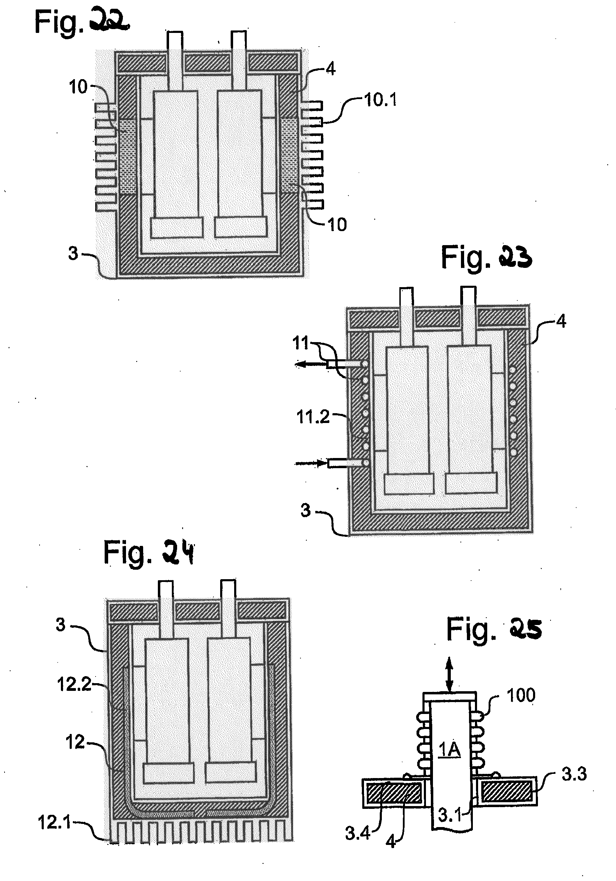

Die Aktuatoranordnung ist mittels einer Schnittstelle lösbar mit einem Instrumentenschaft des chirurgischen Instruments des Chirurgierobotersystems (nicht dargestellt) gekoppelt. Die Schnittstelle weist eine Hülle 100 auf (vgl. Fig. 25), welche Durchtrittsöffnungen 3.1 eines Gehäuses 3 der Antriebseinheit fluiddicht abdeckt und jeweils einen diese Durchtrittsöffnung durchgreifenden Teil einer Abtriebsachse 1A, 1B der Aktuatoranordnung umhüllt. Die Hülle 100 ist balgartig ausgebildet bzw. weist eine Faltung auf und ist dazu vorgesehen, translatorische Bewegungen der Abtriebsachsen 1A, 1B mitzumachen. Die Hülle 100 kann mit dem Gehäuse lösbar, insbesondere durch Schrauben, oder unlösbar, insbesondere stoffschlüssig, vorzugsweise durch Schweißen oder Kleben, verbunden sein. Translatorische Bewegungen der Abtriebsachsen 1A, 1B aktuieren entsprechende Freiheitsgrade eines Endeffektors des Instrumentenschafts (nicht dargestellt).

Die Antriebseinheit weist weiter eine Bauteilanordnung mit mehreren elektronischen Bauteilen in Form von Positionssensoren zur Ermittlung von Positionen der Aktuatoren auf, von denen in Fig. 15 exemplarisch zwei Positionssensoren 2A, 2B dargestellt sind. Die Antriebseinheit kann optional weitere elektronische Bauelemente bzw. Baugruppen enthalten, insbesondere zur Signalerfassung, -aufbereitung und/oder -verarbeitung, zur Ansteuerung der Motoren und/oder zur Kommunikation mit einer übergeordneten Steuerung.

Die Aktuator- und die Bauteilanordnung sind im Inneren des sterilisierbaren Gehäuses 3 angeordnet, welches zwei lösbar miteinander verbindbare Gehäuseteile in Form eines Gehäusetopfes 3.2 und eines damit fluiddicht verschraubten Deckels 3.3 aufweist, zwischen denen eine O-Ring-Dichtung angeordnet ist.

Die beiden Gehäuseteile weisen eine formsteife Gehäusewand 3.4 auf, die im Ausführungsbeispiel als Außenwand aus Metall und/oder Kunststoff besteht.

An dieser Gehäusewand 3.4 ist innenseitig eine thermische Isolierschicht 4 angeordnet, die im Ausführungsbeispiel als Vakuumisolierschicht ausgebildet ist bzw. eine Vakuumisolation aufweist.

Hierzu ist parallel zu der Gehäuseaußenwand 3.4 eine Gehäuseinnenwand angeordnet bzw. die Gehäuseaußenwand 3.4 doppelwandig ausgebildet. Gehäuseaußen- und -innenwand begrenzen zwischen sich einen luftdichten Raum, der mit Luft unter Unterdruck gefüllt bzw. evakuiert ist. Die Gehäuseinnenwand kann optional entfallen, insbesondere falls die Isolationsschicht ohne Vakuum ausgebildet ist.

Die thermische Isolierschicht 4 deckt die innere Oberfläche der Gehäuseaußenwand 3.4 bis auf die Durchtrittsöffnungen 3.1 (vgl. Fig. 25) vollständig ab bzw. umschließt das Gehäuseinnere mit Aktuator- und Bauteilanordnung, wenigstens im Wesentlichen, vollständig. Auf diese Weise kann ein Wärmeeintrag in das Gehäuseinnere während einer Beaufschlagung mit Heißdampf und/oder -luft zur Sterilisierung der Antriebseinheit minimiert und damit eine thermische Überlastung insbesondere der temperaturempfindlichen Positionssensoren 2A, 2B vermieden werden. Die Isolierschicht kann in einer Abwandlung weitere Unterbrechungen aufweisen, insbesondere für Kabeldurchführungen, Steckverbinder, elektrische Kontakte, Schraubenverbindungen oder dergleichen.

Fig. 16 zeigt in Fig. 15 entsprechender Darstellung eine sterilisierbare Antriebseinheit eines chirurgischen Instruments eines Chirurgierobotersystems nach einer weiteren Ausführung der vorliegenden Offenbarung. Übereinstimmende Merkmale sind durch identische Bezugszeichen bezeichnet, so dass auf deren Beschreibung Bezug genommen und nur auf die Unterschiede der Ausführungen eingegangen wird.

In der Ausführung der Fig. 16 ist die thermische Isolierschicht mehrlagig ausgebildet und weist zwei Lagen 4.1, 4.2 auf, von denen eine insbesondere einen Dämmstoff, etwa Mineralwolle oder PUR-Hartschaum aufweisen, die andere insbesondere wie vorstehend beschrieben als Vakuumisolierschicht ausgebildet sein kann. Hierdurch kann die thermische Isolierung des Gehäuses 3 noch erhöht werden.

Fig. 17 zeigt in Fig. 15, 16 entsprechender Darstellung eine sterilisierbare Antriebseinheit eines chirurgischen Instruments eines Chirurgierobotersystems nach einer weiteren Ausführung der vorliegenden Offenbarung. Übereinstimmende Merkmale sind durch identische Bezugszeichen bezeichnet, so dass auf deren Beschreibung Bezug genommen und nur auf die Unterschiede der Ausführungen eingegangen wird. In der Ausführung der Fig. 17 ist zusätzlich zu der thermischen Isolierschicht 4 an der Gehäusewand 3.4 zwischen der Bauteilanordnung 2A, 2B und der Aktuatoranordnung eine thermische Isolierschicht 5 angeordnet, im Ausführungsbeispiel exemplarisch aus Kunststoff mit einer Wärmleitfähigkeit λ < 0,4 W/(K•m). Hierdurch kann vorteilhaft eine Wärmeleitung von der Aktuatoranordnung zu der Bauteilanordnung und damit eine Temperaturbeaufschlagung der Bauteilanordnung reduziert werden. Eine solche zusätzliche thermische Isolierschicht 5 kann gleichermaßen auch bei einer mehrlagigen thermischen Isolierschicht 4.1, 4.2 an der Gehäusewand 3.4 vorgesehen sein, wie sie mit Bezug auf Fig. 16 erläutert wurde.

Während in den Ausführungen der Fig. 15 bis 17 eine thermische Isolierschicht die innere Oberfläche der Gehäuseaußenwand 3.4 bis auf die Durchtrittsöffnungen 3.1 vollständig abdeckt, ist sie in den nachfolgend mit Bezug auf Fig. 18 bis 25 erläuterten Ausführungen jeweils nur auf Teilen bzw. Abschnitten der Gehäusewand 3.4 angeordnet, insbesondere auf Höhe der Bauteilanordnung 2A, 2B bzw. dieser Bauteilanordnung gegenüberliegend.

Fig. 18 zeigt in Fig. 15 bis 17 entsprechender Darstellung eine sterilisierbare Antriebseinheit eines chirurgischen Instruments eines Chirurgierobotersystems nach einer weiteren Ausführung der vorliegenden Offenbarung. Übereinstimmende Merkmale sind durch identische Bezugszeichen bezeichnet, so dass auf deren Beschreibung Bezug genommen und nur auf die Unterschiede der Ausführungen eingegangen wird. In der Ausführung der Fig. 18 durchgreifen stationäre Wärmeleitmittel 6 aus Kupfer, Aluminium oder dergleichen die thermische Isolierschicht 4. Sie sind dauerhaft mit der Aktuatoranordnung verbunden und weisen eine Wärmeabgabefläche 6.1 auf der Außenseite der Gehäusewand 3.4 und eine damit materialverbundene Wärmeaufnahmefläche 6.2 im Inneren des Gehäuses auf, die mit der Befestigung der Aktuatoranordnung mit dem Gehäuse fest verbunden, insbesondere integral ausgebildet sein kann. Insbesondere kann die Wärmeaufnahmefläche 6.2 ein Gehäuse der Elektromotoren kontaktieren bzw. damit wärmeleitend verbunden sein. Durch die Wärmeleitmittel 6 kann während des Betriebs Abwärme der Elektromotoren aus dem abschnittsweise thermisch isolierten Gehäuseinneren abgeführt werden. Hierzu weisen die Wärmeabgabeflächen 6.1 eine vergrößerte Oberfläche mit Kühlrippen, -lamellen und/oder -stiften auf. Die Wärmeabgabeflächen 6.1 können lösbar mit den Wärmeleitmitteln 6 verbunden sein.

Fig. 19 zeigt in Fig. 18 entsprechender Darstellung eine sterilisierbare Antriebseinheit eines chirurgischen Instruments eines Chirurgierobotersystems nach einer weiteren Ausführung der vorliegenden Offenbarung. Übereinstimmende Merkmale sind durch identische Bezugszeichen bezeichnet, so dass auf deren Beschreibung Bezug genommen und nur auf die Unterschiede der Ausführungen eingegangen wird.

In der Ausführung der Fig. 19 sind die Wärmeleitmittel schaltbar ausgebildet und können zwischen einem ersten, wärmeleitenderen Zustand, der in der rechten Hälfte der insoweit geteilten Fig. 19 dargestellt ist, und einem zweiten, weniger wärmeleitenderen Zustand umgeschaltet werden, der in der linken Hälfte der Fig. 19 dargestellt ist. Die Wärmeleitmittel können beispielsweise mit dem Roboter verbundene Kühlkörper 7 aufweisen bzw. sein, die in dem ersten, wärmeleitenderen Zustand Aussparungen in der thermischen Isolierschicht 4 durchgreifen und die Gehäuse der Elektromotoren kontaktieren (vgl. Fig. 19 rechts). Dieser Kontakt kann beim Ankoppeln der Antriebseinheit an den Roboter automatisch hergestellt werden. In dem zweiten, weniger wärmeleitenderen Zustand sind hingegen die Elektromotoren und die Kühlkörper 7 durch einen Spalt beabstandet (vgl. Fig. 19 links).

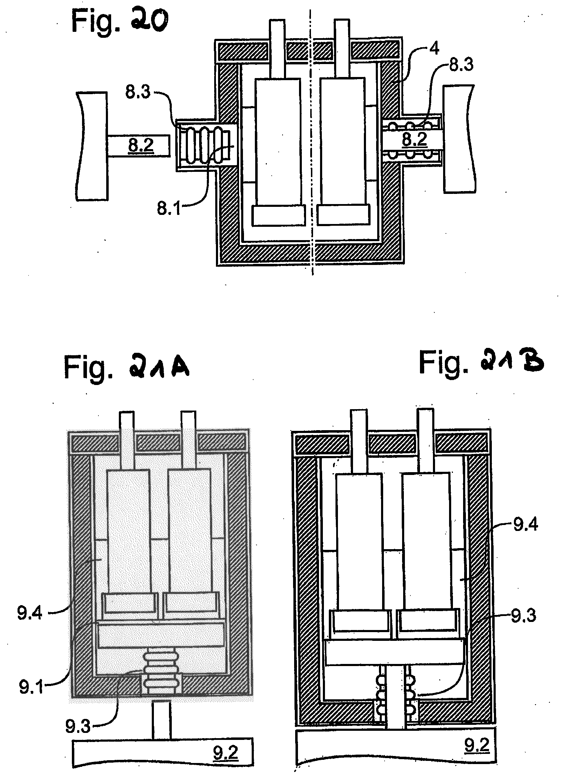

Fig. 20 zeigt in Fig. 19 entsprechender Darstellung eine sterilisierbare Antriebseinheit eines chirurgischen Instruments eines Chirurgierobotersystems nach einer weiteren Ausführung der vorliegenden Offenbarung. Übereinstimmende Merkmale sind durch identische Bezugszeichen bezeichnet, so dass auf deren Beschreibung Bezug genommen und nur auf die Unterschiede der Ausführungen eingegangen wird.

Auch in der Ausführung der Fig. 20 sind die Wärmeleitmittel schaltbar ausgebildet und können zwischen einem ersten, wärmeleitenderen Zustand, der in der rechten Hälfte der insoweit geteilten Fig. 20 dargestellt ist, und einem zweiten, weniger wärmeleitenderen Zustand umgeschaltet werden, der in der linken Hälfte der Fig. 20 dargestellt ist.

Die Wärmeleitmittel weisen einen Spalt 8.1 in der thermischen Isolierschicht 4 und ein bewegliches Element 8.2 zum wahlweisen wärmeleitenden Überbrücken dieses Spaltes auf. Der Spalt 8.1 ist fluiddicht ausgebildet und weist einen Unterdruck bzw. ein Vakuum auf, um seine Wärmeleitfähigkeit zu reduzieren. Der Spalt ist durch eine elastische Hülle 8.3 begrenzt, die eine Faltung aufweist bzw. balgartig ausgebildet ist. Im ersten, wärmeleitenderen Zustand (vgl. Fig. 20 rechts), überbrückt das bewegliche Element 8.2 den Spalt und erhöht so die Wärmeleitfähigkeit des Wärmeleitmittels, im zweiten, weniger wärmeleitenderen Zustand (vgl. Fig. 19 links) wird der Spalt 8.1 nicht überbrückt und isoliert so thermisch, so dass das Wärmeleitmittel durch Bewegen des beweglichen Elements 8.2 umschaltbar ist.

Fig. 21 zeigt in Fig. 20 entsprechender Darstellung eine sterilisierbare Antriebseinheit eines chirurgischen Instruments eines Chirurgierobotersystems nach einer weiteren Ausführung der vorliegenden Erfindung. Übereinstimmende Merkmale sind durch identische Bezugszeichen bezeichnet, so dass auf deren Beschreibung Bezug genommen und nur auf die Unterschiede der Ausführungen eingegangen wird.

Auch in der Ausführung der Fig. 21 ist ein Wärmeleitmittel schaltbar ausgebildet und kann zwischen einem ersten, wärmeleitenderen Zustand, der Fig. 21B dargestellt ist, und einem zweiten, weniger wärmeleitenderen Zustand umgeschaltet werden, der in Fig. 21A dargestellt ist.

Im Unterschied zur Ausführung der Fig. 20 ist bei der Ausführung der Fig. 21 keine direkte Kontaktierung der Elektromotorengehäuse durch das bewegliche Element vorgesehen. In einer Ausführung der vorliegenden Offenbarung, wie sie exemplarisch in Fig. 21 dargestellt ist, ist allgemein ein Wärmesammelmittel (in Fig. 21 exemplarisch 9.4) vorgesehen, welches dauerhaft mehrere, insbesondere alle Aktuatoren der Aktuatoranordnung kontaktiert, und welches durch ein bewegliches Element 9.2 wahlweise kontaktiert werden kann.

Auch bei dieser Ausführung ist ein Spalt 9.1 ausgebildet, welcher durch das bewegliche Element 9.2 wahlweise überbrückt werden kann. Der Spalt ist fluiddicht durch eine elastische Hülle 9.3 begrenzt, die eine Faltung aufweist bzw. balgartig ausgebildet ist. Er weist im Ausführungsbeispiel der Fig. 21 keinen Unterdruck auf. In einer Abwandlung kann jedoch das Gehäuseinnere evakuiert sein, um eine thermische Isolierung der Bauteilanordnung vorteilhaft zu erhöhen, wobei dann auch der Spalt 9.1 Unterdruck aufweist. Allgemein kann in einer Ausführung der vorliegenden Offenbarung ein Gehäuseinneres evakuiert bzw. mit Luft oder Gas unter Unterdruck gefüllt sein.

Im Ausführungsbeispiel der Fig. 21 ist das bewegliche Element zweiteilig ausgebildet, wobei ein Teil, welches dauerhaft in der Hülle 9.3 angeordnet ist, beweglich und unverlierbar in dem Gehäuse 3 angeordnet ist, während ein anderes Teil dieses Teil wahlweise kontaktieren und im Gehäuse bewegen kann. Das in der Hülle 9.3 angeordnete Teil ist durch diese elastisch von dem Wärmesammelmittel 9.4 weg vorgespannt und kann durch das andere Teil gegen das Wärmesammelmittel 9.4 bewegt werden, um den Spalt zu diesem zu überbrücken.

Fig. 22 zeigt in Fig. 15 entsprechender Darstellung eine sterilisierbare Antriebseinheit eines chirurgischen Instruments eines Chirurgierobotersystems nach einer weiteren Ausführung der vorliegenden Offenbarung. Übereinstimmende Merkmale sind durch identische Bezugszeichen bezeichnet, so dass auf deren Beschreibung Bezug genommen und nur auf die Unterschiede der Ausführungen eingegangen wird.

In der Ausführung der Fig. 22 weist das schaltbare Wärmeleitmittel mehrere Peltierelemente 10 auf. Durch Anlegen einer Spannung kann wahlweise eine Temperaturdifferenz und damit ein erster, wärmeleitenderer Zustand erzeugt werden. Die Peltierelemente 10 weisen Wärmeabgabeflächen 10.1 auf, die an der Außenseite des Gehäuses 3 angeordnet sind.

Fig. 23 zeigt in Fig. 22 entsprechender Darstellung eine sterilisierbare Antriebseinheit eines chirurgischen Instruments eines Chirurgierobotersystems nach einer weiteren Ausführung der vorliegenden Offenbarung. Übereinstimmende Merkmale sind durch identische Bezugszeichen bezeichnet, so dass auf deren Beschreibung Bezug genommen und nur auf die Unterschiede der Ausführungen eingegangen wird.

In der Ausführung der Fig. 23 weist das Wärmeleitmittel Fluidpassagen 11 mit einem Arbeitsfluid, beispielsweise einem flüssigen Kältemittel, auf, welches Wärme mit einer Wärmeabgabefläche (nicht dargestellt) auf der Außenseite des Gehäuses 3 und einer Wärmeaufnahmefläche 11.2 des Wärmeleitmittels austauschen kann. Ein Strömungsmittel zum wahlweisen aktiven Strömen in Form einer steuerbaren, wahlweise aktivierbaren, Umwälzpumpe (nicht dargestellt) kann im Betrieb das Arbeitsfluid zwischen Wärmeaufnahme- und -abgabefläche umwälzen, wie in Fig. 23 durch Arbeitsfluidströmungspfeile angedeutet.

Fig. 24 zeigt in Fig. 23 entsprechender Darstellung eine sterilisierbare Antriebseinheit eines chirurgischen Instruments eines Chirurgierobotersystems nach einer weiteren Ausführung der vorliegenden Offenbarung. Übereinstimmende Merkmale sind durch identische Bezugszeichen bezeichnet, so dass auf deren Beschreibung Bezug genommen und nur auf die Unterschiede der Ausführungen eingegangen wird.

In der Ausführung der Fig. 24 sind die Fluidpassagen als umwälzpumpenlose Wärmerohre 12 mit einem Arbeitsfluid ausgebildet, welches unter Phasenumwandlung Wärme mit einer Wärmeabgabefläche 12.1 und einer Wärmeaufnahmefläche 11.2 des Wärmeleitmittels austauschen kann. Ein Strömungsmittel zum wahlweisen Sperren des Arbeitsfluids in Form eines steuerbaren Ventils (nicht dargestellt) kann im Betrieb das Arbeitsfluid ein Strömen von Arbeitsfluid in dem Wärmerohr ermöglichen oder unterbinden.

Zum Sterilisieren einer Antriebseinheit, wie sie vorstehend mit Bezug auf die Fig. 15 bis 25 erläutert wurde, wird eine Außenseite der Antriebseinheit für eine vorgegebene Zeitdauer, vorzugsweise wenigstens 5 Minuten, insbesondere wenigstens 20 Minuten, und/oder bei einem Druck von wenigstens 2 bar, insbesondere wenigstens 3 bar, mit erwärmtem Fluid, insbesondere Dampf oder Luft, vorzugsweise mit wenigstens 100°C, insbesondere wenigstens 120°C, vorzugsweise wenigstens 130°C beaufschlagt. Schaltbare Wärmeleitmittel 7, 8.2, 9.2 10, 11 bzw. 12 sind dabei in den zweiten, weniger wärmeleitenderen Zustand geschaltet (links in Fig. 19, 20; Fig. 21A). Im Betrieb schaltet ein Umschaltmittel diese in den ersten, wärmeleitenderen Zustand um (rechts in Fig. 19, 20; Fig. 21B, 22, 23, 24). Dies kann manuell oder automatisch erfolgen, insbesondere durch eine Ankopplung an den Roboter, durch die die beweglichen Elemente 7, 8.2, 9.2 in Kontakt mit der Aktuatoranordnung bzw. dem Wärmesammelmittel 9.4 gebracht werden können, oder in Abhängigkeit von einer Temperatur in einem Inneren des Gehäuses. Hierzu kann ein Umschaltmittel (nicht dargestellt) eine Temperatur in einem Inneren des Gehäuses 3 erfassen und bei Überschreiten eines vorgegebenen Grenzwertes ein oder mehrere schaltbare Wärmeleitmittel in den ersten, wärmeleitenderen Zustand schalten, beispielsweise eine Umwälzpumpe der Ausführung der Fig. 23 aktivieren, ein Ventil der Ausführung der Fig. 24 öffnen oder ein Peltierelement Ausführung der Fig. 22 bestromen.

Fig. 26 zeigt, wie einleitend beschrieben, ein Chirurgieroboter-System nach einer Ausführung der vorliegenden Offenbarung mit mehreren Robotern 1, 2, und 3, an deren distalem Ende jeweils ein chirurgisches Instrument 4,5 bzw. 6 einer Instrumentenanordnung nach einer Ausführung der vorliegenden Offenbarung lösbar befestigt ist.

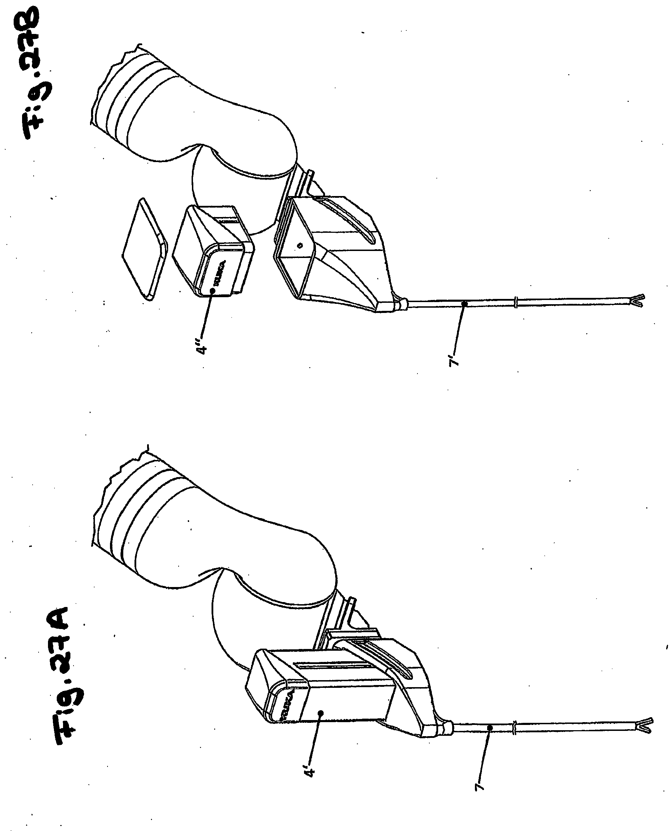

Fig. 27A, 27B zeigt verschiedene Ausführungen eines robotergeführten Instruments mit unterschiedlichen Antriebseinheiten, die mit dem chirurgischen Instrument bzw. Instrumentenschaft lösbar verbunden sind, um eine einfache Aufbereitung und eine möglichst kostengünstige Ausführung des Instruments zu gewährleisten. Bei der Ausführung gemäß Fig. 27A ist die modulare Antriebseinheit 4' intraoperativ vom Instrument wiederholt abtrennbar bzw. kann wiederholt an ein Instrument gefügt werden. Dazu wird die nicht sterile Antriebseinheit 4' präoperativ mit einer sterilen Hülle. Alternativ kann die Antriebseinheit auch als sterilisierbares Modul ausgeführt werden, wodurch die sterile Umhüllung entfallen kann. Im Gegensatz wird bei der Ausführung der Fig. 27B eine nicht sterile Antriebseinheit 4" präoperativ in ein proximales Instrumentengehäuse des Instrumentenschaftes 7' eingesetzt und dieses steril verschlossen. Bei diesem Konzept verbleibt die Antriebseinheit 4" vorteilhaft während der gesamten Dauer eines operativen Eingriffs in dem proximalen Instrumentengehäuse und wird erst nach dessen Ende und vor der Aufbereitung wieder entnommen.

Optional kann eine Schnittstelle zwischen Antriebseinheit und Instrumentenschaft eine durch eine Antriebseinheit dieses Instruments lösbare Befestigungssperre aufweisen, mit der verhindert wird, dass versehentlich ein Instrument ohne Antriebseinheit am Roboter adaptiert wird. Beispielsweise kann das proximale Instrumentengehäuse eine mechanische Sperre aufweisen, die bei eingesetzter Antriebseinheit deaktiviert bzw. gelöst wird. Das Instrumentengehäuse lässt sich nur mit deaktivierter Sperre an einem Manipulatorarm adaptieren. Alternativ oder zusätzlich zu der zuvor beschriebenen mechanischen Sperre bei nicht eingesetzter Antriebseinheit kann das Vorhandensein und/oder die korrekte Position der Antriebseinheit am Instrument mit einer in der Schnittstelle zwischen dem Roboter und der Antriebseinheit integrierten Anwesenheitssensorik überprüft werden.

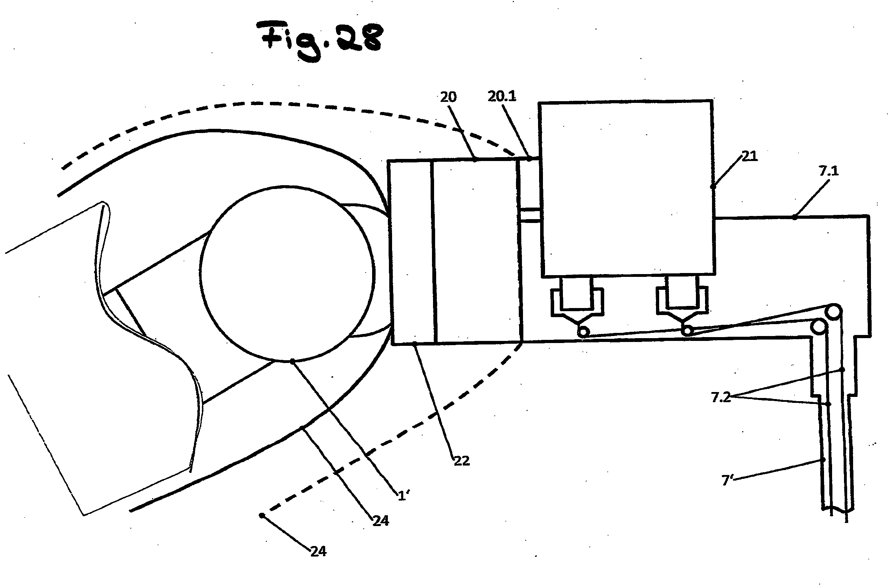

Fig. 28 zeigt ein robotergeführtes Instrument einer Instrumentenanordnung nach einer Ausführung der vorliegenden Offenbarung mit einer Aufteilung der mechatronischen Antriebseinheit in ein Elektronikmodul 20 und ein Antriebsmodul 21. Durch diese Trennung ist es möglich, die Antriebselektronik unabhängig von den Instrumentenantrieben handzuhaben, insbesondere am Roboter anzubringen. Dadurch reduzieren sich Gewicht und Volumen der vom OP-Personal zu handhabenden Module, die Bedienerfreundlichkeit des Systems wird verbessert.

Das Elektronikmodul 20 enthält in einer Ausführung die gesamte Antriebselektronik, zumindest jedoch einen Teil davon. So können in dem Elektronikmodul insbesondere Komponenten einer Signalverarbeitung für Sensorsignale, für die Regelung und Ansteuerung von Antriebsmotoren nötige Einheiten und/oder eine Kommunikationsschnittstelle zum Anschluss an den Roboter enthalten sein. Das Antriebsmodul 21 enthält beispielsweise für jeden Freiheitsgrad des Instruments einen Antriebsmotor, gegebenenfalls ein Untersetzungsgetriebe, ein Sensorsystem zu Geschwindigkeits- und/oder Positionserfassung, und/oder weitere Sensoren, zum Beispiel Kraftsensoren, Momentensensoren, Stromsensoren, Referenz- und Endschalter oder dergleichen.

Das Elektronikmodul 20 ist vorzugsweise mit einem Instrumentenadapter 22 am distalen Ende des Roboters 1' angebracht. Der Instrumentenadapter 22 stellt die mechanische Verbindung zwischen Roboter und der Antriebseinheit her und gewährleistet eine wiederholgenaue Positionierung und Fixierung der Antriebseinheit relativ zum distalen Ende des Roboters. Optional stellt der Instrumentenadapter 22 auch die erforderlichen elektrischen Verbindungen zwischen der Antriebseinheit und dem Roboter her. Das Elektronikmodul 20 kann entweder als sterilisierbares Modul ausgeführt sein oder von einer sterilen Hülle umgeben sein, die vorteilhaft zugleich den Manipulatorarm einschließt. Exemplarisch sind in Fig. 28 durchgezogen bzw. strichliert zwei mögliche Verläufe einer solchen sterilen Hülle 24 dargestellt, die ein nicht steriles Elektronikmodul 20 und einen nicht sterilen Instrumentenadapter 22 zusammen mit dem Roboter 1' (Fig. 28: strichliert) oder bei sterilem Elektronikmodul 20 und sterilem Instrumentenadapter 22 nur den Roboter 1' (Fig. 28: durchgezogen) umhüllen.

Optional eignet sich das beschriebene Elektronikmodul 20 auch für ein Instrument mit integriertem, vom Anwender nicht entnehmbaren Antriebsteil. In diesem Fall können durch die Auslagerung der gesamten oder wesentlichen Teile der Antriebselektronik die Baugröße und Kosten des Instruments reduziert werden.

Schematisch ist in Fig. 28 auch ein proximaler Flansch 7.1 sowie ein Antriebsstrang 7.2 des Instrumentenschaftes 7' sowie eine elektrische Schnittstelle 20.1 zwischen Elektronik- und Antriebsteil 20, 21 angedeutet.

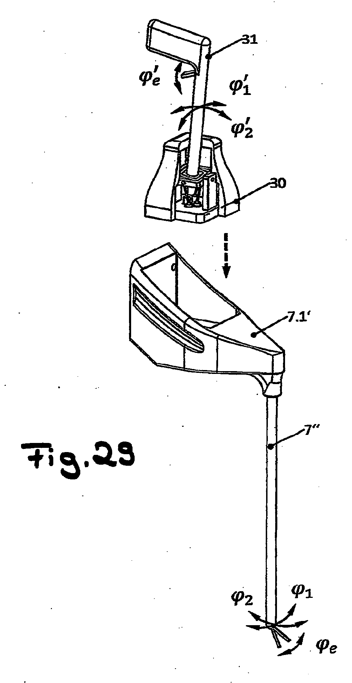

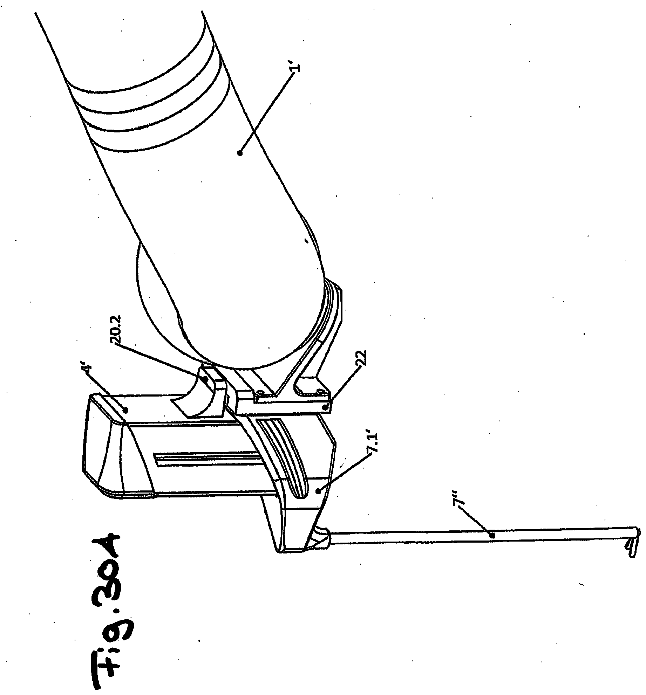

Fig. 29 zeigt eine manuelle Betätigungseinheit einer Instrumentenanordnung nach einer Ausführung der vorliegenden Offenbarung, die anstelle einer modularen Antriebseinheit (in Fig. 29 nicht dargestellt, vgl. etwa Fig. 27A, Fig. 30A), wie sie beispielsweise vorstehend in Fig. 26 bis 28 gezeigt ist, am proximalen Flansch 7.1' des Instrumentenschaftes 7" befestigbar ist.

Die Betätigungseinheit dieses Ausführungsbeispiels ist zur manuellen Aktuierung von zwei Bewegungsfreiheitsgraden ϕ₁ und ϕ₂ und eines Arbeitsfreiheitsgrades ϕₑ eines Endeffektors am distalen Ende des Instrumentenschafts 7" eingerichtet. Als Benutzerschnittstelle dient ein Handhebel 31, der in einer Basis bzw. einem Handhebelgehäuse 30 mit den Freiheitsgraden ϕ'₁ und ϕ'₂ gelagert ist. Diese Freiheitsgrade entsprechen in der gezeigten Ausführung den distalen Bewegungsfreiheitsgraden ϕ₁ und ϕ₂ des Instruments. Außerdem ist an dem Handhebel 31 ein weiterer Freiheitsgrad ϕ'ₑ zur Aktuierung des Arbeitsfreiheitsgrades ϕₑ des distalen Endeffektors vorgesehen. Das Handhebelgehäuse 30 weist eine (in Fig. 29 nicht erkennbare) mechanische Schnittstelle zur wiederholt lösbaren Ankopplung an den Instrumentenschaft auf, die der mechanischen Schnittstelle der zu ersetzenden (nicht dargestellten) mechatronischen Antriebseinheit entspricht.

Des Weiteren enthält das Handhebelgehäuse 30 einen oder mehrere Mechanismen und/oder Getriebe, die die Stellbewegungen des Handhebels 31 in die in der Schnittstelle vorgesehenen Bewegungen umsetzt, optional skaliert und mit den mechanischen Kupplungselementen der Schnittstelle verbindet. Optional kann die Schnittstelle der abnehmbaren Betätigungseinheit auch elektrische Kontakte aufweisen, über die beispielsweise Informationen zwischen Handhebel und Instrument ausgetauscht und/oder Energie zwischen Handhebel und Instrument übertragen werden.

Optional ist in der Betätigungseinheit eine Möglichkeit zur Beschränkung auf ausgewählte distale Freiheitsgrade, die vom Menschen bedient werden können, vorgesehen. Dazu kann die Schnittstelle der Betätigungseinheit eine Blockiervorrichtung zur mechanischen Fixierung eines oder mehrerer distaler Freiheitsgrade in einer vorgegebenen Gelenkstellung enthalten. Vorzugsweise enthält die Blockiervorrichtung mechanische Elemente, mit denen einzelne Teile der instrumentenseitigen Kupplungselemente in einer vorgegebenen Position fixiert werden können.

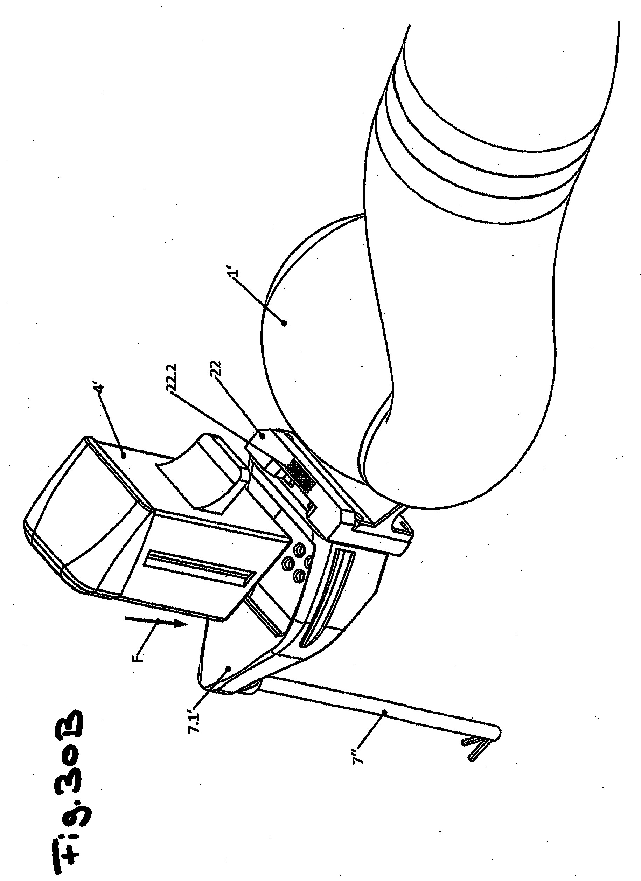

Fig. 30A zeigt das robotergeführte, mit der modularen Antriebseinheit 4' bestückte Chirurgierobotersystem der Fig. 27A aus anderer Blickrichtung. Ebenso wie in Fig. 27 und Fign. 31 bis 33 ist eine sterile Umhüllung des Roboters zur besseren Übersichtlichkeit nicht dargestellt. Diese umschließt den Roboter ganz oder teilweise, insbesondere Teile des sterilen Instrumentenadapters. Bei dieser Ausführung greift die elektrische Schnittstelle 20.2 der Antriebseinheit direkt auf eine elektrische Schnittstelle 22.2 des sterilen Instrumentenadapters 22 (vgl. Fig. 28), so dass die Anzahl an zu sterilisierenden Kontakten minimieren und somit die Kontaktzuverlässigkeit gesteigert werden kann. Eine Detailansicht der elektrischen Schnittstelle 22.2 zwischen sterilem Instrumentenadapter und Antriebseinheit ist in Fig. 30B dargestellt. Es ist zu erkennen, dass die Fügerichtung F der elektrischen Schnittstelle mit der Fügerichtung der Antriebseinheit in den sterilen Instrumentenadapter vorteilhaft übereinstimmt. Um kleinere Lage- und Maßabweichungen zwischen den Kontaktpaaren auszugleichen, kann die elektrische Schnittstelle vorteilhaft Vorrichtungen zum Toleranzausgleich beinhalten.

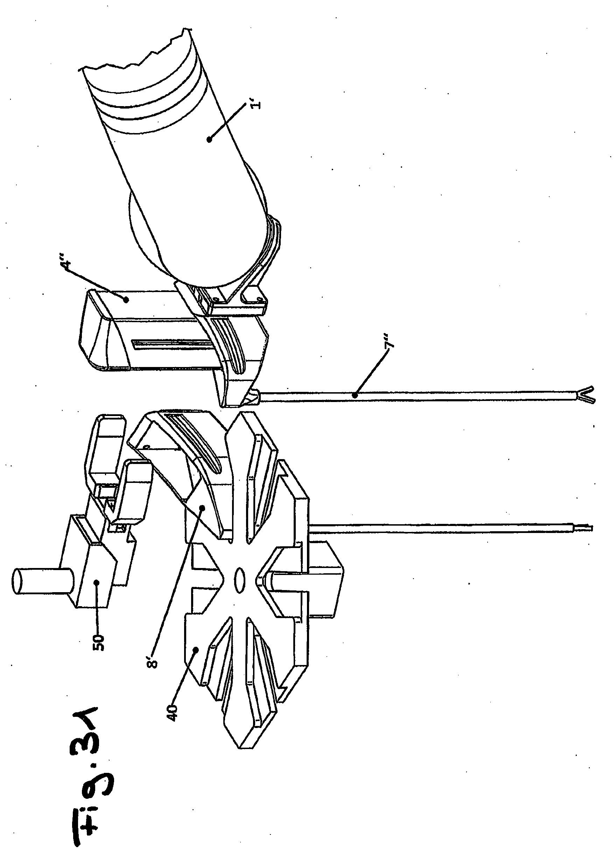

Fig. 31 zeigt ein Chirurgierobotersystem nach einer Ausführung der vorliegenden Offenbarung mit einem Instrumentenmagazin zum wahlweisen Speichern von Instrumenten der Instrumentenanordnung, wie sie beispielsweise vorstehend mit Bezug auf. Fig. 26 bis 30 erläutert wurden.

In dem Instrumentenmagazin 40 können vorbereitete Instrumente, insbesondere mit eingesetzter Antriebseinheit, bis zur Verwendung steril aufbewahrt und zugleich mit Energie versorgt werden. Neben der Energieversorgung besteht optional eine Kommunikationsverbindung zwischen den nicht an den Robotern adaptierten Antriebseinheiten und einer (Instrumenten)Steuerung der Roboteranordnung (nicht dargestellt). Das Instrumentenmagazin kann als sterilisierbare Einheit ausgeführt und/oder mit einer sterilen Hülle umschlossen werden, die vorteilhaft als Einwegartikel realisiert sein kann. Das Instrumentenmagazin kann insbesondere zwei oder mehr Aufnahmeschalen für einzelne oder mehrere Instrumentenschäfte 7", 8' und/oder Antriebseinheiten aufweisen. Durch die Aufnahmeschalen werden die einzelnen Antriebseinheiten an den dafür vorgesehenen Stellen positioniert und die elektrischen Kontakte bzw. andere, insbesondere drahtlose, Energie- und/oder Datenübertragungseinheiten zueinander korrekt ausgerichtet. Gleichermaßen kann das Instrumentenmagazin auch zur freien Ablage von Instrumentenschäften und/oder Antriebseinheiten, insbesondere plattenförmig, ausgebildet sein, d.h. nicht oder nur optional über dedizierte Aufnahmeschalen verfügen. Diese Ausführung ist besonders geeignet für Antriebseinheiten mit drahtloser Energie- und Datenübertragung, bei denen die Energie- und/oder Datenübertragungseinheiten des Instrumentenmagazins rasterförmig angeordnet sein können, so dass beliebige Ablageorte für die Antriebseinheiten möglich sind. Vorteilhaft bei dieser Lösung ist insbesondere die leichtere Reinigung und sterile Abdeckung.

Um ein Instrument bzw. eine Antriebseinheit an einem Roboter zu befestigen, wird sie von dem sterilen Instrumentenmagazin abgenommen. Um auch für die Zeitdauer nach dem Abnehmen von dem Instrumentenmagazins bis zum Adaptieren an einem Roboter die Energieversorgung zumindest der Signalverarbeitungselektronik dieser Antriebseinheit aufrecht zu erhalten, um ein abermaliges Booten und Initialisieren nach der Adaption an einen Manipulatorarm zu vermeiden, weist die Antriebseinheit einen Energiespeicher auf, so dass eine autarke Energieversorgung zumindest der Signalverarbeitungselektronik möglich ist. Dieser Energiespeicher befindet sich in einer Ausführung in der modularen Antriebseinheit und/oder kann bei angeschlossener externer Energieversorgung, insbesondere am Roboter oder im Instrumentenmagazin, regeneriert bzw. aufgeladen werden. Dadurch ist eine einfache Bedienung und Wartung durch das OP-Personal möglich. Gleichermaßen kann die Antriebseinheit auch während der gesamten Dauer eines Eingriffs durch den Energiespeicher versorgt werden. Eine weitere Alternative stellt eine sterile Kabelverbindung zwischen der Roboteranordnung und den adaptierten und/oder nicht adaptierten bzw. an der Roboteranordnung befestigten Instrumenten bzw. Antriebseinheiten zur Energieversorgung und/oder zum Datenaustausch dar. In einer weiteren Ausführung kann eine drahtlose Energieübertragung an Antriebseinheiten vorgesehen sein, die gegenüber insbesondere kabelgebundenen Systemen eine deutlich erhöhte Beweglichkeit der Antriebseinheiten und der Instrumente ermöglichen kann. Vorteilhaft gegenüber einer Versorgung durch einen Energiespeicher sind die geringere Baugröße und das niedrigere Gewicht der Antriebseinheiten. Es können sämtliche elektrische Kontakte entfallen, wodurch sterilen Hüllen deutlich einfacher und kostengünstiger ausgeführt werden können. Durch den Wegfall steriler elektrischer Kontakte vereinfacht sich zudem die Aufbereitung der Instrumenten bzw. Antriebseinheiten.

Insbesondere zur Erhöhung der Betriebssicherheit von energetisch und/oder kommunikativ drahtlos angebundenen Antriebseinheiten kann in einer Ausführung zur Statusübermittlung ein zusätzlicher Kommunikationskanal unabhängig vom eigentlichen Kommunikationskanal vorgesehen sein. Dieser zusätzliche Kommunikationskanal arbeitet vorzugsweise nach einem vom eigentlichen Kommunikationskanal unterschiedlichen physikalischen Prinzip, beispielsweise optisch. Er dient vorzugsweise nicht der Übertragung größerer Datenmengen, sondern lediglich dem Austausch von Statusnachrichten zwischen einem Roboter und einer daran befestigten Antriebseinheit. Der zusätzliche Kommunikationskanal verläuft vorzugsweise parallel zu der eigentlichen Datenverbindung. Er kann nach dem Ruhestromprinzip arbeiten, so dass eine Notabschaltung mindestens des betroffenen Roboters und der betroffenen Antriebseinheit eingeleitet werden kann, sobald die Verbindung unterbrochen oder der Status vom Roboter oder Instrument bzw. Antriebseinheit geändert wird.

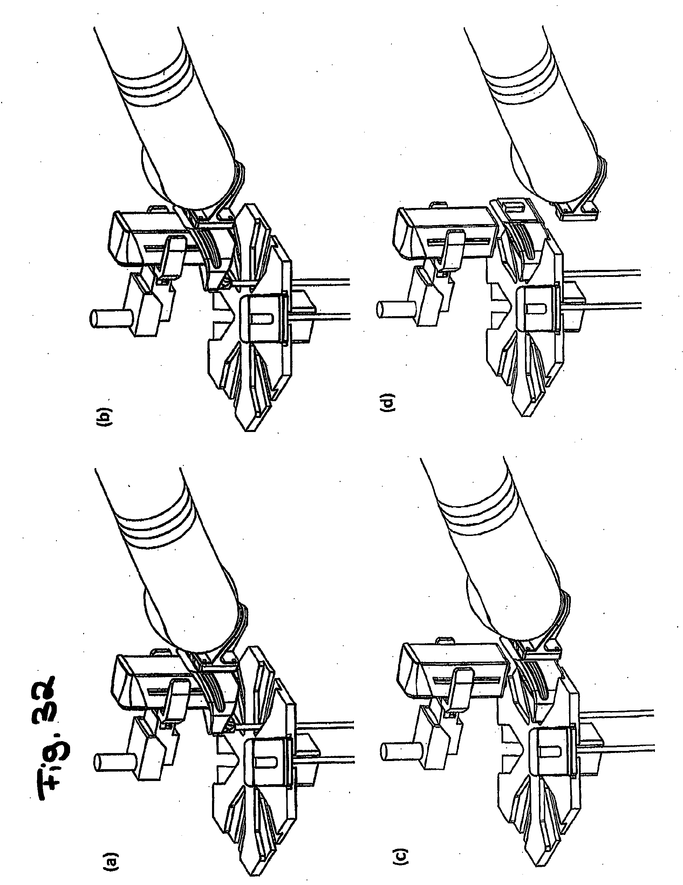

Anhand der Figurenfolgen Fig. 32(a) bis Fig. 33(d) werden nachfolgend Verfahrensschritte eines Verfahrens zum, insbesondere wahlweisen, Bestücken einer Roboteranordnung eines Chirurgierobotersystems mit einem Instrument und eines Instruments mit einer Antriebseinheit nach einer Ausführung der vorliegenden Erfindung näher erläutert.

Eine Registrierung eines an eine Antriebseinheit angekoppelten Instrumentenschafts kann automatisch erfolgen. Dabei laufen nach dem Herstellen einer mechanischen Verbindung von Instrumentenschaft und Antriebseinheit vorzugsweise einer oder mehrere der folgenden Schritte ab:
1) Eine Kopplung mit einem Instrumentenschaft wird erkannt und ein Registrierungsvorgang zur konkreten Bestimmung des angeschlossenen Instruments aktiviert;
2) Das Instrument bzw. der Instrumentenschaft kann sich selbst identifizieren, insbesondere durch eine aktive Kommunikation zwischen der Antriebseinheit und einem im Instrumentenschaft integrierten Controller, vorzugsweise Mikrocontroller. Alternativ kann ein angekoppeltes Instrument identifiziert werden, insbesondere mittels eines nichtflüchtigen Speicherbaustein, beispielsweise eines EEPROMs, im Instrument, wobei die Informationen von der Antriebseinheit abgefragt werden können.
   Ein Instrument bzw. Instrumentenschaft enthält vorzugsweise ein oder mehrere der folgenden Daten: Identifizierungscode, Instrumentenname, Seriennummer, Anzahl der verbleibenden bzw. noch zur Verfügung stehenden Einsätze, Kalibrierparameter zum Ausgleich von Fertigungs- und Montagetoleranzen und/oder kinematische und/oder dynamische Parameter, die einen Instrumententyp charakterisieren, beispielsweise Gewicht, Schwerpunktslage, Trägheitstensor, Ursprung und Orientierung des Endeffektor-Koordinatensystems, kinematikspezifische Transformationsmatrizen, Gelenkwinkelgrenzen bzw. kartesischer Arbeitsraum.
3) Nach einer erfolgreichen Registrierung ist das mit einer Antriebseinheit bestückte Instrument der Roboteranordnung bekannt, so dass einer oder mehrere der folgenden Schritte durchgeführt werden können:
   3.1) Weitergabe der Instrumenten-Daten an die Instrumentensteuerung der Roboteranordnung und/oder eine Steuerungsebene der Antriebseinheit, insbesondere an einen Elektronikteil. Dies kann vorzugsweise bereits bei Verbindung von Instrumentenschaft und Antriebseinheit, insbesondere in einem Instrumentenmagazin, oder auch erst bei Verbindung mit dem Roboter erfolgen.
      Ist beispielsweise eine Stromregelung der Antriebseinheit dezentral in dieser implementiert, eine Positionsregelung zentral in der Instrumentensteuerung der Roboteranordnung, kann die Antriebseinheit als Gateway fungieren und alle Instrumentendaten an die Instrumentensteuerung weiterleitet, beispielsweise über einen Feldbus.
   3.2) Statusänderung der Antriebseinheit nach Bestätigung durch die Instrumentensteuerung, insbesondere Signalisierung des Status an einen Bediener.

Alle registrierten Instrumente können in einer Datenbank hinterlegt werden, die während eines Eingriffs laufend aktualisiert werden kann. Diese informationelle Anbindung aller - nicht nur der an der Roboteranordnung adaptierten bzw. befestigten - Instrumente bzw. Antriebseinheiten an die Instrumentensteuerung der Roboteranordnung bietet sowohl für die Ansteuerung der Roboteranordnung als auch für den Anwender einige Vorteile: Der Operateur hat jederzeit einen Überblick der momentan betriebsbereiten Instrumente und deren Status, der Betriebszustand, zum Beispiel "Betriebsbereit", (verschiedene) Fehlerzustände, abgelaufene Leben und dergleichen, jedes Instruments bzw. jeden Antriebseinheit kann dem OP-Personal signalisiert werden. Dies kann beispielsweise akustisch oder optisch durch eine oder mehrere ein- oder mehrfarbige Signalleuchten, insbesondere LEDs, insbesondere an der Antriebseinheit, erfolgen. Analog können dem Operateur an einer Eingabekonsole die Betriebszustände aller oder einzelner Instrumente bzw. Antriebseinheiten durch entsprechende Einblendungen signalisiert werden.

Bei einem Instrumentenwechsel kann der Operateur an der Eingabekonsole ein registriertes Instrument zum Einwechseln sowie den Roboter, an den das ausgewählte Instrument adaptiert werden soll, auswählen. Diese Informationen können genutzt werden, um einen manuellen Instrumentenwechsel zu unterstützen und für das OP-Personal einfacher zu gestalten oder einen automatischen Instrumentenwechsel zu initiieren.

In einer Ausführung verfügen ein oder mehrere Roboter der Roboteranordnung und/oder ein oder mehrere Instrumente und/oder Antriebseinheit der Instrumentenanordnung über eine Signalvorrichtung, beispielsweise eine, insbesondere mehrfarbige, Signalleuchte. Zur Vorbereitung eines Instrumentenwechsels wird die Signalleuchte des betroffenen Roboters in einer bestimmten Farbe und/oder einer bestimmten Blinksequenz aktiviert. Dadurch wird dem OP-Personal eindeutig signalisiert, welche(r) Roboter von dem anstehenden Instrumentenwechsel betroffen ist bzw. sind. Ebenso wird die Signalleuchte des einzuwechselnden Instruments in einer bestimmten Farbe und/oder einer bestimmten Blinksequenz aktiviert, um dem OP-Personal eindeutig anzuzeigen, welches Instrument einzuwechseln ist. Ebenso kann dem OP-Personal ein erfolgreicher Wechselvorgang durch ein spezielles Farb- oder Blinkmuster der Signalleuchten an Manipulatorarm und Instrument angezeigt werden. Alternativ oder zusätzlich zu einer optischen Signalisierung ist auch ein akustisches Signal möglich.

Fig. 32(a) bis 33(d) zeigt ein erfindungsgemäßes Verfahren zum automatischen Instrumentenwechsel bzw. Bestücken einer Roboteranordnung mit einem Instrument der Instrumentenanordnung sowie eines Instruments mit einer Antriebseinheit. Hierzu weist das Chirurgierobotersystem ein Instrumenten-Wechselmagazin auf, in dem alle benötigten Instrumente einsatzbereit abgelegt sind und bereitgehalten werden. Alle Instrumente sind in dem Wechselmagazin ohne Antriebseinheit abgelegt, da die Applikation der Antriebseinheit an ein Instrument während des Wechselvorgangs erfolgt. Dafür verfügt das Wechselmagazin über einen Antriebseinheits-Manipulator 50 (vgl. Fig. 31) zur Handhabung der Antriebseinheiten während des Instrumentenwechsels. Der Antriebseinheits-Manipulator verfügt in einer Ausführung über keine eigenen Bewegungsfreiheitsgrade mit Ausnahme eines Spannmechanismus für die Antriebseinheit, sämtliche Positionierbewegungen werden von dem Roboter ausgeführt. Beispielsweise kann eine Antriebseinheit mit Hilfe dieses Antriebseinheits-Manipulators von einem Instrumentenschaft getrennt und mit einem anderen verbunden werden. Dadurch kann die Anzahl der Antriebseinheiten reduziert werden, da nicht jedes im Wechselmagazin vorhandene Instrument mit einer eigenen Antriebseinheit ausgestattet sein muss. Außerdem erfordert dieses Konzept einen geringeren Aufwand zur Energieversorgung der Antriebseinheiten, da keine Energieversorgung der im Wechselmagazin abgelegten Instrumente erforderlich ist. Es muss lediglich die Energieversorgung der Antriebseinheiten für den Zeitraum gewährleistet sein, in dem sie nicht an einem Roboter adaptiert ist und von dort mit Energie versorgt werden. Die Energieversorgung der Antriebseinheit während dieses Zeitraums kann in einer Ausführung über den Antriebseinheits-Manipulator erfolgen.

In Fig. 31, 32 ist ein rotatorisches Instrumenten-Wechselmagazin dargestellt. Gleichermaßen kann ein lineares Instrumenten- Wechselmagazin verwendet werden. Um unterschiedliche Antriebseinheiten handhaben zu können, kann der Antriebseinheits-Manipulator optional mit mehreren Greifern, die auch unterschiedlich ausgeprägt sein können, ausgestattet sein. In diesem Fall verfügt der Antriebseinheits-Manipulator vorzugsweise über eine oder mehrere translatorische und/oder eine oder mehrere rotatorische Bewegungsmöglichkeiten, um jeweils die benötigte Antriebseinheit handzuhaben.

Fig. 32(a) bis (d) zeigt Schritte beim Ablegen eines Instruments in dem Instrumenten-Wechselmagazin, wie sie insbesondere während eines automatischen Werkzeugwechsels erfolgen können. Zunächst wird der Roboter aus dem OP-Gebiet zum Werkzeugmagazin in die Ausgangsposition zur Instrumentenablage verfahren (Fig. 32(a)). Dann wird die Antriebseinheit an den Antriebseinheits-Manipulator adaptiert bzw. befestigt, in Fig. 32(b) beispielhaft dargestellt als Zweibackengreifer. Sofern erforderlich, wird eine Fixierung zwischen Antriebseinheit und Instrument gelöst. Optional erfolgt eine Aufrechterhaltung der Energieversorgung der Antriebseinheit, insbesondere kontaktbehaftet oder kontaktlos, etwa durch den Antriebseinheits-Manipulator. Dann wird der Instrumentenschaft im Wechselmagazin abgelegt (Fig. 32(c)), wobei die Verbindung zwischen dem und der vom Antriebseinheits-Manipulator fixierten Antriebseinheit gelöst wird. Schließlich fährt der Roboter von dem Wechselmagazin fort, wobei dadurch oder zuvor die Verbindung zwischen Instrumentenschaft und Roboter gelöst wird (Fig. 32(d)).

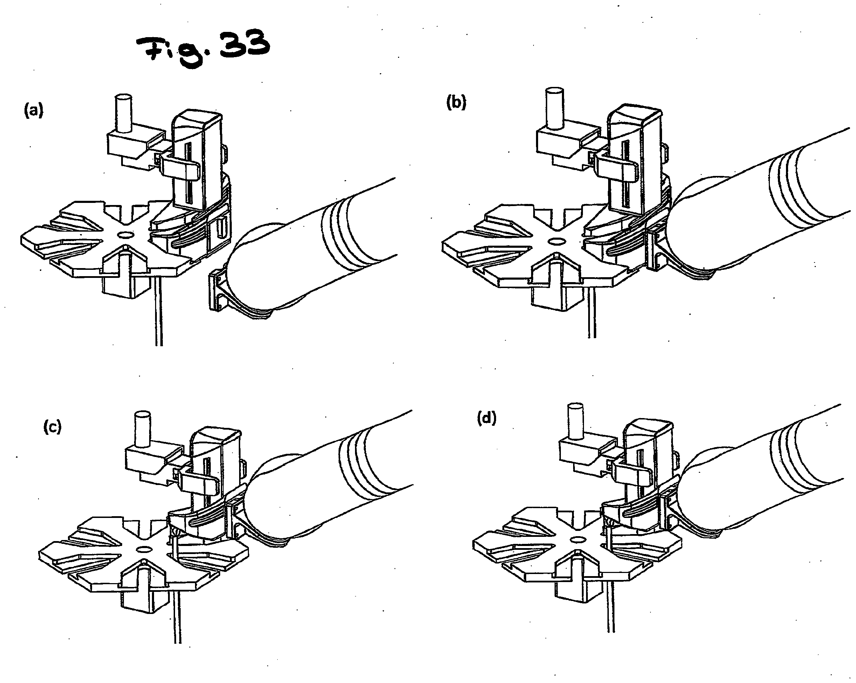

Fig. 33a) bis (d) zeigt Schritte zum Aufnehmen eines Instruments aus dem Wechselmagazin durch einen Roboter: Zunächst wird das einzuwechselnde Instrument durch Verfahren des Instrumenten-Wechselmagazins bereitgestellt, wobei die Antriebseinheit vom Antriebseinheits-Manipulator in der korrekten Position angeordnet wird (Fig. 33(a)). Dann wird der Instrumentenschaft am Roboter fixiert (Fig. 33(b)), der diesen zu der vom Antriebseinheits-Manipulator fixierten Antriebseinheit transportiert, wo diese an dem Instrumentenschaft adaptiert bzw. befestigt wird (Fig. 33(c)). Die Fixierung der Antriebseinheit im Antriebseinheits-Manipulator wird gelöst. Anschließend ist die eingewechselte Instrument-Antriebs-Einheit einsatzbereit (Fig. 33(d)) und kann robotergeführt verfahren werden.

Man erkennt, dass in diesen Verfahrensschritten synergetisch einerseits die Roboteranordnung wahlweise mit einem Instrument bestückt wird (vgl. insbesondere Fig. 32(c) → Fig. 32(d) Ablage eines robotergeführten Instruments; Fig. 33(b) Aufnahme eines gespeicherten Instruments durch einen Roboter), und andererseits ein robotergeführten, in einem Instrumentenmagazin gespeichertes Instrument wahlweise mit einer Antriebseinheit bestückt wird (vgl. insbesondere Fig. 32(b) → Fig. 32(c) Trennen der Antriebseinheit; Fig. 33(b) → Fig. 33(c) Befestigten der Antriebseinheit am Instrumentenschaft).

### Bezugszeichenliste

### In den Figuren 1 bis 14:

- 1: Instrument
- 2: Instrumentengehäuse
- 3: Instrumentenschaft
- 4: Antriebseinheit
- 5: Verschluss
- 6,7: Fixiermittel/-element
- 8,19: Sterilbarriere
- 9,10: Einzelantriebe
- 11,12: Kupplungselemente
- 13,14: Seilzüge
- 15-18: Umlenkrolle
- 101: Instrument
- 102: Instrumentengehäuse
- 103: Instrumentenschaft
- 104: Antriebseinheit
- 105: Verschluss
- 106,107: Fixiermittel/-elemente
- 109,110: Einzelantriebe
- 111,112: Kupplungselemente
- 113,114: Seilzüge
- 115-118: Umlenkrolle
- 119: Sterilbarriere
- 201: Roboter/Manipulatorarm
- 202: proximale Basis
- 203: distales Ende
- 204: sterile Hülle
- 205: Instrument
- 206: Instrumentengehäuse
- 207: Instrumentenkinematik
- 208: chirurgischer Endeffektor
- 209: Instrumentenschaft
- 210: Instrumentenadapter
- 211: Sterilbarriere
- 212: Antriebseinheit
- 213: Verschlussdeckel
- 214: Kontaktpin
- 215: Steckbuchse
- 216: Aussparung
- 217: radialer Absatz
- 601: Sterilschutz
- 701: starre Zwischenplatte
- 702: Seite der starren Zwischenplatte
- 703: gegenüberliegende Seite der starren Zwischenplatte
- 704: Randfläche
- 705: Bewegungsübertragungselement
- 706: Ausstülpung
- 707: elastische Membran
- 801-803: Antriebseinheit
- 805a-805d, 807a-807e, 809a-809d: Einzelantrieb
- 804a-804c: Gehäuse
- 806,808,810: mechanische Kodierung
- 901: Instrument
- 902: Antriebseinheit-Gehäuseteil
- 903: Antriebsachsen-Gehäuseteil
- 904: Instrumentenschaft
- 905: Adapter
- 906,907: Kupplungselement
- 908,909: Seilzüge
- 910-913: Umlenkrollen
- 914: Antriebseinheit
- 915: Gehäuse
- 916: Verschlussdeckel
- 917: Fixiermittel/-element
- 918,919: Einzelantriebe
- 920: Sterilbarriere
- 921,922: Bewegungsübertragungselement
- 1101-1103: Manipulatorarm
- 1104-1106: chirurgisches Instrument
- 1107-1109: Instrumentenschaft
- 1110-1112: Durchtrittspunkt
- 1113: Bauchdecke
- 1114: Operationsgebiet
- 1115: minimaler Abstand

### In den Figuren 15 bis 25:

- 1A, 1B: Abtriebsachse eines Aktuators einer Aktuatoranordnung
- 2A, 2B: Positionssensor (elektronisches Bauteil)
- 3: Gehäuse
- 3.1: Durchtrittsöffnungen
- 3.2: Gehäusetopf (Gehäuseteil)
- 3.3: Gehäusedeckel (Gehäuseteil)
- 3.4: Gehäusewand
- 4; 4.1, 4.2: thermische Isolierschicht
- 5: thermische Isolierschicht
- 6;: stationäres Wärmeleitmittel
- 6.1: Wärmeabgabefläche
- 6.2: Wärmeaufnahmefläche
- 7: bewegliches Element (schaltbares Wärmeleitmittel)
- 8.1; 9.1: Spalt
- 8.2; 9.2: bewegliches Element (schaltbares Wärmeleitmittel)
- 8.3; 9.3: Hülle
- 9.4: Wärmesammelmittel
- 10: Peltierelement (schaltbares Wärmeleitmittel)
- 10.1: Wärmeabgabefläche
- 11: Arbeitsfluidpassage (schaltbares Wärmeleitmittel)
- 11.2: Wärmeaufnahmefläche
- 12: Wärmerohr (schaltbares Wärmeleitmittel)
- 12.1: Wärmeabgabefläche
- 12.2: Wärmeaufnahmefläche
- 100: Hülle

### In den Figuren 26 bis 33:

- 1; 1', 2, 3: Roboter(anordnung)
- 4; 4'; 4", 5, 6: modulare Antriebseinheit
- 7; 7'; 7", 8;: Instrumentenschaft
- 8', 9 7.1; 7.1': Flansch
- 7.2: Antriebsstrang
- 10,11,12: Öffnung
- 13: Bauchdecke
- 14: Operationsgebiet
- 20: Elektronikmodul (Antriebseinheit)
- 20.1; 20.2, 22.2: Schnittstelle
- 21: Antriebsmodul (Antriebseinheit)
- 22: Instrumentenadapter
- 24: sterile Hülle
- 30: Basis (Betätigungseinheit)
- 31: Handhebel (Betätigungseinheit)
- 40: Instrumentenmagazin
- 50: Antriebseinheits-Manipulator

## Patentansprüche

1. Instrumentenanordnung für ein Chirurgierobotersystem, das eine Roboteranordnung mit wenigstens einem Roboter (201; 1101, 1102, 1103) aufweist, mit wenigstens einem durch die Roboteranordnung führbaren Instrument (1; 101; 205; 901; 1104, 1105, 1106) mit
einem Instrumentenschaft (3; 103; 209; 904; 1107, 1108, 1109), der zur teilweisen Einführung (1110, 1111, 1112) in einen Patienten vorgesehen ist;
einer Antriebsstranganordnung mit wenigstens einem Antriebsstrang (11-18; 111-118; 906-913) zur Aktuierung eines Freiheitsgrades des Instrumentenschaftes, insbesondere eines Endeffektors (208; 1114); einer modularen Antriebseinheit (4; 104; 212; 801, 802, 803; 914) zur Aktuierung der Antriebsstranganordnung; und
einem Instrumentengehäuse mit
wenigstens einem Antriebsstrang-Gehäuseteil (2; 102; 206; 903), an dem wenigstens ein Teil der Antriebsstranganordnung angeordnet ist; und
wenigstens einem Antriebseinheit-Gehäuseteil (2; 102; 206; 902, 915) mit einem Hohlraum, der zur Aufnahme der Antriebseinheit eingerichtet ist, wobei das Antriebseinheit-Gehäuseteil eine dynamische Sterilbarriere (8; 19; 119; 211; 920) aufweist, die den Hohlraum steril abgrenzt und über die hinweg die Antriebsstranganordnung aktuierbar ist,
**dadurch gekennzeichnet, dass**
das Antriebseinheit-Gehäuseteil einen Verschluss (5; 105; 213; 916) zur sterilen Abdichtung einer Einführöffnung des Hohlraums aufweist.

2. Instrumentenanordnung nach Anspruch 1, **dadurch gekennzeichnet, dass** das Antriebsstrang-Gehäuseteil und das Antriebseinheit-Gehäuseteil lösbar (903, 902, 915) oder dauerhaft miteinander verbunden, insbesondere integral miteinander ausgebildet (2; 102; 206) sind.

3. Instrumentenanordnung nach Anspruch 2, **gekennzeichnet durch** wenigstens zwei von dem Antriebsstrang-Gehäuseteil oder dem Antriebseinheit-Gehäuseteil zur wahlweisen Verbindung mit dem anderen von dem Antriebsstrang-Gehäuseteil und dem Antriebseinheit-Gehäuseteil.

4. Instrumentenanordnung nach einem der vorhergehenden Ansprüche, **gekennzeichnet durch** wenigstens zwei Antriebseinheiten (801, 802, 803) und/oder Antriebseinheit-Gehäuseteile mit unterschiedlichen, insbesondere mechanischen, Kodierungen.

5. Instrumentenanordnung nach einem der vorhergehenden Ansprüche, **dadurch gekennzeichnet, dass** die dynamische Sterilbarriere lösbar (8; 211) oder dauerhaft (19; 119; 920) mit dem Antriebsstrang- und/oder Antriebseinheit-Gehäuseteil verbunden ist.

6. Instrumentenanordnung nach einem der vorhergehenden Ansprüche, **gekennzeichnet durch** eine elektro-mechanische Schnittstelle (210) zur lösbaren Anbindung des Instrumentengehäuses, insbesondere des Antriebsstrang-Gehäuseteils, an die Roboteranordnung.

7. Instrumentenanordnung nach Anspruch 6, **dadurch gekennzeichnet, dass** die elektro-mechanische Schnittstelle mittels einer mechanischen Steckverbindung (216, 217), insbesondere unter Durchdringung einer statischen Sterilbarriere (204), mit dem Instrumentengehäuse und/oder der Roboteranordnung verbunden ist.

8. Instrumentenanordnung nach einem der vorhergehenden Ansprüche, **gekennzeichnet durch** ein, insbesondere spannendes, verrastendes und/oder verschlussfestes, Fixiermittel (6, 7; 106, 107; 917) zur Fixierung der Antriebseinheit in dem Hohlraum.

9. Instrumentenanordnung nach einem der vorhergehenden Ansprüche, **dadurch gekennzeichnet, dass** die Einführöffnung auf einer dem Instrumentenschaft ab- oder zugewandten Stirnseite oder einer seitlichen Mantelfläche des Instrumentengehäuses angeordnet ist.

10. Chirurgierobotersystem mit:
einer Roboteranordnung mit wenigstens einem Roboter (201; 1101, 1102, 1103), und
einer Instrumentenanordnung mit wenigstens einem durch die Roboteranordnung geführten Instrument (1; 101; 205; 901; 1104, 1105, 1106) nach einem der vorangehenden Ansprüche.

11. Verfahren zur Montage einer Instrumentenanordnung nach einem der vorhergehenden Ansprüche 1 bis 9, mit den Schritten:
(S100) steriles Abdecken der Umgebung der Einführöffnung;
(S300) Einführen der Antriebseinheit in den Hohlraum des Antriebseinheit-Gehäuseteils; und
(S500) steriles Abdichten des Verschlusses.

12. Verfahren nach Anspruch 11 zum, insbesondere wahlweisen, Bestücken wenigstens eines, insbesondere robotergeführten und/oder in einem Instrumentenmagazin gespeicherten, Instruments eines Chirurgierobotersystems nach Anspruch 10 mit der Antriebseinheit.

13. Verfahren, insbesondere nach einem der Ansprüche 11 oder 12, zur Montage eines Chirurgierobotersystems nach Anspruch 10, mit den Schritten
(S10) steriles Verpacken der Roboteranordnung und/oder wenigstens einer Antriebseinheit der Instrumentenanordnung in einem Antriebseinheit-Gehäuseteil;
(S20) Herstellen einer mechanischen Steckverbindung einer elektro-mechanischen Schnittstelle zwischen der Roboteranordnung und der Instrumentenanordnung;
(S30) insbesondere durch die mechanische Steckverbindung geführtes, Durchstoßen einer statischen Sterilbarriere der Roboteranordnung und/oder des Instrumentengehäuses; und
(S40) Fixieren der elektro-mechanischen Schnittstelle.

14. Verfahren nach Anspruch 13 zum, insbesondere wahlweisen, Bestücken einer Roboteranordnung eines Chirurgierobotersystems nach Anspruch 10 mit wenigstens einem Instrument der Instrumentenanordnung des Chirurgierobotersystems.

## Claims

1. An instrument assembly for a surgical robot system, which comprises a robot assembly with at least one robot (201; 1101, 1102, 1103), with at least one instrument (1; 101; 205; 901; 1104, 1105, 1106) which can be guided by means of the robot assembly and which comprises
an instrument shaft (3; 103; 209; 904; 1107, 1108, 1109) which is intended for partial insertion (1110, 1111, 1112) into a patient;
a drive train assembly comprising at least one drive train (11-18; 111-118; 906-913) for actuating a degree of freedom of the instrument shaft, in particular of an end effector (208; 1114);
a modular drive unit (4; 104; 212; 801, 802, 803; 914) for actuating the drive train assembly; and
an instrument housing comprising
at least one drive train housing portion (2; 102; 206; 903) on which at least a portion of the drive train assembly is arranged; and
at least one drive unit housing portion (2; 102; 206; 902, 915) having a cavity which is arranged to receive the drive unit, wherein the drive unit housing portion comprises a dynamic sterile barrier (8; 19; 119; 211; 920) which delimits the cavity in a sterile manner and through which the drive train assembly can be actuated,
**characterised in that**
the drive unit housing portion comprises a closure (5; 105; 213; 916) for sealing an insertion opening of the cavity in a sterile manner.

2. The instrument assembly according to claim 1, **characterised in that** the drive train housing portion and the drive unit housing portion are releasably (903, 902, 915) or permanently connected to one another, in particular integrally formed with one another (2; 102; 206).

3. The instrument assembly according to claim 2, **characterised by** at least two of the drive train housing portion or the drive unit housing portion for selective connection to the other of the drive train housing portion and the drive unit housing portion.

4. The instrument assembly according to any one of the preceding claims, **characterised by** at least two drive units (801, 802, 803) and / or drive unit housing portions with different coding features, in particular different mechanical coding features.

5. The instrument assembly according to any one of the preceding claims, **characterised in that** the dynamic sterile barrier is releasably (8; 211) or permanently (19; 119; 920) connected to the drive train housing portion and / or the drive unit housing portion.

6. The instrument assembly according to any one of the preceding claims, **characterised by** an electromechanical interface (210) for releasably connecting the instrument housing, in particular the drive train housing portion, to the robot assembly.

7. The instrument assembly according to claim 6, **characterised in that** the electromechanical interface is connected to the instrument housing and / or the robot assembly by means of a mechanical plug-in connection (216, 217), in particular by penetrating a static sterile barrier (204).

8. The instrument assembly according to any one of the preceding claims, **characterised by** a fixing means (6, 7; 106, 107; 917) for fixing the drive unit in the cavity, in particular a tensioning, latching and / or locking fixing means (6, 7; 106, 107; 917).

9. The instrument assembly according to any one of the preceding claims, **characterised in that** the insertion opening is arranged on an end face which faces away from the instrument shaft or which faces towards the instrument shaft or on a lateral outer surface of the instrument housing.

10. A surgical robot system comprising:
a robot assembly comprising at least one robot (201; 1101, 1102, 1103), and
an instrument assembly comprising at least one instrument (1; 101; 205; 901; 1104, 1105, 1106) guided by the robot assembly according to any one of the preceding claims.

11. A method of assembling an instrument assembly according to any one of the preceding claims 1 to 9, comprising the steps of:
(S100) covering the vicinity of the insertion opening in a sterile manner;
(S300) introducing the drive unit into the cavity of the drive unit housing portion; and
(S500) sealing of the closure in a sterile manner.

12. The method according to claim 11 for equipping at least one instrument of a surgical robot system according to claim 10, in particular an instrument guided by a robot and / or an instrument stored in an instrument magazine, with the drive unit, in particular in a selective manner.

13. A method, in particular the method according to any one of the claims 11 or 12, for assembling a surgical robot system according to claim 10, comprising the steps of:
(S10) packaging of the robot assembly and / or of at least one drive unit of the instrument assembly in a drive unit housing portion in a sterile manner;
(S20) establishing of a mechanical plug-in connection of an electromechanical interface between the robot assembly and the instrument assembly;
(S30) piercing of a static sterile barrier of the robot assembly and / or of the instrument housing, in particular guided by the mechanical plug-in connection; and
(S40) fixing of the electro-mechanical interface.

14. The method according to claim 13 for equipping a robot assembly of a surgical robot system according to claim 10, in particular selectively, with at least one instrument of the instrument assembly of the surgical robot system.

## Revendications

1. Agencement d'instrument pour un système de robot chirurgical, qui présente un agencement de robot avec au moins un robot (201 ; 1101, 1102, 1103), avec au moins un instrument (1 ; 101 ; 205 ; 901 ; 1104, 1105, 1106) pouvant être guidé par l'agencement de robot avec
un arbre d'instrument (3 ; 103 ; 209 ; 904 ; 1107, 1108, 1109), qui est prévu pour l'introduction partielle (1110, 1111, 1112) dans un patient ;
un agencement de chaîne cinématique avec au moins une chaîne cinématique (11-18 ; 111-118 ; 906-913) pour l'actionnement d'un degré de liberté de l'arbre d'instrument, en particulier d'un effecteur d'extrémité (208 ; 1114) ;
une unité d'entraînement modulaire (4 ; 104 ; 212 ; 801, 802, 803 ; 914) pour l'actionnement de l'agencement de chaîne cinématique ; et
un boîtier d'instrument avec
au moins une partie de boîtier de chaîne cinématique (2 ; 102 ; 206 ; 903), au niveau de laquelle au moins une partie de l'agencement de chaîne cinématique est agencée ; et
au moins une partie de boîtier d'unité d'entraînement (2 ; 102 ; 206 ; 902, 915) avec une cavité, qui est aménagée pour la réception de l'unité d'entraînement, dans lequel la partie de boîtier d'unité d'entraînement présente une barrière stérile dynamique (8 ; 19 ; 119 ; 211 ; 920), qui délimite de manière stérile la cavité et au-delà de laquelle l'agencement de chaîne cinématique est actionnable,
**caractérisé en ce que**
la partie de boîtier d'unité d'entraînement présente une fermeture (5 ; 105 ; 213 ; 916) pour l'étanchéification stérile d'une ouverture d'introduction de la cavité.

2. Agencement d'instrument selon la revendication 1, **caractérisé en ce que** la partie de boîtier de chaîne cinématique et la partie de boîtier d'unité d'entraînement sont reliées l'une à l'autre de manière amovible (903, 902, 915) ou durable, en particulier réalisées l'une avec l'autre de manière intégrale (2 ; 102 ; 206).

3. Agencement d'instrument selon la revendication 2, **caractérisé par** au moins deux parmi la partie de boîtier de chaîne cinématique ou la partie de boîtier d'unité d'entraînement pour la liaison sélective avec l'autre parmi la partie de boîtier de chaîne cinématique et la partie de boîtier d'unité d'entraînement.

4. Agencement d'instrument selon l'une quelconque des revendications précédentes, **caractérisé par** au moins deux unités d'entraînement (801, 802, 803) et/ou parties de boîtier d'unité d'entraînement avec différents codages, en particulier mécaniques.

5. Agencement d'instrument selon l'une quelconque des revendications précédentes, **caractérisé en ce que** la barrière stérile dynamique est reliée de manière amovible (8 ; 211) ou durable (19 ; 119 ; 920) à la partie de boîtier de chaîne cinématique et/ou d'unité d'entraînement.

6. Agencement d'instrument selon l'une quelconque des revendications précédentes, **caractérisé par** une interface électromécanique (210) pour le raccordement amovible du boîtier d'instrument, en particulier de la partie de boîtier de chaîne cinématique, à l'agencement de robot.

7. Agencement d'instrument selon la revendication 6, **caractérisé en ce que** l'interface électromécanique est reliée au boîtier d'instrument et/ou à l'agencement de robot au moyen d'une liaison enfichable mécanique (216, 217), en particulier par traversée d'une barrière stérile statique (204).

8. Agencement d'instrument selon l'une quelconque des revendications précédentes, **caractérisé par** un moyen de fixation (6, 7 ; 106, 107 ; 917), en particulier par serrage, par encliquetage et/ou par fermeture, pour la fixation de l'unité d'entraînement dans la cavité.

9. Agencement d'instrument selon l'une quelconque des revendications précédentes, **caractérisé en ce que** l'ouverture d'introduction est agencée sur un côté frontal tourné vers ou opposé à l'arbre d'instrument ou une surface d'enveloppe latérale du boîtier d'instrument.

10. Système de robot chirurgical avec :
un agencement de robot avec au moins un robot (201 ; 1101, 1102, 1103), et
un agencement d'instrument avec au moins un instrument (1 ; 101 ; 205 ; 901 ; 1104, 1105, 1106) guidé par l'agencement de robot selon l'une quelconque des revendications précédentes.

11. Procédé de montage d'un agencement d'instrument selon l'une quelconque des revendications précédentes 1 à 9, avec les étapes de :
(S100) recouvrement stérile de l'environnement de l'ouverture d'introduction ;
(S300) introduction de l'unité d'entraînement dans la cavité de la partie de boîtier d'unité d'entraînement ; et
(S500) étanchéification stérile de la fermeture.

12. Procédé selon la revendication 11 pour l'équipement, en particulier sélectif, d'au moins un instrument, en particulier guidé par robot et/ou enregistré dans un magasin d'instrument, d'un système de robot chirurgical selon la revendication 10 avec l'unité d'entraînement.

13. Procédé, en particulier selon l'une quelconque des revendications 11 ou 12, pour le montage d'un système de robot chirurgical selon la revendication 10, avec les étapes de
(S10) conditionnement stérile de l'agencement de robot et/ou d'au moins une unité d'entraînement de l'agencement d'instrument dans une partie de boîtier d'unité d'entraînement ;
(S20) établissement d'une liaison enfichable mécanique d'une interface électromécanique entre l'agencement de robot et l'agencement d'instrument ;
(S30) transperçage, en particulier guidé par la liaison enfichable mécanique, d'une barrière stérile statique de l'agencement de robot et/ou du boîtier d'instrument ; et (S40) fixation de l'interface électromécanique.

14. Procédé selon la revendication 13 pour l'équipement, en particulier sélectif, d'un agencement de robot d'un système de robot chirurgical selon la revendication 10 avec au moins un instrument de l'agencement d'instrument du système de robot chirurgical.
